# EUROPEAN PATENT APPLICATION

(11) **EP 1 304 376 A1**
(43) Date of publication of application: **23.04.2003**
(21) Application number: 01984291.3
(22) Date of filing: 25.07.2001
(51) Int. Cl.: C12N 15/10, C12N 1/21, C12P 21/02, C12Q 1/02

(54) **PROCESS FOR PRODUCING RECOMBINANT PROTEIN**

(30) Priority: 25.07.2000 JP 2000229064
(71) Applicant: Takeda Chemical Industries, Ltd., Osaka-shi, Osaka 541-0045 (JP)
(72) Inventor: ITO, Takashi, Kobe-shi, Hoyogo 658-0053 (JP); TANAKA, Yoko, Kyotanabe-shi, Kyoto 610-0331 (JP); KONDO, Mitsuyo, Kawanishi-shi, Hyogo 666-0104 (JP)
(74) Representative: Rickard, Timothy Mark Adrian
(86) International application number: JP0106392
(87) International publication number: WO02008417

(57) **Abstract**

It is intended to produce a target protein in procaryotic or eucaryotic cells. A DNA encoding the full-length amino acid sequence or a part of the same of a target protein is prepared and the target protein is produced by a gene recombination technique with the use of this DNA.

[Sequence Listing]

<110> Takeda Chemical Industries, Ltd.

<120> Method for production of recombinant protein

<130> P2001-141PCT

<150> JP 2000-229064
<151> 2000-07-25

<160> 122

<210> 1
<211> 28
<212> DNA
<213> Artificial sequence

<220>
<223> Primer

<400> 1
cgttatccgg atcacatgaa acggcatg     28

<210> 2
<211> 28
<212> DNA
<213> Artificial sequence

<220>
<223> Primer

<400> 2
catgccgttt catgtgatcc ggataacg     28

<210> 3

<211> 33
<212> DNA
<213> Artificial sequence

<220>
<223> Synthetic DNA

<400> 3
agcttcatat gttcgaagta ctagatctgg tac     33

<210> 4
<211> 25
<212> DNA
<213> Artificial sequence

<220>
<223> Synthetic DNA

<400> 4
cagatctagt acttcgaaca tatga     25

<210> 5
<211> 109
<212> DNA
<213> Artificial sequence

<220>
<223> Synthetic DNA

<400> 5
aattctataa aaataattgt tgacatattt tataaatttt ggcataatag atctaattgt 60
gagcggataa caattctgca gaagcttgag ctcggtaccc ggggatcct     109

<210> 6
<211> 109
<212> DNA

<213> Artificial sequence

<220>
<223> Synthetic DNA

<400> 6
ctagaggatc cccgggtacc gagctcaagc ttctgcagaa ttgttatccg ctcacaatta 60
gatctattat gccaaaattt ataaaatatg tcaacaatta tttttatag 109

<210> 7
<211> 77
<212> DNA
<213> Artificial sequence

<220>
<223> Synthetic DNA

<400> 7
aggtaaccag cactatttaa agtccanccn ccncgnccnc grtgngcngg ngccatatgt 60
atatctcctt cttaaag 77

<210> 8
<211> 77
<212> DNA
<213> Artificial sequence

<220>
<223> Synthetic DNA

<400> 8
aggtaaccag cactatttaa agtccanccn ccyctnccyc trtgngcngg ngccatatgt 60
atatctcctt cttaaag 77

<210> 9
<211> 24
<212> DNA

<213> Artificial sequence

<220>
<223> Primer

<400> 9
ctttaagaag gagatataca tatg    24

<210> 10
<211> 27
<212> DNA
<213> Artificial sequence

<220>
<223> Primer

<400> 10
ttcatacacg gtgcctgact gcgttag    27

<210> 11
<211> 23
<212> DNA
<213> Artificial sequence

<220>
<223> Primer

<400> 11
tcaacaagaa ttgggacaac tcc    23

<210> 12
<211> 23
<212> DNA
<213> Artificial sequence

<220>

<223> Primer

<400> 12
agcatatggc acctgctcac agg     23

<210> 13
<211> 27
<212> DNA
<213> Artificial sequence

<220>
<223> Primer

<400> 13
ccctcggggc aggtcatcct tggagag     27

<210> 14
<211> 77
<212> DNA
<213> Artificial sequence

<220>
<223> Synthetic DNA

<400> 14
gggatgcttc gtggggtgta ggagatgcag cagtcagcac tagtngcrtg raayctrtcc 60
atatgttggc gcgcctt                                                 77

<210> 15
<211> 77
<212> DNA
<213> Artificial sequence

<220>
<223> Synthetic DNA

<400> 15
gggatgcttc gtggggtgta ggagatgcag cagtcagcac tagtngcrtg raancgrtcc 60
atatgttggc gcgcctt 77

<210> 16
<211> 18
<212> DNA
<213> Artificial sequence

<220>
<223> Primer

<400> 16
AAGGCGCGCC AACATATG 18

<210> 17
<211> 69
<212> DNA
<213> Artificial sequence

<220>
<223> Synthetic DNA

<400> 17
tgagcatgca tactagtctc gagtaatccc acagccgccg ccagttccgc tggcggcggc 60
attttcgat 69

<210> 18
<211> 64
<212> DNA
<213> Artificial sequence

<220>
<223> Synthetic DNA

<400> 18

cgaaaatgcc gccgccagcg gaactggcgg cggctgtggg attactcgag actagtatgc 60
atgc 64

<210> 19
<211> 20
<212> DNA
<213> Artificial sequence

<220>
<223> Primer

<400> 19
tatggatcga tttcacgcaa 20

<210> 20
<211> 22
<212> DNA
<213> Artificial sequence

<220>
<223> Synthetic DNA

<400> 20
ctagttgcgt gaaatcgatc ca 22

<210> 21
<211> 14
<212> PRT
<213> Human

<400> 21
Ala Pro Ala His Arg Gly Arg Gly Gly Trp Thr Leu Asn Ser 14

<210> 22
<211> 42
<212> DNA

<213> Human

<400> 22
gcacctgctc acaggggacg aggaggctgg accctcaata gt          42

<210> 23
<211> 42
<212> DNA
<213> Artificial sequence

<220>
<223> Synthetic DNA

<400> 23
gctccagcgc atcgtgggcg tggtggttgg actttaaata gt          42

<210> 24
<211> 42
<212> DNA
<213> Artificial sequence

<220>
<223> Synthetic DNA

<400> 24
gcacctgcac atcgtgggcg aggaggctgg actttaaata gt          42

<210> 25
<211> 42
<212> DNA
<213> Artificial sequence

<220>
<223> Synthetic DNA

<400> 25

gcaccacacg cacgcggacg agggggatgg actttaaata gt 42

<210> 26
<211> 42
<212> DNA
<213> Artificial sequence

<220>
<223> Synthetic DNA

<400> 26
gcaccagcac acagaggaag gggcggatgg actttaaata gt 42

<210> 27
<211> 42
<212> DNA
<213> Artificial sequence

<220>
<223> Synthetic DNA

<400> 27
gcaccagctc accgaggccg aggaggctgg actttaaata gt 42

<210> 28
<211> 42
<212> DNA
<213> Artificial sequence

<220>
<223> Synthetic DNA

<400> 28
gcacccgctc accgcggacg tggaggctgg actttaaata gt 42

<210> 29

<211> 42
<212> DNA
<213> Artificial sequence

<220>
<223> Synthetic DNA

<400> 29
gcaccagcac atagaggaag aggaggctgg actttaaata gt          42

<210> 30
<211> 42
<212> DNA
<213> Artificial sequence

<220>
<223> Synthetic DNA

<400> 30
gcaccggcac accgcggacg agggggttgg actttaaata gt          42

<210> 31
<211> 42
<212> DNA
<213> Artificial sequence

<220>
<223> Synthetic DNA

<400> 31
gcaccggcgc accgcggacg tggcagatgg actttaaata gt          42

<210> 32
<211> 42
<212> DNA
<213> Artificial sequence

<220>
<223> Synthetic DNA

<400> 32
gccccagcac atcgcggacg tggcggatgg actttaaata gt 42

<210> 33
<211> 42
<212> DNA
<213> Artificial sequence

<220>
<223> Synthetic DNA

<400> 33
gccccagcac accgcggtcg cggcggttgg actttaaata gt 42

<210> 34
<211> 42
<212> DNA
<213> Artificial sequence

<220>
<223> Synthetic DNA

<400> 34
gctccagcac accgggggcg ggggggatgg actttaaata gt 42

<210> 35
<211> 42
<212> DNA
<213> Artificial sequence

<220>
<223> Synthetic DNA

<400> 35
gcaccggcac accgcggccg cggaggatgg actttaaata gt          42

<210> 36
<211> 42
<212> DNA
<213> Artificial sequence

<220>
<223> Synthetic DNA

<400> 36
gcgccagctc atcggggtcg tggcgggtgg actttaaata gt          42

<210> 37
<211> 42
<212> DNA
<213> Artificial sequence

<220>
<223> Synthetic DNA

<400> 37
gctccagctc atcgaggacg cggcggttgg actttaaata gt          42

<210> 38
<211> 42
<212> DNA
<213> Artificial sequence

<220>
<223> Synthetic DNA

<400> 38
gcgccagcac accgtggccg aggcggctgg actttaaata gt          42

<210> 39
<211> 42
<212> DNA
<213> Artificial sequence

<220>
<223> Synthetic DNA

<400> 39
gcaccggcgc accgcggacg tggcagatgg actttaaata gt          42

<210> 40
<211> 42
<212> DNA
<213> Artificial sequence

<220>
<223> Synthetic DNA

<400> 40
gccccagcgc atcgggggcg tggcggctgg actttaaata gt          42

<210> 41
<211> 5
<212> PRT
<213> Human

<400> 41
Asp Arg Phe His Ala   5

<210> 42
<211> 15
<212> DNA
<213> Human

<400> 42
gacagattcc acgct        15

<210> 43
<211> 15
<212> DNA
<213> Artificial sequence

<220>
<223> Synthetic DNA

<400> 43
gacagattcc acgca        15

<210> 44
<211> 15
<212> DNA
<213> Artificial sequence

<220>
<223> Synthetic DNA

<400> 44
gacagattcc acgcc        15

<210> 45
<211> 15
<212> DNA
<213> Artificial sequence

<220>
<223> Synthetic DNA

<400> 45
gacagattcc atgca        15

<210> 46
<211> 15
<212> DNA
<213> Artificial sequence

<220>
<223> Synthetic DNA

<400> 46
gacagatttc acgca          15

<210> 47
<211> 15
<212> DNA
<213> Artificial sequence

<220>
<223> Synthetic DNA

<400> 47
gacagatttc acgcc          15

<210> 48
<211> 15
<212> DNA
<213> Artificial sequence

<220>
<223> Synthetic DNA

<400> 48
gacagatttc acgcg          15

<210> 49
<211> 15
<212> DNA

<213> Artificial sequence

<220>
<223> Synthetic DNA

<400> 49
gacaggttcc acgca        15

<210> 50
<211> 15
<212> DNA
<213> Artificial sequence

<220>
<223> Synthetic DNA

<400> 50
gacagattcc acgcg        15

<210> 51
<211> 15
<212> DNA
<213> Artificial sequence

<220>
<223> Synthetic DNA

<400> 51
gacaggtttc acgct        15

<210> 52
<211> 15
<212> DNA
<213> Artificial sequence

<220>

<223> Synthetic DNA

<400> 52
gacaggtttc atgcc        15

<210> 53
<211> 15
<212> DNA
<213> Artificial sequence

<220>
<223> Synthetic DNA

<400> 53
gaccgattcc acgcg        15

<210> 54
<211> 15
<212> DNA
<213> Artificial sequence

<220>
<223> Synthetic DNA

<400> 54
gaccgattcc acgct        15

<210> 55
<211> 15
<212> DNA
<213> Artificial sequence

<220>
<223> Synthetic DNA

<400> 55

gaccgattcc atgcc        15

<210> 56
<211> 15
<212> DNA
<213> Artificial sequence

<220>
<223> Synthetic DNA

<400> 56
gaccgattcc atgcg        15

<210> 57
<211> 15
<212> DNA
<213> Artificial sequence

<220>
<223> Synthetic DNA

<400> 57
gaccgattcc atgct        15

<210> 58
<211> 15
<212> DNA
<213> Artificial sequence

<220>
<223> Synthetic DNA

<400> 58
gaccgatttc atgcc        15

<210> 59

<211> 15
<212> DNA
<213> Artificial sequence

<220>
<223> Synthetic DNA

<400> 59
gaccgcttcc acgcc        15

<210> 60
<211> 15
<212> DNA
<213> Artificial sequence

<220>
<223> Synthetic DNA

<400> 60
gaccgcttcc acgcg        15

<210> 61
<211> 15
<212> DNA
<213> Artificial sequence

<220>
<223> Synthetic DNA

<400> 61
gaccgcttcc acgct        15

<210> 62
<211> 15
<212> DNA
<213> Artificial sequence

<220>
<223> Synthetic DNA

<400> 62
gaccgctttc acgca        15

<210> 63
<211> 15
<212> DNA
<213> Artificial sequence

<220>
<223> Synthetic DNA

<400> 63
gaccggttcc acgca        15

<210> 64
<211> 15
<212> DNA
<213> Artificial sequence

<220>
<223> Synthetic DNA

<400> 64
gaccggttcc atgcc        15

<210> 65
<211> 15
<212> DNA
<213> Artificial sequence

<220>
<223> Synthetic DNA

<400> 65
gaccggtttc acgca        15

<210> 66
<211> 15
<212> DNA
<213> Artificial sequence

<220>
<223> Synthetic DNA

<400> 66
gatagattcc acgct        15

<210> 67
<211> 15
<212> DNA
<213> Artificial sequence

<220>
<223> Synthetic DNA

<400> 67
gatagatttc acgcc        15

<210> 68
<211> 15
<212> DNA
<213> Artificial sequence

<220>
<223> Synthetic DNA

<400> 68
gatagatttc acgcg        15

<210> 69
<211> 15
<212> DNA
<213> Artificial sequence

<220>
<223> Synthetic DNA

<400> 69
gatagatttc acgct          15

<210> 70
<211> 15
<212> DNA
<213> Artificial sequence

<220>
<223> Synthetic DNA

<400> 70
gataggttcc acgct          15

<210> 71
<211> 15
<212> DNA
<213> Artificial sequence

<220>
<223> Synthetic DNA

<400> 71
gatcgatttc acgca          15

<210> 72
<211> 15

<212> DNA
<213> Artificial sequence

<220>
<223> Synthetic DNA

<400> 72
gatcgcttcc acgca        15

<210> 73
<211> 15
<212> DNA
<213> Artificial sequence

<220>
<223> Synthetic DNA

<400> 73
gatcgctttc acgct        15

<210> 74
<211> 15
<212> DNA
<213> Artificial sequence

<220>
<223> Synthetic DNA

<400> 74
gatcggttcc acgca        15

<210> 75
<211> 63
<212> DNA
<213> Artificial sequence

<220>
<223> Synthetic DNA

<400> 75
ctagcgaatt cgagcatatg agcactagtg catgcgagcc atattcaacg ggaaacgtct 60
tgc 63

<210> 76
<211> 63
<212> DNA
<213> Artificial sequence

<220>
<223> Synthetic DNA

<400> 76
tcgagcaaga cgtttcccgt tgaatatggc tcgcatgcac tagtgctcat atgctcgaat 60
tcg 63

<210> 77
<211> 24
<212> DNA
<213> Artificial sequence

<220>
<223> Synthetic DNA

<400> 77
tatgagggta ccgccggctg catg 24

<210> 78
<211> 18
<212> DNA
<213> Artificial sequence

<220>

<223> Synthetic DNA

<400> 78
cagccggcgg taccctca          18

<210> 79
<211> 26
<212> DNA
<213> Artificial sequence

<220>
<223> Synthetic DNA

<400> 79
tatgagggta ccgccggcac tgcatg          26

<210> 80
<211> 20
<212> DNA
<213> Artificial sequence

<220>
<223> Synthetic DNA

<400>80
cactgccggc ggtaccctca          20

<210> 81
<211> 30
<212> DNA
<213> Artificial sequence

<220>
<223> Synthetic DNA

<400> 81

ctcatatggc tgtgatcacc ggtgcctgtg 30

<210> 82
<211> 30
<212> DNA
<213> Artificial sequence

<220>
<223> Synthetic DNA

<400> 82
gcggatccct aaaaattgat gttcttcaag 30

<210>83
<211> 88
<212> DNA
<213> Artificial sequence

<220>
<223> Synthetic DNA

<400> 83
tatggcggtg attaccggtg cgtgcgaacg tgatgtgcag tgcggtgcgg gtacctgctg 60
cgcgattagc ctgtggctgc gtggtctg 88

<210> 84
<211> 92
<212> DNA
<213> Artificial sequence

<220>
<223> Synthetic DNA

<400> 84
cgtatgtgca ccccgctggg tcgtgaaggt gaagaatgcc atccgggtag ccataaagtg 60

ccgttcttcc gtaaacgtaa acatcatacc tg 92

<210> 85
<211> 86
<212> DNA
<213> Artificial sequence

<220>
<223> Synthetic DNA

<400> 85
cccgtgcctg ccgaacctgc tgtgcagccg tttcccggat ggtcgttatc gttgcagcat 60
ggatctgaaa aacattaact tttagg 86

<210> 86
<211> 94
<212> DNA
<213> Artificial sequence

<220>
<223> Synthetic DNA

<400> 86
cacatacgca gaccacgcag ccacaggcta atcgcgcagc aggtacccgc accgcactgc 60
acatcacgtt cgcacgcacc ggtaatcacc gcca 94

<210> 87
<211> 93
<212> DNA
<213> Artificial sequence

<220>
<223> Synthetic DNA

<400> 87
aggcacgggc aggtatgatg tttacgttta cggaagaacg gcactttatg gctacccgga 60

tggcattctt caccttcacg acccagcggg gtg                                93

<210> 88
<211> 81
<212> DNA
<213> Artificial sequence

<220>
<223> Synthetic DNA

<400> 88
gatccctaaa agttaatgtt tttcagatcc atgctgcaac gataacgacc atccgggaaa    60
cggctgcaca gcaggttcgg c                                              81

<210> 89
<211> 85
<212> DNA
<213> Artificial sequence

<220>
<223> Synthetic DNA

<400> 89
agagtttgcc ggcaccggtc cggtacccgc accgcactgc acrtcncgyt crcangcncc    60
ngtdatnacn gccatatgtt ggcgc                                          85

<210> 90
<211> 85
<212> DNA
<213> Artificial sequence

<220>
<223> Synthetic DNA

<400> 90
agagtttgcc ggcaccggtc cggtacccgc accgcactgc acrtcyctyt crcangcncc    60

ngtdatnacn gccatatgtt ggcgc 85

<210> 91
<211> 13
<212> DNA
<213> Artificial sequence

<220>
<223> Synthetic DNA

<400> 91
gcgccaacat atg 13

<210>92
<211> 23
<212> DNA
<213> Artificial sequence

<220>
<223> Synthetic DNA

<400> 92
acctgattgc ccgacattat cgc 23

<210> 93
<211> 23
<212> DNA
<213> Artificial sequence

<220>
<223> Synthetic DNA

<400> 93
caccctcatc agtgccaaca tag 23

<210> 94

<211> 20
<212> DNA
<213> Artificial sequence

<220>
<223> Synthetic DNA

<400> 94
taatacgact cactataggg        20

<210>95
<211> 32
<212> DNA
<213> Artificial sequence

<220>
<223> Synthetic DNA

<400> 95
atacatggat cctcaggtca tccttggaga gc        32

<210>96
<211> 100
<212> DNA
<213> Artificial sequence

<220>
<223> Synthetic DNA

<400> 96
tatggctcca gcgcatcgtg ggcgtggtgg ttggacttta aatagtgctg gatacttgtt    60
aggtccagtg ttacatttaa gcagcaaagc aaaccagggc                          100

<210>97
<211> 88
<212> DNA

<213> Artificial sequence

<220>
<223> Synthetic DNA

<400> 97
aggaagacag actcagctct tgagatccta gatctgtgga aggccatag atgggcttcct 60
tattctcggt ctccccgcat gacgtgag 88

<210> 98
<211> 90
<212> DNA
<213> Artificial sequence

<220>
<223> Synthetic DNA

<400> 98
ttgctttgct gcttaaatgt aacactggac ctaacaagta tccagcacta tttaaagtcc 60
aaccaccacg cccacgatgc gctggtgcca 90

<210> 99
<211> 100
<212> DNA
<213> Artificial sequence

<220>
<223> Synthetic DNA

<400> 99
gatcctcacg tcatgcgggg agaccgagaa taaggaagcc catctatggc cttccacaga 60
tctaggatct caagagctga gtctgtcttc ctgccctggt 100

<210>100
<211> 30
<212> DNA

<213> Artificial sequence

<220>
<223> Synthetic DNA

<400> 100
ggaattcgct cgcaccggta gaaaaaatgg   30

<210>101
<211> 36
<212> DNA
<213> Artificial sequence

<220>
<223> Synthetic DNA

<400> 101
ctagccattt tttctaccgg tgcgagcgaa ttccgc        36

<210>102
<211> 55
<212> DNA
<213> Artificial sequence

<220>
<223> Synthetic DNA

<400> 102
aattccgtcg tacaccatgg gcctccacct ccgcccctac cgtgtggggc tgctc        55

<210>103
<211> 55
<212> DNA
<213> Artificial sequence

<220>

<223> Synthetic DNA

<400> 103
ccgggagcag ccccacacgg taggggcgga ggtggaggcc catggtgtac gacgg 55

<210>104
<211> 55
<212> DNA
<213> Artificial sequence

<220>
<223> Synthetic DNA

<400> 104
aattccgtcg tacaccatgg gcctgcacct gcggccctac cgggtgggcc tgctg 55

<210>105
<211> 55
<212> DNA
<213> Artificial sequence

<220>
<223> Synthetic DNA

<400> 105
ccggcagcag gcccacccgg tagggccgca ggtgcaggcc catggtgtac gacgg 55

<210>106
<211> 55
<212> DNA
<213> Artificial sequence

<220>
<223> Synthetic DNA

<400> 106

aattccgtcg tacaccatgg gacttcacct tagaccttac agagtgggac ttctt 55

<210>107
<211> 55
<212> DNA
<213> Artificial sequence

<220>
<223> Synthetic DNA

<400> 107
ccggaagaag tcccactctg taaggtctaa ggtgaagtcc catggtgtac gacgg 55

<210>108
<211> 55
<212> DNA
<213> Artificial sequence

<220>
<223> Synthetic DNA

<400> 108
aattccgtcg tacaccatgg gtctacatct acgtccgtat cgtgtaggtc tacta 55

<210>109
<211> 55
<212> DNA
<213> Artificial sequence

<220>
<223> Synthetic DNA

<400> 109
ccggtagtag acctacacga tacggacgta gatgtagacc catggtgtac gacgg 55

<210>110

<211> 55
<212> DNA
<213> Artificial sequence

<220>
<223> Synthetic DNA

<400> 110
aattccgtcg tacaccatgg ggctacatct acgcccgtat cgcgtagggc tacta        55

<210>111
<211> 55
<212> DNA
<213> Artificial sequence

<220>
<223> Synthetic DNA

<400> 111
ccggtagtag ccctacgcga tacgggcgta gatgtagccc catggtgtac gacgg        55

<210>112
<211> 55
<212> DNA
<213> Artificial sequence

<220>
<223> Synthetic DNA

<400> 112
aattccgtcg tacaccatgg gtctacatct tagaccctat cgtgtaggac tgctt        55

<210>113
<211> 55
<212> DNA
<213> Artificial sequence

<220>
<223> Synthetic DNA

<400> 113
ccggaagcag tcctacacga tagggtctaa gatgtagacc catggtgtac gacgg        55

<210>114
<211> 55
<212> DNA
<213> Artificial sequence

<220>
<223> Synthetic DNA

<400> 114
aattccgtcg tacaccatgg gtctacatct tagaccctac cgagttggat tgctt        55

<210>115
<211>  55
<212> DNA
<213> Artificial sequence

<220>
<223> Synthetic DNA

<400> 115
ccggaagcaa tccaactcgg tagggtctaa gatgtagacc catggtgtac gacgg        55

<210> 116
<211> 23
<212> PRT
<213> Human

<400> 116
Trp Tyr Lys His Val Ala Ser Pro Arg Tyr His Thr Val Gly Arg Ala

1                   5                   10                  15
Ala Gly Leu Leu Met Gly Leu
                20              23

<210> 117
<211> 86
<212> PRT
<213> Human

<400> 117

Ala Val Ile Thr Gly Ala Cys Glu Arg Asp Val Gln Cys Gly Ala Gly
                    5                   10                  15
Thr Cys Cys Ala Ile Ser Leu Trp Leu Arg Gly Leu Arg Met Cys Thr
                20                  25                  30
Pro Leu Gly Arg Glu Gly Glu Glu Cys His Pro Gly Ser His Lys Val
            35                  40                  45
Pro Phe Phe Arg Lys Arg Lys His His Thr Cys Pro Cys Leu Pro Asn
        50                  55                  60
Leu Leu Cys Ser Arg Phe Pro Asp Gly Arg Tyr Arg Cys Ser Met Asp
65                  70                  75                  80
Leu Lys Asn Ile Asn Phe
                    85

<210> 118
<211> 146
<212> PRT
<213> Human

<400> 118
Pro Ala Leu Pro Glu Asp Gly Gly Ser Gly Ala Phe Pro Pro Gly His
1                   5                   10                  15
Phe Lys Asp Pro Lys Arg Leu Tyr Cys Lys Asn Gly Gly Phe Phe Leu
                20                  25                  30
Arg Ile His Pro Asp Gly Arg Val Asp Gly Val Arg Glu Lys Ser Asp
            35                  40                  45

Pro His Ile Lys Leu Gln Leu Gln Ala Glu Glu Arg Gly Val Val Ser
    50                  55                  60
Ile Lys Gly Val Ser Ala Asn Arg Tyr Leu Ala Met Lys Glu Asp Gly
65                  70                  75                  80
Arg Leu Leu Ala Ser Lys Ser Val Thr Asp Glu Cys Phe Phe Phe Glu
                85                  90                  95
Arg Leu Glu Ser Asn Asn Tyr Asn Thr Tyr Arg Ser Arg Lys Tyr Thr
            100                 105                 110
Ser Trp Tyr Val Ala Leu Lys Arg Thr Gly Gln Tyr Lys Leu Gly Ser
        115                 120                 125
Lys Thr Gly Pro Gly Gln Lys Ala Ile Leu Phe Leu Pro Met Ser Ala
    130                 135                 140
Lys Ser
145 146

<210> 119
<211> 180
<212> DNA
<213> Artificial sequence

<220>
<223> Synthetic DNA

<400> 119
gctccagcgc atcgtgggcg tggtggttgg actttaaata gtgctggtta cctcctgggt    60
cctgtcctcc acctttcctc aaaggccaac cagggcagga agacagactc agctcttgag   120
atcctagacc tgtggaaggc catagatggg ctcccttatt cccgctctcc aaggatgacc   180

<210> 120
<211> 228
<212> DNA
<213> Artificial sequence

<220>
<223> Synthetic DNA

<400> 120
gatcgatttc acgcaactag tgctgactgc tgcatctcct acaccccacg aagcatcccg   60
tgttcactcc tggagagtta ctttgaaacg aacagcgagt gctccaagcc gggtgtcatc  120
ttcctcacca agaaggggcg acgtttctgt gccaacccca gtgataagca agttcaggtt  180
tgcatgagaa tgctgaagct ggacacacgg atcaagacca ggaagaat              228

<210> 121
<211> 258
<212> DNA
<213> Artificial sequence

<220>
<223> Synthetic DNA

<400> 121
gcggtgatta ccggtgcgtg cgaacgtgat gtgcagtgcg gtgcgggtac ctgctgcgcg   60
attagcctgt ggctgcgtgg tctgcgtatg tgcaccccgc tgggtcgtga aggtgaagaa  120
tgccatccgg gtagccataa agtgccgttc ttccgtaaac gtaaacatca tacctgcccg  180
tgcctgccga acctgctgtg cagccgtttc ccggatggtc gttatcgttg cagcatggat  240
ctgaaaaaca ttaacttt                                                258

<210> 122
<211> 180
<212> DNA
<213> Artificial sequence

<220>
<223> Synthetic DNA

<400> 122
gctccagcgc atcgtgggcg tggtggttgg actttaaata gtgctggata cttgttaggt   60
ccagtgttac atttaagcag caaagcaaac cagggcagga agacagactc agctcttgag  120
atcctagatc tgtggaaggc catagatggg cttccttatt ctcggtctcc ccgcatgacg  180

## Description

### TECHNICAL FIELD

The present invention relates to a process for producing proteins. More specifically, the present invention relates to a process for producing a protein of interest in a prokaryotic or eukaryotic cell, as well as a process for preparing a DNA that encodes a protein of interest and is suitable for expression in a prokaryotic or eukaryotic cell.

### BACKGROUND ART

Various attempts have been made to produce useful proteins in procaryotic or eukaryotic cells using recombinant DNA technology. It is very difficult to produce proteins with relatively high molecular weights by chemical synthesis. On the other hand, proteins with low molecular weights can be produced efficiently in the form of a fusion protein using recombinant DNA technology. Expression of useful proteins using prokaryotes, especially *Escherichia coli,* can be performed rapidly and cheaply. However, depending on the type of the protein to be expressed by recombinant DNA technology, there are some cases where expression is hardly recognized or, even if expressed, the expression level is very low.

When a protein of interest is expressed in a heterologous organism (e.g., a protein of a eukaryote is expressed in a prokaryote), codon usage is different; or when a secretion protein from eukaryotes is expressed in prokaryotes, only the mature protein moiety is expressed in many cases excluding the signal peptide moiety at which translation originally starts in eukaryotes. Thus, there arise such problems that a protein highly expressed in eukaryotes is not expressed in prokaryotes or, even if expressed, the expression level is extremely low. Further, even when a protein of interest is expressed in a host similar to the original organism in species (e.g., a human-derived protein is expressed in an animal cell), the expression level may differ greatly depending on the type of the protein. Development of a technology to allow the high expression of proteins of interest in prokaryotic and eukaryotic cells using recombinant DNA technology is extremely useful from an industrial point of view.

### DISCLOSURE OF THE INVENTION

The present inventors have found that the difference in expression levels when a useful protein is produced in a prokaryotic or eukaryotic cell using recombinant DNA technology is mainly attributable to the sequence of the DNA encoding the protein. As a result of further researches, the inventors have achieved the present invention.

The present invention relates to:
(1) A process of producing a DNA that encodes a protein of interest and is suitable for expression, comprising the following steps:
   1) preparing recombinant vectors by using a mixture of a plurality of DNAs encoding the full length or a partial amino acid sequence of the protein of interest and integrating each of the DNAs into a vector retaining a promoter DNA functional in a host cell and a reporter gene so that the DNA is located downstream of the promoter DNA and upstream of the reporter gene,
   2) transforming the host cell with the recombinant vectors,
   3) culturing the resultant transformants,
   4) measuring the expression of the reporter gene, and
   5) preparing a DNA that comprises the same nucleotide sequence as that of the DNA encoding the full length or the partial amino acid sequence of the protein of interest possessed by a transformant in which a high expression of the reporter gene has been confirmed and that encodes the protein of interest;
(2) The production process of (1) above, wherein the full length or a partial amino acid sequence of the protein of interest is the full length of the protein of interest or a partial amino acid sequence comprising the N-terminal of the protein of interest;
(3) A process of producing a protein of interest or a salt thereof, comprising the following steps:
   1) preparing recombinant vectors by using a mixture of a plurality of DNAs encoding the full length or a partial amino acid sequence of the protein of interest and integrating each of the DNAs into a vector retaining a promoter DNA functional in a host cell and a reporter gene so that the DNA is located downstream of the promoter DNA and upstream of the reporter gene,
   2) transforming the host cell with the recombinant vectors,
   3) culturing the resultant transformants,
   4) measuring the expression of the reporter gene,
   5) preparing a recombinant vector comprising a DNA that comprises the same nucleotide sequence as that of the DNA encoding the full length or the partial amino acid sequence of the protein of interest possessed by a transformant in which a high expression of the reporter gene has been confirmed and that encodes the protein of interest,
   6) transforming a host cell with the recombinant vector, and
   7) culturing the resultant transformant;
(4) The production process of (2) above, wherein the full length or a partial amino acid sequence of the protein of interest is the full length of the protein of interest or a partial amino acid sequence comprising the N-terminal of the protein of interest;
(5) A process of producing a DNA that encodes a eukaryote-derived protein of interest and is suitable for expression in a prokaryote, comprising the following steps:
   1) preparing recombinant vectors by using a mixture of a plurality of DNAs encoding an N-terminal amino acid sequence of the eukaryote-derived protein of interest and integrating each of the DNAs into a vector retaining a promoter DNA functional in prokaryotes and a reporter gene so that the DNA is located downstream of the promoter DNA and upstream of the reporter gene,
   2) transforming the prokaryote with the recombinant vectors,
   3) culturing the resultant prokaryotic transformants,
   4) measuring the expression of the reporter gene, and
   5) preparing a DNA that comprises the same nucleotide sequence as that of the DNA encoding the N-terminal amino acid sequence of the protein of interest possessed by a transformant in which a high expression of the reporter gene has been confirmed and that encodes the protein of interest;
(6) A process of producing a eukaryote-derived protein of interest or a salt thereof in a prokaryote, comprising the following steps:
   1) preparing recombinant vectors by using a mixture of a plurality of DNAs encoding an N-terminal amino acid sequence of the eukaryote-derived protein of interest and integrating each of the DNAs into a vector retaining a promoter DNA functional in prokaryotes and a reporter gene so that the DNA is located downstream of the promoter DNA and upstream of the reporter gene,
   2) transforming the prokaryote with the recombinant vectors,
   3) culturing the resultant prokaryotic transformants,
   4) measuring the expression of the reporter gene,
   5) preparing a recombinant vector comprising a DNA that comprises the same nucleotide sequence as that of the DNA encoding the N-terminal amino acid sequence of the protein of interest possessed by a transformant in which a high expression of the reporter gene has been confirmed and that encodes the protein of interest,
   6) transforming the prokaryote with the recombinant vector, and
   7) culturing the resultant prokaryotic transformant;
(7) A DNA obtained by the production process of (1) above that comprises the same nucleotide sequence as that of the DNA encoding the full length or a partial amino acid sequence of a protein of interest possessed by a transformant in which a high expression of the reporter gene has been confirmed and that encodes the full length of the protein of interest;
(8) A non-wild type DNA obtained by the production process of (1) above encoding the full length of a protein of interest, wherein the DNA is composed of a DNA encoding a partial amino acid sequence of the protein of interest possessed by a transformant in which a high expression of the reporter gene has been confirmed and a DNA(s) encoding the remaining amino acid sequence(s) of the protein ligated to the 3' end or/and 5' end of the DNA;
(9) A DNA having the nucleotide sequence as shown in SEQ ID NO: 119, SEQ ID NO: 120, SEQ ID NO: 121 or SEQ ID NO: 122;
(10) A recombinant vector comprising the DNA of any one of (7) to (9) above;
(11) A transformant transformed with the recombinant vector of (10) above; and
(12) A process of producing a protein of interest or a salt thereof, comprising culturing the transformant of (11) above and allowing the transformant to produce the protein of interest.

### BRIEF DESCRIPTION OF THE DRAWINGS

Fig. 1 shows a construction diagram for plasmid pGFPNP.
Fig. 2 shows a construction diagram for plasmid pGFPNPRG.
Fig. 3 shows a construction diagram for plasmid pTFCRGAL.
Fig. 4 shows a construction diagram for plasmid pTFCS4.
Fig. 5 shows the growth curves of *E. coli* MM294 (DE3)/pTFCRGAL and *E*. *coli* MM294 (DE3)/pTFCS4.
Fig. 6 shows the results of analysis of the products from *E*. *coli* MM294 (DE3)/pTFCRGAL and *E. coli* MM294 (DE3)/pTFCS4.
Fig. 7 shows a construction diagram for plasmid pGFPNPMP.
Fig. 8 shows a construction diagram for plasmid pNPHGHlacts.
Fig. 9 shows a construction diagram for plasmid pNPMPIF.
Fig. 10 shows a construction diagram for plasmid pNPMPIFNO.
Fig. 11 shows the fluorescence intensities of the *E. coli* clones obtained in Example 6.
Fig. 12 shows the growth curves and MPIF1Δ23 expression levels of *E. coli* JM109/pNPMPIF retaining plasmid pNPMPIF having the wild-type nucleotide sequence and *E. coli* JM109/pNPMPIFNO retaining plasmid pNPMPIFNO having a modified nucleotide sequence. In this Figure, mark ○ represents the growth curve, and mark ● represents the MPW1Δ23 expression level.
Fig. 13 shows a construction diagram for plasmid pKANP.
Fig. 14 shows the amino acid sequence and the wild-type nucleotide sequence of ZAQ-1 ligand.
Fig. 15 is a construction diagram for plasmid pTCTZAQN.
Fig. 16 shows the amino acid sequence, the wild-type nucleotide sequence and a nucleotide sequence comprising highly frequently used codons, of ZAQ-1 ligand.
Fig. 17 shows the synthetic DNAs used for the construction of a ZAQ-1 structural gene using highly frequently used codons.
Fig. 18 shows a construction diagram for plasmid pTCIIZAQS.
Fig. 19 shows a construction diagram for plasmid pKANPZAQ.
Fig. 20 shows the nucleotide sequence obtained in Example 14.

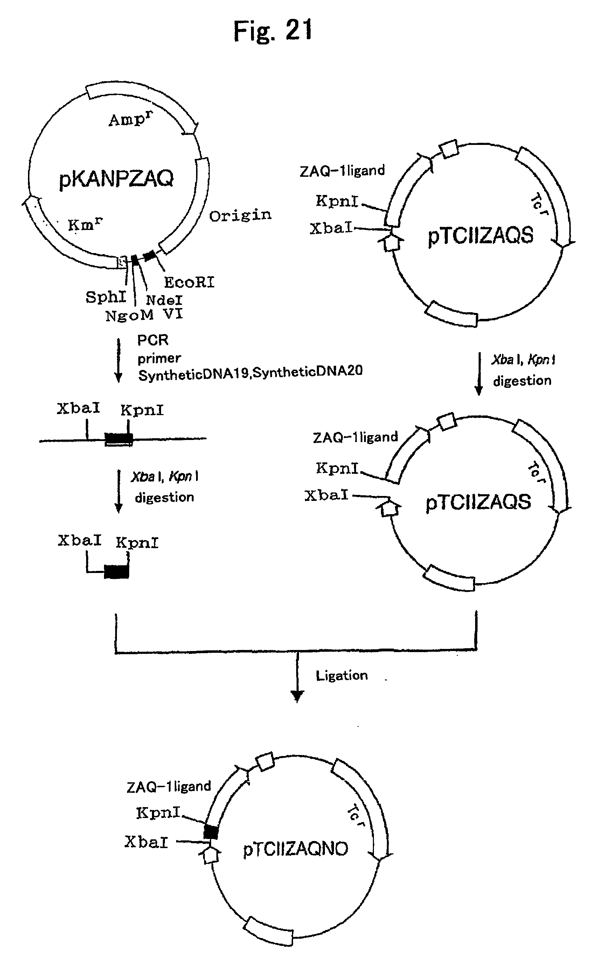

Fig. 21 shows a construction diagram for plasmid pTCIIZAQNO.
Fig. 22 shows the results of the expression analysis carried out in Example 16.
Fig. 23 shows a construction diagram for plasmid pTCRGDE.
Fig. 24 shows a construction diagram for plasmid pTCRGNODE.
Fig. 25 shows the rat GALP full length-optimized nucleotide sequence obtained in Example 19.
Fig. 26 shows a construction diagram for plasmid pTCRGTODE.
Fig. 27 shows the results of SDS-PAGE obtained in Example 21.
Fig. 28 shows a construction diagram for plasmid pQBI25-3.
Fig. 29 shows the nucleotide sequences encoding an N-terminal portion of LLPL used in Example 23.
Fig. 30 shows the results of the fluorescence measurement carried out in Example 24.
Fig. 31 shows the results of the analysis carried out in Example 25.

### BEST MODES FOR CARRYING OUT THE INVENTION

Codons encoding the individual amino acids composing proteins are as described below.

Phe: TTY (TTT or TTC)

Leu: TTR or CTN (TTA, TTG, CTT, CTC, CTA or CTG)

Ile: ATH (ATT, ATC or ATA)

Met: ATG

Val: GTN (GTT, GTC, GTA or GTG)

Ser: TCN or AGY (TCT, TCC, TCA, TCG, AGT, AGC)

Pro: CCN (CCT, CCC, CCA or CCG)

Thr: ACN (ACT, ACC, ACA or ACG)

Ala: GCN (GCT, GCC, GCA or GCG)

Tyr: TAY (TAT or TAC)

His: CAY (CAT or CAC)

Gln: CAR (CAA or CAG)

Asn: AAY (AAT or AAC)

Lys: AAR (AAA or AAG)

Asp: GAY (GAT or GAC)

Glu: GAR (GAA or GAG)

Cys: TGY (TGT or TGC)

Trp: TGG

Arg: CGN or AGR (CGT, CGC, CGA, CGG, AGA or AGG)

Gly: GGN (GGT, GGC, GGA or GGG)

Stop codon: TAR or TGA (TAA, TAG or TGA)

In the present specification, a protein of interest is written with the N-terminal (amino terminal) on the left end and the C-terminal (carboxyl terminal) on the right end according to the convention in peptide representation.

As a protein of interest, a protein that may be used in drugs, reagents, raw materials, feeds, etc. is used. More specifically, a eukaryote-derived protein having physiological activity is used as a protein of interest. Specific examples of such a protein include growth factors, interleukins, interferons, chemokines, and physiologically active peptides [e.g. KISS-1 peptide (WO 00/24890), RFRP-1 (WO 00/29441), apelin (WO 99/33976), PrRP (WO 97/24436), GALP (WO 99/48920), GPR8 ligand, ZAQ-1 ligand, angiotensin, bradykinin, calcitonin, conantokin, corticotropin-releasing factor, dynorphin, endorphin, enkephalin, galanin, GALP (ligand peptide for rat galanin receptor; WO 99/48920), MPIF-1Δ23 (myeloid progenitor inhibitory factor-1Δ23; WO 95/17092), gastrin, glucagon, growth hormone-releasing factor, FMRF-amide, neurokinin, neuromedin, neuropeptide, nociceptin, nocistatin, orexin-B, selectin, substance P, urocortin, VIP, PACAP, ACTH, various opioids].

As GPR8 ligand mentioned above, any polypeptide may be used as long as it has ligand activity for a 7-transmembrane type receptor protein GPR8 (O'Dowd, B.F. et al., Genomics, 28:84-91, 1995) (e.g. GPR8 binding activity) or cell stimulating activity against GPR-expressing cells (e.g. activities to promote arachidonate liberation, acetylcholine liberation, intracellular Ca²⁺ liberation, intracellular cAMP production, intracellular cGMP production, inositol phosphate production, cellular membrane voltage variation, phposphorylation of intracellular proteins, c-fos activation, pH lowering, GTP γ S binding activity, etc.). Preferably, a peptide having the amino acid sequence as shown in SEQ ID NO: 116 may be used.

As ZAQ-1 ligand mentioned above, ligands for ZAQ receptor may be used. Specifically, a peptide having the amino acid sequence as shown in SEQ ID NO: 117 or the like may be used.

These proteins may be used alone in their intrinsic forms. Alternatively, they may be used in a form where another eukaryote-derived protein, a prokaryote-derived protein or a tag such as polyhistidine is fused to the C-terminal.

As a eukaryote-derived protein to be fused to the C-terminal, a fibroblast growth factor (aFGF, bFGF, etc.) or a mutein thereof, or a part (fragment) of such a factor or its mutein (e.g. bFGF CS23 mutein, etc.) may be used preferably.

As bFGF CS23 mutein, a protein having the amino acid sequence as shown in SEQ ID NO: 118 may be used, for example.

A protein of interest may be in the form of a salt. As a salt of a protein of interest, a physiologically acceptable acid-addition salt is particularly preferable. Examples of such salts useful in the invention include salts formed with inorganic acids (e.g. hydrochloric acid, phosphoric acid, hydrobromic acid or sulfuric acid) and salts formed with organic acids (e.g. acetic acid, formic acid, propionic acid, fumaric acid, maleic acid, succinic acid, tartaric acid, citric acid, malic acid, oxalic acid, benzoic acid, methanesulfonic acid or benzenesulfonic acid).

As a reporter gene, such a gene may be used for which the presence or absence of expression can be confirmed easily. Specific examples of reporter genes include genes of fluorescent proteins such as green fluorescent protein (GFP) gene, enzyme genes such as alkali phosphatase gene, β-galactosidase gene, dihydrofolate reductase gene, and drug resistance genes such as kanamycin resistance gene, chloramphenicol resistance gene, neomycin resistance gene, hygromycin resistance gene. One or a plurality (preferably, two or three) of these genes may be used. Among all, green fluorescent protein (GFP) gene is preferable.

By using a plurality of codons encoding a single amino acid, a mixture of a plurality of DNAs encoding the same amino acid sequence is obtained. A plurality of DNAs encoding the full length or a partial amino acid sequence of a protein of interest can be synthesized chemically according to conventional methods using mixed bases encoding amino acids composing the relevant amino acid sequence.

The full-length amino acid sequence of a protein of interest refers to an amino acid sequence encoding the full length of the protein of interest.

A partial amino acid sequence of a protein of interest refers to any partial amino acid sequence of the full-length amino acid sequence of the protein of interest. Usually, an amino acid sequence consisting of about 5 or more, preferably about 5-50, more preferably about 5-15, still more preferably about 5-10 amino acid residues is used. A preferable partial amino acid sequence is a partial amino acid sequence encoding an N-terminal portion of the full-length amino acid sequence of a protein of interest; usually, an amino acid sequence consisting of about 5 or more, preferably about 5-50, more preferably about 5-15, still more preferably about 5-10 amino acid residues counted from the N-terminal is preferably used. In particular, an amino acid sequence spanning from the N-terminal to about the 15th amino acid residue, preferably from the N-terminal to about the 10th amino acid residue is used.

It is preferable to provide DNA fragments with different restriction sites at the 5' ends and the 3' ends in order to avoid ligation among DNA fragments.

By using a mixture of a plurality of DNAs and integrating each of the DNA into a vector retaining a promoter DNA functional in a host cell and a reporter gene downstream of the promoter DNA and upstream of the reporter gene, a mixture of a plurality of recombinant vectors for confirming the expression of a reporter gene can be obtained.

By transforming a host cell with the resultant mixture of recombinant vectors, a plurality of transformants are obtained.

The resultant mixture of a plurality of transformants is cultured, followed by confirmation of the presence or absence of the expression of the reporter gene.

When the host is a prokaryote, the expression levels of the reporter gene in individual transformants can be easily confirmed by coating the mixture of transformants on a solid medium, such as agar medium, and culturing them. Also, a single clone can be easily isolated. The expression levels of the reporter gene can be measured by determining the amounts of the reporter gene product. For example, when GFP gene is used as a reporter gene, the amount of GFP can be determined by exciting at the wavelength of 350-500 and measuring the fluorescence intensity at 450-550 nm. When a drug resistance gene is used as a reporter gene, only those transformants with high expression of the reporter gene can be grown by coating a mixture of transformants on a solid medium (such as agarose medium) containing the relevant drug at an appropriate concentration. When an enzyme gene is used as a reporter gene, enzyme activities produced by transformants can be measured by conventional methods.

When the host is a eukaryotic cell, if GFP gene is used as a reporter gene, it is possible to obtain those clones with high expression of GFP gene by culturing a mixture of transformants and utilizing the cell sorting function of a flowcytometer. Also, it is possible to obtain a single cell by limiting dilution, culture the cell and then determining the expression level of the reporter gene by measuring the fluorescence intensity or the activity of the enzyme produced by the transformant according to known methods.

When the host is a prokaryote, a recombinant vector can be extracted by conventional methods from the transformant (clone) in which expression of the reporter gene has been confirmed. By digesting the extracted recombinant vector with appropriate restriction enzymes, a DNA fragment can be obtained that is suitable for the production of the protein of interest in a prokaryote and encodes the full length or a partial amino acid sequence of the protein of interest. If necessary, the nucleotide sequence of the resultant DNA fragment may be confirmed. Further, by amplifying by colony PCR a DNA encoding an N-terminal amino acid sequence of the protein of interest from a transformant (clone) in which expression of the reporter gene has been confirmed and then digesting the DNA with appropriate restriction enzymes, a DNA fragment can be obtained that is suitable for the production of the protein of interest in a prokaryote and encodes the N-terminal amino acid sequence of the protein of interest. If necessary, the nucleotide sequence of the resultant DNA fragment may be confirmed. Also, a DNA comprising the same nucleotide sequence as that of the DNA fragment obtained and encoding the ful length or a partial amino acid sequence of the protein of interest may be synthesized chemically.

When the host is a eukaryotic cell, mRNA may be extracted by conventional methods from the transformant (clone) in which expression of the reporter gene has been confirmed. By preparing cDNA from the extracted mRNA using a reverse transcriptase, a DNA fragment encoding the full length or a partial amino acid sequence of a protein of interest can be obtained. If necessary, the nucleotide sequence of the resultant DNA fragment may be confirmed. Further, by extracting DNA from the transformant (clone) by conventional methods, amplifying the DNA by PCR and then digesting with appropriate restriction enzymes, it is possible to obtain a DNA fragment that is suitable for production of the protein of interest and encodes an N-terminal amino acid sequence of the protein of interest. If necessary, the nucleotide sequence of the resultant DNA fragment may be confirmed. Also, a DNA comprising the same nucleotide sequence as that of the DNA fragment obtained and encoding the full length or a partial amino acid sequence of the protein of interest may be synthesized chemically.

The DNA thus obtained according to the invention encoding the full length or a partial amino acid sequence of a protein of interest is sometimes referred to as "the resultant DNA encoding the full length of a protein of interest" or the like.

For the production of a protein of interest, a DNA sequence obtained from a clone exhibiting a higher expression of a reporter gene than a clone retaining the wild-type DNA (e.g. cDNA or genomic DNA) sequence encoding the full length or a partial amino acid sequence of the protein of interest is used. Usually, a DNA sequence obtained from a clone exhibiting the highest expression of the reporter gene is used.

A DNA encoding the full-length amino acid sequence of the protein of interest, or a DNA having a DNA sequence encoding a partial amino acid sequence (e.g. N-terminal amino acid sequence) of the protein of interest and a DNA sequence downstream thereof encoding the remaining part of the protein of interest (e.g. DNA encoding an amino acid sequence spanning from the center to the C-terminal) is prepared. The DNA encoding the full length or a partial amino acid sequence of the protein of interest may be a DNA fragment obtained from the clone exhibiting the highest expression of the reporter gene or a chemically synthesized DNA fragment. The DNA encoding the remaining part of the protein of interest may be a cDNA, genomic DNA or synthesized DNA fragment. As long as the DNA fragment encodes the remaining part of the protein of interest, its nucleotide sequence may be different from the wild-type DNA sequence.

A DNA encoding the full length of the protein of interest can be prepared by ligating the above-obtained DNA fragment encoding a partial amino acid sequence of the protein of interest to the DNA fragment encoding the remaining part of the protein of interest. The ligation of these DNA fragment can be carried out according to known methods.

As a DNA suitable for industrial production of a protein of interest obtainable by the process of production of the present invention, the following examples may be given:
(1) a non-wild type DNA that comprises the same nucleotide sequence as that of the DNA obtained by the process of the invention encoding the full length or a partial amino acid sequence of the protein of interest contained in a transformant where high expression of the reporter gene has been confirmed, and encodes the full length of the protein of interest;
(2) a non-wild type DNA prepared by ligating to the 3' end or/and 5' end of the DNA obtained by the process of the invention encoding a partial amino acid sequence of the protein of interest contained in a transformant where high expression of the reporter gene has been confirmed a DNA/DNAs encoding the remaining amino acid sequence of the protein of interest.

A non-wild type DNA encoding the full length of a protein of interest means that this DNA does not comprise a sequence completely identical to the sequence of the naturally occurring DNA encoding the full length of the protein of interest.

More specifically, the following DNAs may be given as examples.
(I) In the case of rat GALP
   A DNA having the nucleotide sequence as shown in SEQ ID NO: 119.
(2) In the case of MPIF-1Δ23
   A DNA having the nucleotide sequence as shown in SEQ ID NO: 120.
(3) In the case of ZAQ-1 ligand
   A DNA having the nucleotide sequence as shown in SEQ ID NO: 121.
(4) In the case of rat GALP
   A DNA having the nucleotide sequence as shown in SEQ ID NO: 122.

A transformant capable of expressing the protein of interest can be obtained by integrating the resultant DNA encoding the full length of the protein of interest in a vector retaining a promoter functional in a host cell downstream of the promoter and then transforming a prokaryote with the resultant recombinant vector.

The protein of interest can be produced by culturing the resultant transformant.

Examples of vectors useful in the invention include plasmids derived from *Escherichia coli* (e.g. pBR322, pBR325, pUC12, and pUC13); plasmids derived from *Bacillus subtilis* (e.g. pUB110, pTP5 and pC194); plasmids derived from yeast (e.g. pSH19 and pSH15); bacteriophages such as λ-phage; animal viruses such as retrovirus, vaccinia virus, baculovirus; and other vectors such as pA1-11, pXT1, pRc/CMV, pRc/RSV, pcDNAI/Neo and so on.

Any promoter may be used in the invention as long as it is appropriate for the host that will be used for expressing a gene of interest. When the host is an animal cell, examples of promoters useful in the invention include SR α promoter, SV40 promoter, LTR promoter, CMV promoter, HSV-TK promoter and so on.

Among these promoters, CMV promoter, SR α promoter or the like is preferably used. When the host is a bacteria belonging to the genus *Escherichia,* trp promoter, lac promoter, recA promoter, λPL promoter, lpp promoter or the like is preferably used. When the host is a bacterium belonging to the genus *Bacillus*, SPO1 promoter, SPO2 promoter, penP promoter or the like is preferably used. When the host is a yeast, PHO5 promoter, PGK promoter, GAP promoter, ADH promoter, or the like is preferably used. When the host is an insect cell, polyhedrin promoter, P10 promoter or the like is preferably used.

The expression vectors may, if desired, further comprise enhancers, splicing signals, polyadenylation signals, selective markers, SV40 replication origin (hereinafter, sometimes abbreviated to "SV40 ori") and the like. Examples of selective markers useful in the invention include dihydrofolate reductase (hereinafter, sometimes abbreviated to "dhfr") gene [methotorexate (MTX) resistance], ampicillin resistance gene (hereinafter, sometimes abbreviated to "Amp^{r}"), neomycin resistance gene (hereinafter, sometimes abbreviated to "Neo^{r}": G418 resistance) and the like. In particular, when CHO (dhfr) cells are used in combination with dhfr gene as a selective marker, transformants with a gene of interest can be selected even in a thymidine-free medium.

A reporter gene can be inserted into the vector down stream of the promoter DNA according to known methods. For insertion of a DNA encoding the full length of a partial amino acid sequence of a protein of interest, usually, one or a plurality (preferably two or three) restriction enzyme recognition sites may be provided downstream of the promoter DNA and upstream of the reporter gene.

If desired, vectors may contain selective markers. Examples of selective markers useful in the invention include ampicillin resistance gene (Amp^{r}) and tetracycline resistance gene.

Transformants can be prepared by transforming a host with recombinant vectors into which a DNA encoding the full length or a partial amino acid sequence of the protein of interest has been inserted.

Examples of hosts useful for this purpose include bacteria belonging to the genus *Escherichia*, bacteria belonging to the genus *Bacillus,* yeasts, insect cells, insects, and animal cells.

Specific examples of bacteria belonging to the genus *Escherichia* useful in the invention include *E. coli* K12 DH1 [Proc. Natl. Acad. Sci. USA, Vol. 60, 160 (1968)], JM103 [Nucleic Acids Research, Vol. 9, 309 (1981)], JA221 [Journal of Molecular Biology, Vol. 120, 517 (1978)]), HB101 [Journal of Molecular Biology, Vol, 41, 459 (1969)] and C600 [Genetics, Vol. 39, 440 (1954)].

Specific examples of bacteria belonging to the genus *Bacillus* useful in the invention include *B. subtilis* MI114 [Gene, Vol. 24, 255 (1983)] and 207-21 [Journal of Biochemistry, Vol. 95, 87 (1984)].

Specific examples of yeasts useful in the invention include *Saccharomyces cerevisiae* AH22, AH22R⁻, NA87-11A, DKD-5D and 20B-12, *Schizosaccharomyces pombe* NCYC1913 and NCYC2036, and *Pichia pastoris*.

Specific examples of insect cells useful in the invention include, when the virus used is AcNPV, a cell line derived from larvae of *Spodoptera frugiperda* (Sf cells), MG1 cells derived from the midgut of *Trichoplusia ni,* High Five™ cells derived from eggs of *Trichoplusia ni, Mamestra brassicae*-derived cells and *Estigmena acrea*-derived cells. When the virus used is BmNPV, insect cells such as a silkworm-derived cell line (Bombyx mori N cells; BmN cells) may be used. Specific examples of Sf cells useful in the invention include Sf9 cells (ATCC CRL 1711) and Sf21 cells [both disclosed in Vaughn J. L. et al., In Vivo, 13, 213-217 (1977)).

Specific examples of insects useful in the invention include larvae of silkworm (Maeda et al., Nature, 315, 592 (1985)).

Specific examples of animal cells useful in the invention include a simian cell COS-7, Vero cells, a Chinese hamster cell CHO (hereinafter, abbreviated to "CHO cells"), a sdhfr gene-deficient Chinese hamster cell CHO (hereinafter, abbreviated to "CHO(dhfr⁻) cells"), mouse L cells, mouse AtT-20 cells, mouse myeloma cells, rat GH3 cells, and human FL cells.

Transformation of bactera belonging to the genus *Escherichia* can be performed in accordance with methods disclosed, for example, in Proc. Natl. Acad. Sci. USA, Vol. 69, 2110 (1972) and Gene, Vol. 17, 107 (1982). Transformation of bacteria belonging to the genus *Bacillus* can be performed in accordance with methods disclosed, for example, in Molecular & General Genetics, Vol. 168, 111 (1979).

Transformation of yeasts can be performed in accordance with methods disclosed, for example, in Methods in Enzymology, 194, 182-187(1991) and Proc. Natl. Acad. Sci. USA, Vo. 75, 1929 (1978).

Transformation of insect cells or insects can be performed in accordance with methods disclosed, for example, in Bio/Technology, 6, 47-55 (1988).

Transformation of animal cells can be performed by methods disclosed, for example, in Cell Engineering, Separate Vol. 8, New Cell Engineering Experiment Protocol, 263-267 (1995) (Shujunsha Co.) and Virology, Vol. 52, 456 (1973).

As a medium to culture transformants obtained from *Escherichia* or *Bacillus* bacteria as hosts, a liquid medium is appropriate. The medium is allowed to contain carbon sources, nitrogen sources, minerals, and so on which are necessary for the growth of the transformant. As carbon sources, glucose, dextrin, soluble starch, sucrose or the like may be enumerated. As nitrogen sources, organic or inorganic substances such as ammonium salts, nitrates, corn steep liquor, peptone, casein, meat extract, bean cake, potato extract, or the like may be enumerated. As minerals, calcium chloride, sodium dihydrogen phosphate, magnesium chloride, or the like may be enumerated. Further, yeast, vitamins, growth-promoting factors, etc. may also be added to the medium. Preferable pH of the medium is about 5-8.

As a medium to culture *Escherichia* bacteria, M9 medium containing glucose and casamino acid [Miller, Journal of Experiments in Molecular Genetics, 431-433, Cold Spring Harbor Laboratory, New York, (1972)] is preferable, for example. If necessary, drugs such as 3β-indolyl acrylic acid can be added to the medium to improve the efficiency of the promoter. When the host is an *Escherichia* bacterium, the transformant is cultured usually at about 15-43 °C for about 3-24 hours. If necessary, aeration and stirring may be applied.

When the host is a *Bacillus* bacterium, the transformant is cultured usually at about 30-40 °C for about 6-24 hours. If necessary, aeration and stirring may also be applied.

As a medium to culture transformants obtained from yeasts as hosts, a medium such as Burkholder minimum medium [Bostian, K.L. et al., Proc. Natl. Acad. Sci. USA, Vol. 77, 4505 (1980)] or SD medium containing 0.5% casamino acid [Bitter, G.A. et al., Proc. Natl. Acad. Sci. USA, Vol. 81, 5330 (1984)] may be used, for example. It is preferable that the pH of the medium be adjusted to about 5-8. The transformant is cultured usually at about 20-35 °C for about 24-72 hours. If necessary, aeration and stirring may be applied.

As a medium to culture transformants obtained from insect cells or insects as hosts, Grace's Insect Medium [Grace, T.C.C., Nature, 195, 788 (1962)] supplemented with additives such as inactivated 10% bovine serum may be used, for example. It is preferable that the pH of the medium be adjusted to about 6.2-6.4. The transformant is cultured usually at about 27 °C for about 3-5 days. If necessary, aeration and stirring may be applied.

As a medium to culture transformants obtained from animal cells as hosts, examples of useful media include MEM medium [Science, Vol. 122, 501 (1952)], DMEM medium [Virology, Vol. 8, 396 (1959)], RPMI 1640 medium [Journal of the American Medical Association, Vol. 199, 519 (1967)] and 199 medium [Proceedings of the Society of the Biological Medicine, Vol. 73, 1 (1950)] each containing about 5-20% fetal calf serum. Preferable pH of the medium is about 6 to about 8. The transformant is cultured usually at about 30-40 °C for about 15-60 hours. If necessary, aeration and stirring may be applied.

Separation and purification of the polypeptide from the resultant culture can be carried out, for example, according to the methods described below.

For extraction of the protein of interest from cultured microorganisms or cells, the microorganism cells are harvested by known methods after the cultivation, suspended in a suitable buffer, and disrupted by sonication or by lysozyme and/or freezing and thawing, etc. Then, a crude extract of the polypeptide is obtained by centrifugation or filtration. The buffer may contain a protein denaturing agent such as urea or guanidine hydrochloride, or a surfactant such as Triton X-100™. If the protein of interest is secreted into the culture broth, the supernatant is separated from the microorganisms or cells after completion of the cultivation and collected by known methods.

Purification of the protein of interest contained in the thus obtained culture supernatant or extract can be performed by an appropriate combination of known methods for separation and purification. These known methods include methods utilizing solubility (such as salting out or sedimentation with solvents), methods mainly utilizing difference in molecular weight (such as dialysis, ultrafiltration, gel filtration and SDS-polyacrylamide gel electrophoresis), methods utilizing difference in electric charge (such as ion-exchange chromatography), methods utilizing specific affinity (such as affinity chromatography), methods utilizing difference in the hydrophobicity (such as reversed-phase high-performance liquid chromatography), and methods utilizing difference in isoelectric point (such as isoelectric electrophoresis).

When the thus obtained protein of interest is a free protein, the protein can be converted into the above-described salt by known methods or methods based thereon. On the contrary, when the protein of interest is obtained in a salt form, the salt can be converted into a free protein or another salt according to known methods or methods based thereon.

The protein of interest produced by the transformant can be arbitrarily modified or a part thereof can be removed therefrom by using an appropriate protein modification enzyme before or after the purification. Examples of such enzymes include trypsin, chymotrypsin, arginyl endopeptidase and protein kinase.

The extracted or separated/purified protein of interest is subjected to refolding, if necessary. Refolding can be performed by known methods described, for example, in Folding of Proteins, R.H. Pain (Ed.), 245-279 (1995) published by Springer-Verlag Tokyo or methods based thereon. Refolding can be performed, for example, by one-step or multi-step dilution in a buffer with no extraction agents (e.g. chaotropic solubilizers such as guanidine hydrochloride and urea, surfactants such as n-lauryl methyl glycine and SDS) or with extraction agents at low concentrations, dialysis with a semipermeable membrane, or replacement of a buffer using gel filtration. In order to prevent the aggregation of the protein of interest, arginine, polyethylene glycol, neutral surfactants, etc. may be added. For the formation of disulfide bonds in the protein, air oxidation, an oxidation-reduction buffer system, etc. may be employed. Examples of oxidation-reduction buffers include those based on glutathione, cysteine, dithiothreitol, 2-mercaptoethanol or cysteamine.

Further, the Met at the N-terminal of the protein of interest may be removed based on the method described in EP 0812856.

The resultant protein of interest may be used as a medicine, reagent, raw material, feed and so on depending on the nature of the protein.

When the protein of interest is used as a medicine, the protein may be used in a conventional manner. For example, the protein of interest may be used orally in the form of tablets (sugar-coated or enteric coated, if necessary), capsules, elixirs, microcapsules or the like; or parenterally in the form of injections such as aseptic solutions or suspensions in water or other pharmaceutically acceptable liquids. These preparations may be produced, for example, by mixing the compound or a salt thereof with physiologically acceptable carriers, flavoring agents, excipients, vehicles, antiseptics, stabilizers, binders, etc. in unit dosage forms. The amounts of active ingredients in these formulations are decided so that an appropriate dose within the specified range can be obtained.

Examples of additives which may be mixed in tablets, capsules, etc. include binders such as gelatin, corn starch, tragacanth gum and gum arabic, excipients such as crystalline cellulose, swelling agents such as corn starch, gelatin and alginic acid, lubricants such as magnesium stearate, sweetening agents such as sucrose, lactose and saccharin, and flavoring agents such as peppermint, akamono oil and cherry. When the unit dosage form is capsule, liquid carriers such as oils and fats may further be included in addition to the above-mentioned materials. Sterile compositions for injection can be formulated according to conventional practices in pharmaceutical manufacturing, e.g., by dissolving or suspending active ingredients in vehicles such as water for injection, naturally occurring vegetable oils such as sesame oil, coconut oil, etc.

Examples of aqueous liquids for injection include physiological saline and isotonic solutions containing glucose and other auxiliary agents (e.g. D-sorbitol, D-mannitol, sodium chloride, etc.). They may be used in combination with a suitable auxiliary solubilizer such as alcohol (e.g. ethanol), polyalcohol (e.g. propylene glycol, polyethylene glycol), nonionic surface-active agent (e.g. Polysorbate 80™, HCO-50). Examples of oily liquids for injection include sesame oil, soybean oil, etc. They may be used in combination with an auxiliary solubilizer such as benzyl benzoate, benzyl alcohol, etc.

In addition, buffers (e.g. phosphate buffer, sodium acetate buffer, etc.), analgesic agents (e.g. benzalkonium chloride, procaine hydrochloride), stabilizers (e.g. human serum albumin, polyethylene glycol, etc.), preservatives (e.g. benzyl alcohol, phenol, etc.), antioxidants, etc. may also be admixed therewith. Usually, the prepared injections are filled in appropriate ampoules.

The thus obtained preparations may be administered to human or other mammals (e.g., mouse, rat, guinea pig, rabbit, sheep, pig, bovine, cat, dog, monkey, etc.).

In the specification and drawings of the present application, the abbreviations used for bases (nucleotides), amino acids and so forth are those recommended by the IUPAC-IUB Commission on Biochemical Nomenclature or those conventionally used in the art. Examples of such abbreviations are given below. Amino acids which may have optical isomers are intended to represent their L-isomer unless otherwise specified.
DNA: Deoxyribonucleic acid S
cDNA: Complementary deoxyribonucleic acid
A: Adenine
T: Thymine
G: Guanine
C: Cytosine
M: A or C
R: G or A
W:A or T
S: G or C
Y: T or C
K: G or T
V: A or G or C
H: A or C or T
D: A or G or T
B: G or C or T
N: A or C or G or T
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetracetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine

The SEQ ID NOS of the SEQUENCE LISTING of the present specification represent the sequences as indicated below.

### [SEQ ID NO: 1]

This shows the DNA sequence of primer 1.

### [SEQ ID NO: 2]

This shows the DNA sequence of primer 2.

### [SEQ ID NO: 3]

This shows the DNA sequence of synthetic DNA 1.

### [SEQ ID NO: 4]

This shows the DNA sequence of synthetic DNA 2.

### [SEQ ID NO: 5]

This shows the DNA sequence of synthetic DNA 3.

### [SEQ ID NO: 6]

This shows the DNA sequence of synthetic DNA 4.

### [SEQ ID NO: 7]

This shows the DNA sequence of synthetic DNA 5.

### [SEQ ID NO: 8]

This shows the DNA sequence of synthetic DNA 6.

### [SEQ ID NO: 9]

This shows the DNA sequence of primer 3.

### [SEQ ID NO: 10]

This shows the DNA sequence of primer 4.

### [SEQ ID NO: 11]

This shows the DNA sequence of primer 5.

### [SEQ ID NO: 12]

This shows the DNA sequence of primer 6.

### [SEQ ID NO: 13]

This shows the DNA sequence of primer 7.

### [SEQ ID NO: 14]

This shows the DNA sequence of synthetic DNA 7.

### [SEQ ID NO: 15]

This shows the DNA sequence of synthetic DNA 8.

### [SEQ ID NO: 16]

This shows the DNA sequence of primer 8.

### [SEQ ID NO: 17]

This shows the DNA sequence of synthetic DNA 9.

### [SEQ ID NO: 18]

This shows the DNA sequence of synthetic DNA 10.

### [SEQ ID NO: 19]

This shows the DNA sequence of synthetic DNA 11.

### [SEQ ID NO: 20]

This shows the DNA sequence of synthetic DNA 12.

### [SEQ ID NO: 21]

This shows the amino acid sequence of the N-terminal 15 amino acid residues of rat GALP.

### [SEQ ID NO: 22]

This shows the nucleotide sequence of a DNA encoding the N-terminal 15 amino acid residues of rat GALP.

### [SEQ ID NO: 23]

This shows the nucleotide sequence obtained from *E. coli* clone No. 1 obtained in Example 2.

### [SEQ ID NO: 24]

This shows the nucleotide sequence obtained from *E. coli* clone No. 2 obtained in Example 2.

### [SEQ ID NO: 25]

This shows the nucleotide sequence obtained from *E. coli* clone No. 3 obtained in Example 2.

### [SEQ ID NO: 26]

This shows the nucleotide sequence obtained from *E. coli* clone No. 4 obtained in Example 2.

### [SEQ ID NO: 27]

This shows the nucleotide sequence obtained from *E. coli* clone No. 5 obtained in Example 2.

### [SEQ ID NO: 28]

This shows the nucleotide sequence obtained from *E. coli* clone No. 6 obtained in Example 2.

### [SEQ ID NO: 29]

This shows the nucleotide sequence obtained from *E. coli* clone No. 7 obtained in Example 2.

### [SEQ ID NO: 30]

This shows the nucleotide sequence obtained from *E. coli* clone No. 8 obtained in Example 2.

### [SEQ ID NO: 31]

This shows the nucleotide sequence obtained from *E. coli* clone No. 9 obtained in Example 2.

### [SEQ ID NO: 32]

This shows the nucleotide sequence obtained from *E. coli* clone No. 10 obtained in Example 2.

### [SEQ ID NO: 33]

This shows the nucleotide sequence obtained from *E. coli* clone No. 11 obtained in Example 2.

### [SEQ ID NO: 34]

This shows the nucleotide sequence obtained from *E. coli* clone No. 12 obtained in Example 2.

### [SEQ ID NO: 35]

This shows the nucleotide sequence obtained from *E. coli* clone No. 13 obtained in Example 2.

### [SEQ ID NO: 36]

This shows the nucleotide sequence obtained from *E. coli* clone No. 14 obtained in Example 2.

### [SEQ ID NO: 37]

This shows the nucleotide sequence obtained from *E. coli* clone No. 15 obtained in Example 2.

### [SEQ ID NO: 38]

This shows the nucleotide sequence obtained from *E*. *coli* clone No. 16 obtained in Example 2.

### [SEQ ID NO: 39]

This shows the nucleotide sequence obtained from *E. coli* clone No. 17 obtained in Example 2.

### [SEQ ID NO: 40]

This shows the nucleotide sequence obtained from *E. coli* clone No. 18 obtained in Example 2.

### [SEQ ID NO: 41]

This shows the amino acid sequence of the N-terminal 5 amino acid residues of MPIF 1Δ23.

### [SEQ ID NO: 42]

This shows the nucleotide sequence of a DNA encoding the N-terminal 5 amino acid sequence of MPIF 1Δ23.

### [SEQ ID NO: 43]

This shows the nucleotide sequence obtained from *E*. *coli* clone No. 1 obtained in Example 5.

### [SEQ ID NO: 44]

This shows the nucleotide sequence obtained from *E*. *coli* clone No. 2 obtained in Example 5.

### [SEQ ID NO: 45]

This shows the nucleotide sequence obtained from *E*. *coli* clone No. 3 obtained in Example 5.

### [SEQ ID NO: 46]

This shows the nucleotide sequence obtained from *E*. *coli* clone No. 4 obtained in Example 5.

### [SEQ ID NO: 47]

This shows the nucleotide sequence obtained from *E. coli* clone No. 5 obtained in Example 5.

### [SEQ ID NO: 48]

This shows the nucleotide sequence obtained from *E. coli* clone No. 6 obtained in Example 5.

### [SEQ ID NO: 49]

This shows the nucleotide sequence obtained from *E. coli* clone No. 7 obtained in Example 5.

### [SEQ ID NO: 50]

This shows the nucleotide sequence obtained from *E. coli* clone No. 8 obtained in Example 5.

### [SEQ ID NO: 51]

This shows the nucleotide sequence obtained from *E. coli* clone No. 9 obtained in Example 5.

### [SEQ ID NO: 52]

This shows the nucleotide sequence obtained from *E. coli* clone No. 10 obtained in Example 5.

### [SEQ ID NO: 53]

This shows the nucleotide sequence obtained from *E. coli* clone No. 11 obtained in Example 5.

### [SEQ ID NO: 54]

This shows the nucleotide sequence obtained from *E. coli* clone No. 12 obtained in Example 5.

### [SEQ ID NO: 55]

This shows the nucleotide sequence obtained from *E. coli* clone No. 13 obtained in Example 5.

### [SEQ ID NO: 56]

This shows the nucleotide sequence obtained from *E. coli* clone No. 14 obtained in Example 5.

### [SEQ ID NO: 57]

This shows the nucleotide sequence obtained from *E. coli* clone No. 15 obtained in Example 5.

### [SEQ ID NO: 58]

This shows the nucleotide sequence obtained from *E. coli* clone No. 16 obtained in Example 5.

### [SEQ ID NO: 59]

This shows the nucleotide sequence obtained from *E. coli* clone No. 17 obtained in Example 5.

### [SEQ ID NO: 60]

This shows the nucleotide sequence obtained from *E. coli* clone No. 18 obtained in Example 5.

### [SEQ ID NO: 61]

This shows the nucleotide sequence obtained from *E. coli* clone No. 19 obtained in Example 5.

### [SEQ ID NO: 62]

This shows the nucleotide sequence obtained from *E. coli* clone No. 20 obtained in Example 5.

### [SEQ ID NO: 63]

This shows the nucleotide sequence obtained from *E. coli* clone No. 21 obtained in Example 5.

### [SEQ ID NO: 64]

This shows the nucleotide sequence obtained from *E. coli* clone No. 22 obtained in Example 5.

### [SEQ ID NO: 65]

This shows the nucleotide sequence obtained from *E. coli* clone No. 23 obtained in Example 5.

### [SEQ ID NO: 66]

This shows the nucleotide sequence obtained from *E. coli* clone No. 24 obtained in Example 5.

### [SEQ ID NO: 67]

This shows the nucleotide sequence obtained from *E. coli* clone No. 25 obtained in Example 5.

### [SEQ ID NO: 68]

This shows the nucleotide sequence obtained from *E. coli* clone No. 26 obtained in Example 5.

### [SEQ ID NO: 69]

This shows the nucleotide sequence obtained from *E. coli* clone No. 27 obtained in Example 5.

### [SEQ ID NO: 70]

This shows the nucleotide sequence obtained from *E. coli* clone No. 28 obtained in Example 5.

### [SEQ ID NO: 71]

This shows the nucleotide sequence obtained from *E. coli* clone No. 29 obtained in Example 5.

### [SEQ ID NO: 72]

This shows the nucleotide sequence obtained from *E. coli* clone No. 30 obtained in Example 5.

### [SEQ ID NO: 73]

This shows the nucleotide sequence obtained from *E. coli* clone No. 31 obtained in Example 5.

### [SEQ ID NO: 74]

This shows the nucleotide sequence obtained from *E. coli* clone No. 32 obtained in Example 5.

### [SEQ ID NO: 75]

This shows the nucleotide sequence of synthetic DNA 13.

### [SEQ ID NO: 76]

This shows the nucleotide sequence of synthetic DNA 14.

### [SEQ ID NO: 77]

This shows the nucleotide sequence of synthetic DNA 15.

### [SEQ ID NO: 78]

This shows the nucleotide sequence of synthetic DNA 16.

### [SEQ ID NO: 79]

This shows the nucleotide sequence of synthetic DNA 17.

### [SEQ ID NO: 80]

This shows the nucleotide sequence of synthetic DNA 18.

### [SEQ ID NO: 81]

This shows the nucleotide sequence of synthetic DNA 19.

### [SEQ ID NO: 82]

This shows the nucleotide sequence of synthetic DNA 20.

### [SEQ ID NO: 83]

This shows the nucleotide sequence of synthetic DNA 21.

### [SEQ ID NO: 84]

This shows the nucleotide sequence of synthetic DNA 22.

### [SEQ ID NO: 85]

This shows the nucleotide sequence of synthetic DNA 23.

### [SEQ ID NO: 86]

This shows the nucleotide sequence of synthetic DNA 24.

### [SEQ ID NO: 87]

This shows the nucleotide sequence of synthetic DNA 25.

### [SEQ ID NO: 88]

This shows the nucleotide sequence of synthetic DNA 26.

### [SEQ ID NO: 89]

This shows the nucleotide sequence of synthetic DNA 27.

### [SEQ ID NO: 90]

This shows the nucleotide sequence of synthetic DNA 28.

### [SEQ ID NO: 91]

This shows the nucleotide sequence of synthetic DNA 29.

### [SEQ ID NO: 92]

This shows the nucleotide sequence of synthetic DNA 30.

### [SEQ ID NO: 93]

This shows the nucleotide sequence of synthetic DNA 31.

### [SEQ ID NO: 94]

This shows the nucleotide sequence of synthetic DNA 32.

### [SEQ ID NO: 95]

This shows the nucleotide sequence of synthetic DNA 33.

### [SEQ ID NO: 96]

This shows the nucleotide sequence of synthetic DNA 34.

### [SEQ ID NO: 97]

This shows the nucleotide sequence of synthetic DNA 35.

### [SEQ ID NO: 98]

This shows the nucleotide sequence of synthetic DNA 36.

### [SEQ ID NO: 99]

This shows the nucleotide sequence of synthetic DNA 37.

### [SEQ ID NO: 100]

This shows the nucleotide sequence of synthetic DNA 38.

### [SEQ ID NO: 101]

This shows the nucleotide sequence of synthetic DNA 39.

### [SEQ ID NO: 102]

This shows the nucleotide sequence of synthetic DNA 40.

### [SEQ ID NO: 103]

This shows the nucleotide sequence of synthetic DNA 41.

### [SEQ ID NO: 104]

This shows the nucleotide sequence of synthetic DNA 42.

### [SEQ ID NO: 105]

This shows the nucleotide sequence of synthetic DNA 43.

### [SEQ ID NO: 106]

This shows the nucleotide sequence of synthetic DNA 44.

### [SEQ ID NO: 107]

This shows the nucleotide sequence of synthetic DNA 45.

### [SEQ ID NO: 108]

This shows the nucleotide sequence of synthetic DNA 46.

### [SEQ ID NO: 109]

This shows the nucleotide sequence of synthetic DNA 47.

### [SEQ ID NO: 110]

This shows the nucleotide sequence of synthetic DNA 48.

### [SEQ ID NO: 111]

This shows the nucleotide sequence of synthetic DNA 49.

### [SEQ ID NO: 112]

This shows the nucleotide sequence of synthetic DNA 50.

### [SEQ ID NO: 113]

This shows the nucleotide sequence of synthetic DNA 51.

### [SEQ ID NO: 114]

This shows the nucleotide sequence of synthetic DNA 52.

### [SEQ ID NO: 115]

This shows the nucleotide sequence of synthetic DNA 53.

### [SEQ ID NO: 116]

This shows the amino acid sequence of a GPR8 ligand.

### [SEQ ID NO: 117]

This shows the amino acid sequence of a ZAQ-1 ligand.

### [SEQ ID NO: 118]

This shows the amino acid sequence of a bFGF CS23 mutein.

### [SEQ ID NO: 119]

This shows the nucleotide sequence of a DNA encoding a rat GALP obtained by the production process of the invention.

### [SEQ ID NO: 120]

This shows the nucleotide sequence of a DNA encoding an MPIF-1Δ23 obtained by the production process of the invention.

### [SEQ ID NO: 121]

This shows the nucleotide sequence of a DNA encoding a ZAQ-1 ligand obtained by the production process of the invention.

### [SEQ ID NO: 122]

This shows the nucleotide sequence of a DNA encoding a rat GALP obtained by the production process of the invention.

*Escherichia coli* (MM294 (DE3), pTCHGH-Na), which is a transformant carrying plasmid pTCHGH-Na that is used in Example 1 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) located at Central 6, 1-1 Higashi 1-chome, Tsukuba City, Ibaraki Pref. since December 10, 1997 under the Accession No. FERM BP-6888, and with the Institute for Fermentation, Osaka (IFO) located at 17-85 Jusanboncho 2-chome, Yodogawa-ku, Osaka City, Osaka Pref. since October 16, 1997 under the Accession No. IFO 16124.

*Escherichia coli* JM109/pGFPNP, a transformant obtained in Example 1 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) since July 17, 2000 under the Accession No. FERM BP-7223, and with the Institute for Fermentation, Osaka (IFO) since April 24, 2000 under the Accession No. IFO 16426.

*Escherichia coli* MM294 (DE3)/pTFCRGAL, a transformant obtained in Example 3 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) since July 17, 2000 under the Accession No. FERM BP-7226, and with the Institute for Fermentation, Osaka (IFO) since April 24, 2000 under the Accession No. IFO 16429.

*Escherichia coli* MM294 (DE3)/pTFCS4, a transformant obtained in Example 4 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) since July 17, 2000 under the Accession No. FERM BP-7227, and with the Institute for Fermentation, Osaka (IFO) since April 24, 2000 under the Accession No. IFO 16430.

*Escherichia coli* MM294 (DE3)/pTCIId23-MPIF1, which is a transformant carrying plasmid pTCII/d23-MPIF1 that is used in Example 8 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) since November 24, 1998 under the Accession No. FERM BP-6582, and with the Institute for Fermentation, Osaka (IFO) since October 27, 1998 under the Accession No. IFO 16212.

*Escherichia coli* JM109/pNPMPIF, a transformant obtained in Example 8 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) since July 17, 2000 under the Accession No. FERM BP-7224, and with the Institute for Fermentation, Osaka (IFO) since April 24, 2000 under the Accession No. IFO 16427.

*Escherichia coli* JM109/pNPMPIFNO, a transformant obtained in Example 9 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) since July 17, 2000 under the Accession No. FERM BP-7225, and with the Institute for Fermentation, Osaka (IFO) since April 24, 2000 under the Accession No. IFO 16428.

*Escherichia coli* TOP10/pHMITG, which is a transformant carrying plasmid pHMITG that is used in Example 12 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) since July 13, 2000 under the Accession No. FERM BP-7220, and with the Institute for Fermentation, Osaka (IFO) since May 26, 2000 under the Accession No. IFO 16441.

*Escherichia coli* MM294 (DE3)/pTCIIZAQN, a transformant obtained in Example 12 described later, has been deposited with the International Patent Organism Depository, National Institute of Advanced Industrial Science and Technology since May 24, 2001 under the Accession No. FERM BP-7608, and with the Institute for Fermentation, Osaka (IFO) since May 15, 2001 under the Accession No. IFO 16627.

*Escherichia coli* MM294 (DE3)/pTCIIZAQS, a transformant obtained in Example 13 described later, has been deposited with the International Patent Organism Depository, National Institute of Advanced Industrial Science and Technology since May 24, 2001 under the Accession No. FERM BP-7609, and with the Institute for Fermentation, Osaka (IFO) since May 15, 2001 under the Accession No. IFO 16628.

*Escherichia coli* MM294 (DE3)/pTCIIZAQNO, a transformant obtained in Example 15 described later, has been deposited with the International Patent Organism Depository, National Institute of Advanced Industrial Science and Technology since May 24, 2001 under the Accession No. FERM BP-7610, and with the Institute for Fermentation, Osaka (IFO) since May 15, 2001 under the Accession No. IFO 16629.

*Escherichia coli* MM294 (DE3)/pTCRGDE, a transformant obtained in Example 17 described later, has been deposited with the International Patent Organism Depository, National Institute of Advanced Industrial Science and Technology since May 24, 2001 under the Accession No. FERM BP-7612, and with the Institute for Fermentation, Osaka (IFO) since May 17, 2001 under the Accession No. IFO 16631.

*Escherichia coli* MM294 (DE3)/pTCRGNODE, a transformant obtained in Example 18 described later, has been deposited with the International Patent Organism Depository, National Institute of Advanced Industrial Science and Technology since May 24, 2001 under the Accession No. FERM BP-7614, and with the Institute for Fermentation, Osaka (IFO) since May 17, 2001 under the Accession No. IFO 16633.

*Escherichia coli* MM294 (DE3)/pTCRGTODE, a transformant obtained in Example 20 described later, has been deposited with the International Patent Organism Depository, National Institute of Advanced Industrial Science and Technology since May 24, 2001 under the Accession No. FERM BP-7613, and with the Institute for Fermentation, Osaka (IFO) since May 17, 2001 under the Accession No. IFO 16632.

### EXAMPLES

Hereinbelow, the present invention will be described in more detail with reference to the following Examples. However, these Examples are not intended to limit the scope of the present invention. Gene manipulations using *E. coli* were performed in accordance with those methods described in the book "Molecular Cloning".

### EXAMPLE 1

### Construction of Plasmids for Searching for Optimized Sequences

### 1) Construction of Plasmid pGFPuvqc

In order to eliminate the NdeI restriction site located in the GFPuv structural gene in pGFPuv (Clontech), the codon of the His at position 77 was changed from CAT to CAC through site-directed mutagenesis (QuickChange™ Site Directed Mutagenesis Kit, Stratagene) using primer 1: CGTTATCCGGATCACATGAAACGGCATG (SEQ ID NO: 1) and primer 2: CATGCCGTTTCATGTGATCCGGATAACG (SEQ ID NO: 2). Thus, plasmid pGFPuvqc was obtained.

### 2) Construction of Plasmid pGFPuvqd

Plasmid pGFPuvqc was digested with HindIII (Takara Shuzo) and KpnI (Takara Shuzo), followed by agarose gel electrophoresis to cut out an approx. 3.3 kbp band. The DNA was recovered from the gel using QIAquick Gel Extraction Kit (Qiagen). Synthetic DNA 1: AGCTTCATATGTTCGAAGTACTAGATCTGGTAC (SEQ ID NO: 3) and synthetic DNA 2: CAGATCTAGTACTTCGAACATATGA (SEQ ID NO: 4) (100 pmol each) were dissolved in TE buffer, retained at 90°C for 10 min, and then gradually cooled to room temperature to perform annealing. The thus annealed DNA fragment was inserted into pGFPuvqc between the HindIII and KpnI sites using Ligation kit ver. 2 (Takara Shuzo) to thereby obtain plasmid pGFPuvqd.

### 3) Construction of Plasmid pGFPNP

Plasmid pNPHGH in which the T7 promoter of pTCHGH-Na described in Reference Example 1 in WO 00/20439 is replaced with a synthetic promoter NP2 was constructed as described below. Briefly, plasmid pTCHGH-Na was digested with EcoRI (Takara Shuzo) and XbaI (Takara Shuzo), followed by agarose gel electrophoresis to cut out an approx. 4.6 kbp band. The DNA was recovered from the gel using QIAquick Gel Extraction Kit (Qiagen). One hundred pmol each of synthetic DNA 3: AATTCTATAAAAATAATTGTTGACATATTTTATAAATTTTGGCATAATAGATCTAAT TGTGAGCGGATAACAATTCTGCAGAAGCTTGAGCTCGGTACCCGGGGATCCT (SEQ ID NO: 5) comprising the synthetic promoter NP2, and synthetic DNA 4: CTAGAGGATCCCCGGGTACCGAGCTCAAGCTTCTGCAGAATTGTTATCCGCTCA CAATTAGATCTATTATGCCAAAATTTATAAAATATGTCAACAATTATTTTTATAG (SEQ ID NO: 6) comprising a nucleotide sequence complementary to the synthetic promoter NP2 were dissolved in TE buffer, retained at 90°C for 10 min, and then gradually cooled to room temperature to perform annealing. The resultant double-stranded DNA fragment was inserted into pTCHGH-Na between the EcoRI and XbaI sites using Ligation kit ver. 2 (Takara Shuzo) to thereby obtain plasmid pNPHGH.

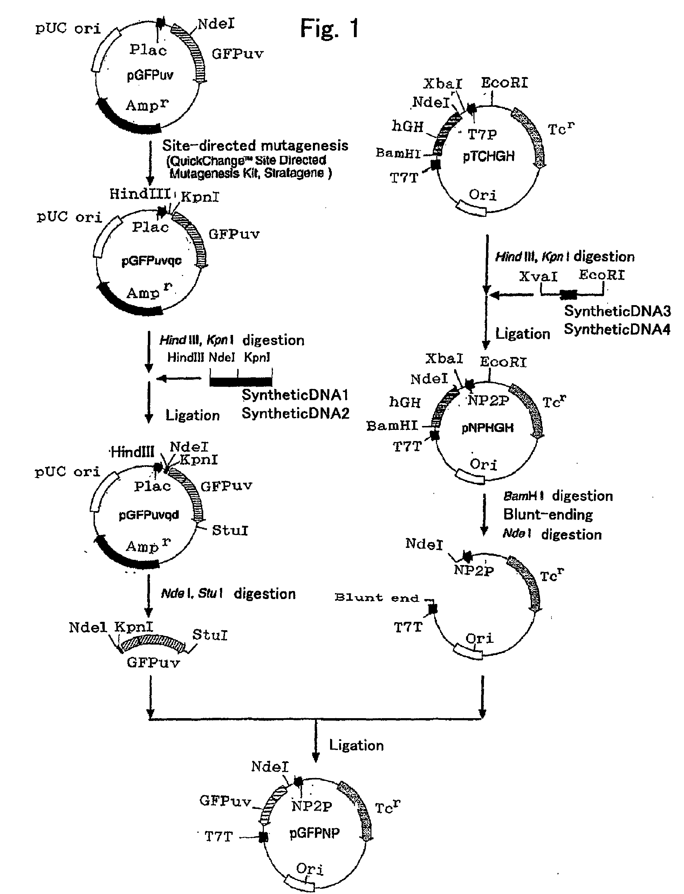

Plasmid pNPHGH was digested with BamHI (Takara Shuzo), followed by blunt-ending with DNA Blunting Kit (Takara Shuzo). After further digestion with NdeI, the digest was subjected to agarose gel electrophoresis to cut out an approx. 4.6 kbp band. The DNA was recovered from the gel using QIAquick Gel Extraction Kit (Qiagen). Plasmid pGFPuvqd was digested with NdeI and StuI, followed by agarose gel electrophoresis to cut out an approx. 0.8 kbp band. A DNA comprising the GFPuv structural gene was recovered from the gel using QIAquick Gel Extraction Kit (Qiagen). The thus recovered DNA was inserted into pNPHGH between the NdeI site and BamHI (blunted) site using Ligation kit ver. 2 (Takara Shuzo) to thereby obtain plasmid pGFPNP for use in selecting N-terminal optimized sequences (Fig. 1).

Plasmid pGFPNP is an expression plasmid containing a GFPuv structural gene to be transcribed from NP2 promoter and having NdeI, ScaI and AgeI restriction sites upstream of the GFPuv structural gene. It is possible to insert DNA fragments into this plasmid using these restriction enzyme sites.

*E. coli* JM109 was transformed with plasmid pGFPNP to thereby obtain *E. coli* JM109/pGFPNP.

### EXAMPLE 2

### Search for Rat GALP Sequences Optimized for Expression

Plasmid pGFPNP was digested with Ndel (Takara Shuzo) and ScaI (Takara Shuzo), followed by agarose gel electrophoresis to cut out an approx. 4.9 kbp band. The DNA was recovered from the gel using QIAquick Gel Extraction Kit (Qiagen).

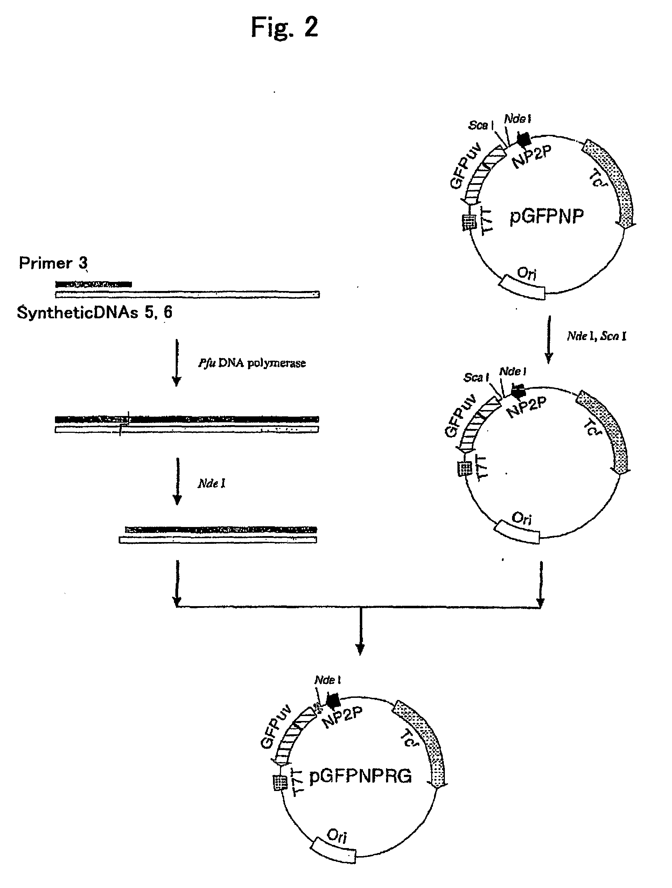

Random nucleotide sequences encoding N-terminal amino acids of rat GALP (a rat type ligand peptide for rat galanin receptor (1-60); WO 99/48920) were prepared as described below. Briefly, double-stranded DNA fragments were prepared with *Pfu* DNA polymerase (Stratagene) using mixed bases-containing synthetic DNA 5: AGGTAACCAGCACTATTTAAAGTCCANCCNCCNCGNCCNCGRTGNGCNGGNGC CATATGTATATCTCCTTCTTAAAG (SEQ ID NO: 7) and synthetic DNA 6: AGGTAACCAGCACTATTTAAAGTCCANCCNCCYCTNCCYCTRTGNGCNGGNGC CATATGTATATCTCCTTCTTAAAG (SEQ ID NO: 8) as templates and primer 3: CTTTAAGAAGGAGATATACATATG (SEQ ID NO: 9) as a primer. The extension reaction solution was composed of 5 µl of *Pfu* DNA polymerase, 5 µl each of 10x Reaction buffer attached to the polymerase and dNTP mixture (Takara Shuzo), 1 µI each of synthetic DNA 3 (42.2 µmol/L), synthetic DNA 4 (5 µmol/L) and primer 3 (100 µmol/L), and 32 µl of distilled water. The extension reaction was performed at 94°C for 30 sec, at 58°C for 30 sec, and at 72°C for 20 min. The resultant reaction solution was concentrated and desalted with Microcon 30 (Nihon Millipore), followed by digestion of resultant double-stranded DNA fragments with NdeI (Takara Shuzo). This digestion with NdeI was conducted as described below. Briefly, a reaction solution consisting of 10 µl of the above concentrated/desalted reaction solution, 2 µl of Buffer H (Takara Shuzo), 7 µl of distilled water and 1 µl of NdeI was retained at 37°C for 3 hr to perform digestion. From the reaction solution after this NdeI digestion, DNA fragments were purified using QIAquick Gel Extraction Kit (Qiagen). These DNA fragments were inserted individually into pGFPNP2 between the NdeI and ScaI sites using Ligation kit ver. 2 (Takara Shuzo) to thereby prepare a set of expression plasmid pGFPNPRG having at the N-terminal of GFPuv a random nucleotide sequence encoding N-terminal amino acids of rat GALP (Fig. 2).

*E. coli* JM109 was transformed with this set of expression plasmid pGFPNPRG, coated on LB agar medium containing 10 mg/L tetracycline, and cultured overnight at 37°C for colony formation. The thus formed colonies were observed under a UV ramp at 354 nm, followed by selection of 2 colonies emitting intense fluorescence and 16 colonies emitting weak fluorescence. These colonies were subjected to colony PCR using primer 4: TTCATACACGGTGCCTGACTGCGTTAG (SEQ ID NO: 10) and primer 5: TCAACAAGAATTGGGACAACTCC (SEQ ID NO: 11), followed by agarose gel electrophoresis to confirm that a DNA fragment of interest is inserted. The products of this colony PCR were purified with QIAquick PCR Purification Kit (Qiagen), and their nucleotide sequences were analyzed with primers 4 and 5. As a result, the nucleotide sequences shown in Table 1 were obtained. Of these, the nucleotide sequences No. 1 and No. 2 were obtained from those *E. coli* clones emitting intense fluorescence.

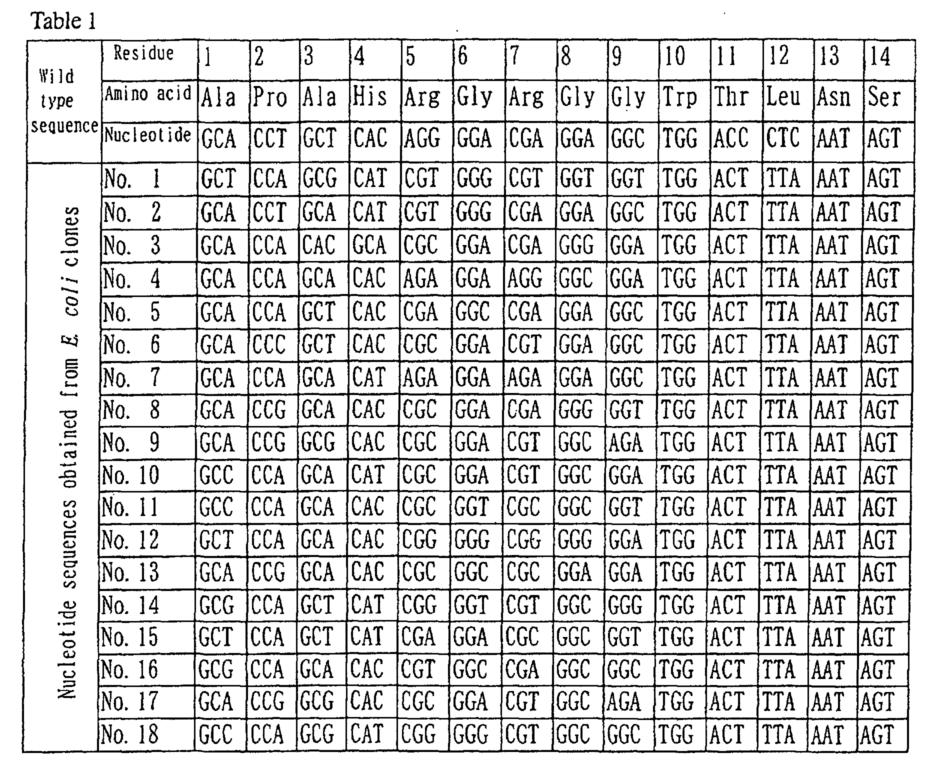

### EXAMPLE 3

Construction of Clones that Express Rat GALP Encoded by Wild Type Nucleotide Sequence

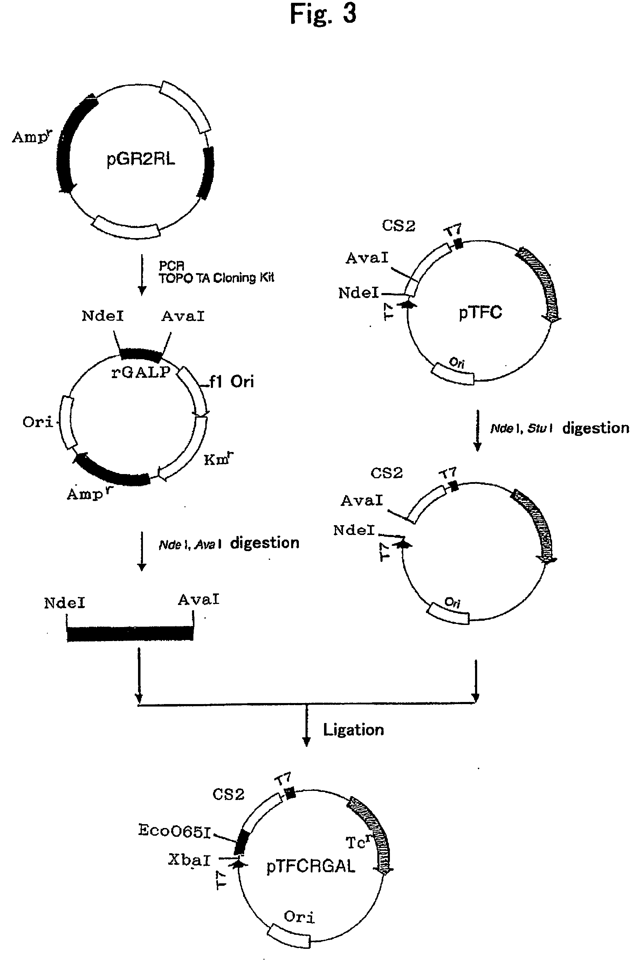

As a rat GALP expression plasmid, plasmid pTFCRGAL was constructed as described below for expression of rat GSLP-CS23 fusion protein from T7 promoter (Fig. 3).

Plasmid pTFC described in Example 21 in WO 99/48920 was digested with NdeI and AvaI, followed by agarose gel electrophoresis to cut out an approx. 3.9 kbp band. The DNA was recovered from the gel using QIAquick Gel Extraction Kit (Qiagen).

A fragment containing a rat GALR2L structural gene was amplified by PCR from plasmid pGR2RL4 (described in Example 18 in WO 99/48920) where a rat GALP structural gene is cloned, using primer 6: AGCATATGGCACCTGCTCACAGG (SEQ ID NO: 12) having an initiation codon ATG and an NdeI restriction site upstream of rat GALR2L structural gene and primer 7: CCCTCGGGGCAGGTCATCCTTGGAGAG (SEQ ID NO: 13) having a TGC codon encoding Cys and an AvaI restriction site downstream of the GALP structural gene. The resultant fragment was cloned using TOPO TA Cloning Kit to thereby obtain plasmid pCR2.1TOPO/rGAL. This plasmid pCR2.1TOPO/rGAL was digested with NdeI and AvaI, followed by agarose gel electrophoresis to cut out an approx. 0.2 kbp band. The DNA was recovered from the gel using QIAquick Gel Extraction Kit (Qiagen). This DNA fragment was ligated to pTFC between the NdeI and AvaI sites using Ligation kit ver. 2 (Takara Shuzo) to thereby obtain plasmid pTFCRGAL. This plasmid comprises a DNA (SEQ ID NO: 119) comprising the nucleotide sequence No. 1 described in Example 2 and having a nucleotide sequence encoding rat GALP.

*E. coli* MM294 (DE3) was transformed with this expression plasmid pTFCRGAL to thereby obtain *E. coli* MM294 (DE3)/pTFCRGAL having the ability to express rGAL-CS23 fusion protein.

### EXAMPLE 4

### Expression of Rat GALP using Optimized Sequences

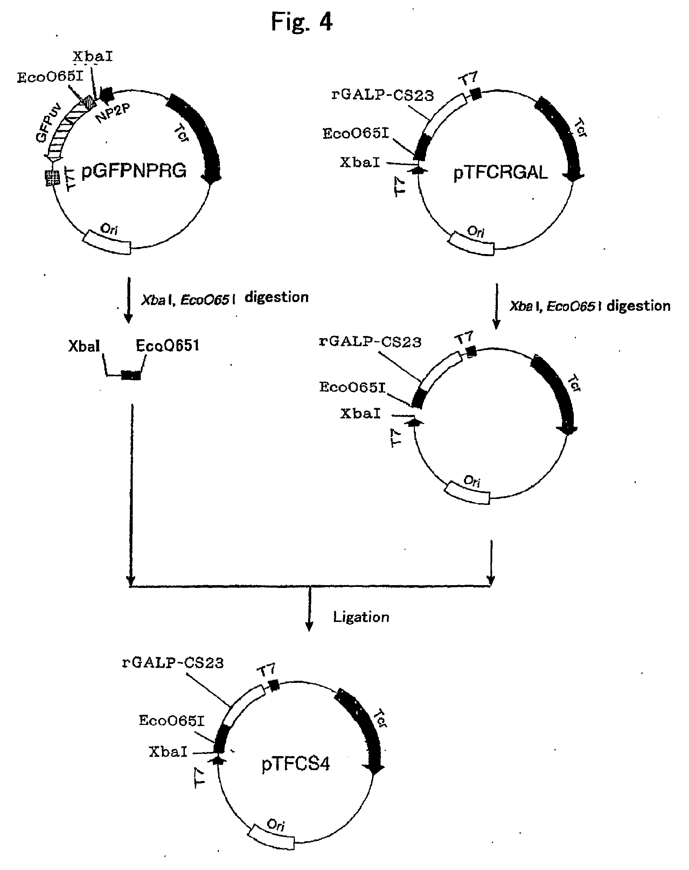

A nucleotide sequence encoding an N-terminal portion of the rat GALP structural gene in pTFCRGAL was changed from the corresponding wild type sequence to the nucleotide sequence obtained from an intense fluorescence-emitting colony in Example 2, as described below (Fig. 4).

Rat GALP-CS23 expression plasmid pTFCRGAL encoding the wild-type nucleotide sequence was digested with XbaI and EcoO65I, followed by agarose gel electrophoresis to cut out an approx. 4.5 kbp band. The DNA was recovered from the gel using QIAquick Gel Extraction Kit (Qiagen). Colony No. 1 obtained in Example 2 was inoculated into LB medium containing 10 mg/L tetracycline and cultured overnight at 37°C. From the resultant cells, plasmid pGFPNP/rGAL1 was purified using QIAprep8 Miniprep Kit (Qiagen). This plasmid pGFPNP/rGAL1 was digested with XbaI and EcoO65I, followed by agarose gel electrophoresis to cut out an approx. 0.1 kbp band. The DNA was recovered from the gel using QIAquick Gel Extraction Kit (Qiagen). This DNA fragment was ligated to pTFCRGAL between the XbaI and EcoO65I sites using Ligation kit ver. 2 (Takara Shuzo) to thereby obtain plasmid pTFCS4. This is an expression plasmid for rat GALP-CS23 fusion protein having the nucleotide sequence obtained from clone No. 1 selected in Example 2 at an N-terminal portion of rat GALP.

*E. coli* MM294 (DE3) was transformed with this expression plasmid pTFCS4 to thereby obtain *E. coli* MM294 (DE3)/pTFCS4 having the ability to express rGALP-CS4 fusion protein.

### EXAMPLE 5

### Comparison of Expression Levels of Rat GALP Proteins Encoded by Wild-Type and Optimized Sequences

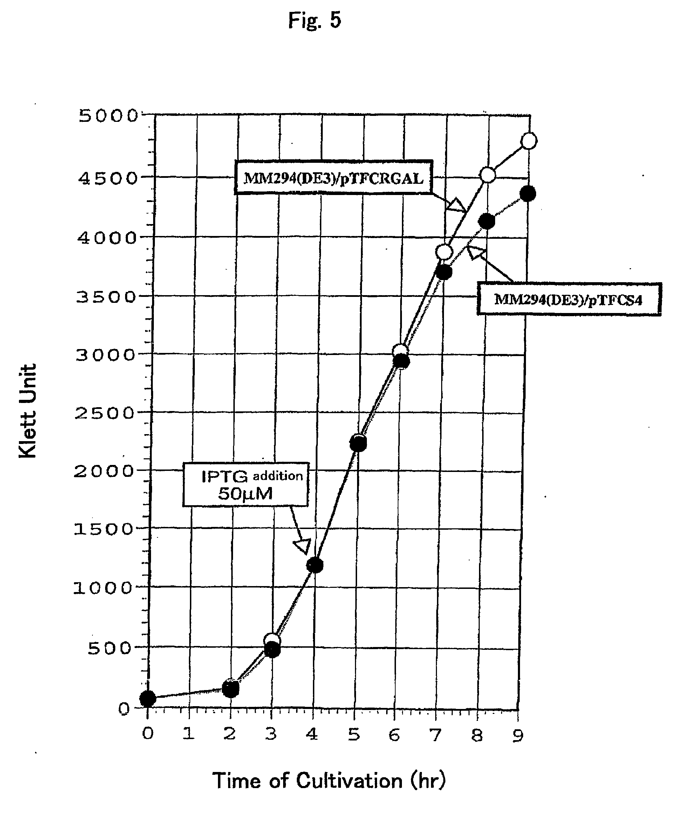

The rat GALP-CS23 fusion protein-expressing *E. coli* MM294 (DE3)/pTFCRGAL and *E. coli* MM294 (DE3)/pTFCS4 obtained in Examples 3 and 4, respectively, were cultured separately in 1 L of LB medium (1% peptone, 0.5% yeast extract, 0.5% sodium chloride) containing 10 mg/L tetracycline at 30°C for 16 hr. The resultant culture broth (75 ml each) was transferred to a 3-L jar fermenter containing 1.5 L of a medium for primary fermentation (1.68% sodium monohydrogenphosphate, 0.3% sodium dihydrogenphosphate, 0.1% ammonium chloride, 0.05% sodium chloride, 0.024% magnesium sulfate, 0.02% New Pole LB-625, 0.0005% thiamine hydrochloride, 1.5% glucose, 1.0% casamino acids, 1.0% yeast extract). Then, aeration agitation culture was started at 37°C under a gas flow of 16 L/min at an agitation speed of 200 rpm. When the turbidity of the culture broth reached approx. 1200 Klett units, isopropyl-β-D-thiogalactopyranoside (IPTG) was added at 5.95 mg/l min. Twenty-four min, 130 min and 200 min after the addition of IPTG, 0.75% glucose was added. The cultivation was continued up to 9 hr after the start of the cultivation (Fig. 5).

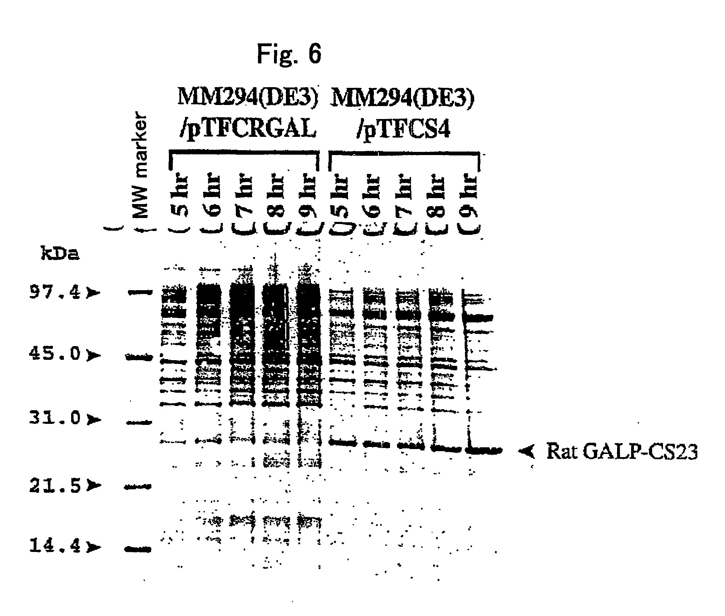

A 0.2 ml sample was taken from the culture broth at regular intervals during the cultivation and centrifuged at 12000 rpm for 10 min. The supernatant was discarded, and the resultant cells were subjected to SDS-PAGE to confirm the expression of rat GALP-CS23. Briefly, the cells obtained from 0.2 ml of the culture broth were suspended in a sample buffer (125 mM Tris-HCl, 1% sodium dodecyl sulfate, 15% glycerol, 5% 2-mercaptoethanol, 0.005% Bromophenol Blue) and treated at 95°C for 5 min, followed by electrophoresis in Multigel 15/25 (Daiichi Pure Chemicals Co., Ltd.). The resultant gel was stained with Rapid CBB KANTO (Kanto Kagaku). As a result, a higher expression of rat GALP-CS23 was recognized in *E. coli* MM294 (DE3)/pTFCS4 carrying an expression plasmid in which an N-terminal portion of rat GALP is encoded by the nucleotide sequence of clone No. 1 selected in Example 2, compared to *E. coli* MM294 (DE3)/pTFCRGAL carrying an expression plasmid in which the corresponding N-terminal portion of rat GALP is encoded by the wild-type sequence (Fig. 6).

### EXAMPLE 6

### Search for MPIF-1Δ23 Sequences Optimized for Expression

Plasmid pGFPNP was digested with NdeI (Takara Shuzo) and Scal (Takara Shuzo), followed by agarose gel electrophoresis to cut out an approx. 4.9 kbp band. The DNA was recovered from the gel using QIAquick Gel Extraction Kit (Qiagen).

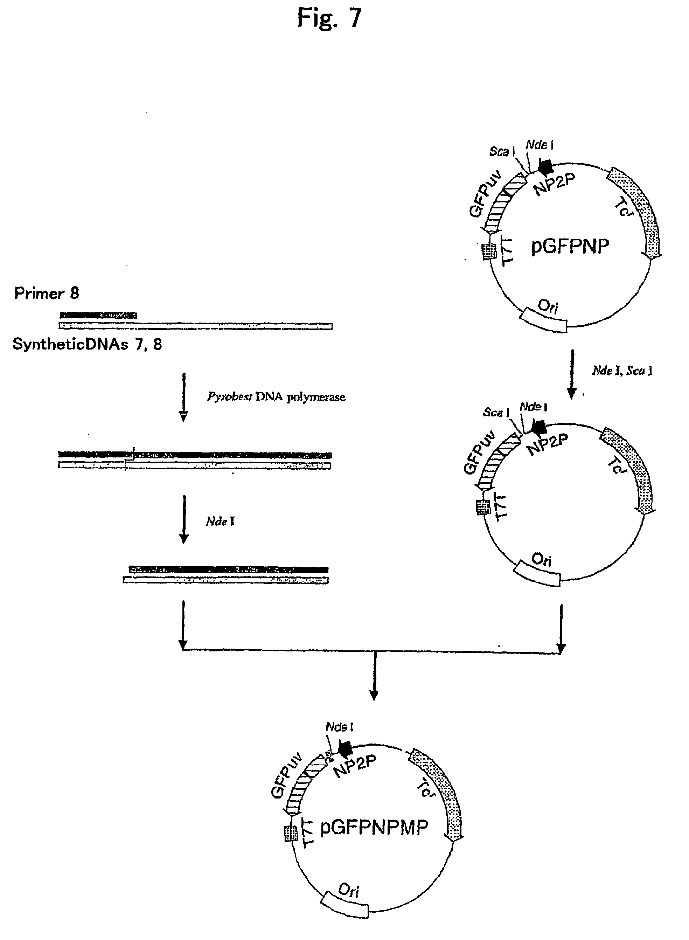

Random nucleotide sequences encoding N-terminal amino acids of MPIF-1Δ23 (myeloid progenitor inhibitory factor-1Δ23; WO 95/17092) were prepared as described below. Briefly, double-stranded DNA fragments were prepared with *Pyrobest* DNA polymerase (Takara Shuzo) using a 1:2 mixture of mixed bases-containing synthetic DNA 7: GGGATGCTTCGTGGGGTGTAGGAGATGCAGCAGTCAGCACTAGTNGCRTGRAA YCTRTCCATATGTTGGCGCGCCTT (SEQ ID NO: 14) and synthetic DNA 8: GGGATGCTTCGTGGGGTGTAGGAGATGCAGCAGTCAGCACTAGTNGCRTGRAA NCGRTCCATATGTTGGCGCGCCTT (SEQ ID NO: 15) as templates and primer 8: AAGGCGCGCCAACATATG (SEQ ID NO: 16). The extension reaction solution was composed of 3 µl of *Pyrobest* DNA polymerase (5 U/µl), 10 µl of 10x Reaction buffer attached to the polymerase, 16 µl of dNTP mixture, 0.5 µl of synthetic DNA 7 (1 µg/µl), 1 µl of synthetic DNA 8 (1 µg/µl), 2 µl of primer 8 (100µmol/L), and 67.5 µl of distilled water. The extension reaction was performed at 94°C for 30 sec, at 58°C for 30 sec, and at 70°C for 5 min. DNA fragments were purified from the resultant reaction solution using QIAquick Gel Extraction Kit (Qiagen), followed by digestion of the double-stranded DNA fragments with NdeI (Takara Shuzo). This digestion was conducted as described below. Briefly, a reaction solution consisting of 27 µl of the purified DNA fragment solution, 3.5 µl of Buffer H (Takara Shuzo), 1.5 µl of distilled water and 3 µl of NdeI was retained at 37°C for 2 hr to perform digestion. Then, the reaction solution was retained at 70°C for 15 min to deactivate NdeI and concentrated/desalted with Microcon 10 (Nihon Millipore). The resultant DNA fragments were individually inserted into pGFPNP between the NdeI and Scal sites using Ligation kit ver. 2 (Takara Shuzo) to thereby construct a set of plasmid pGFPNPMP for searching for optimized nucleotide sequences (Fig. 7).

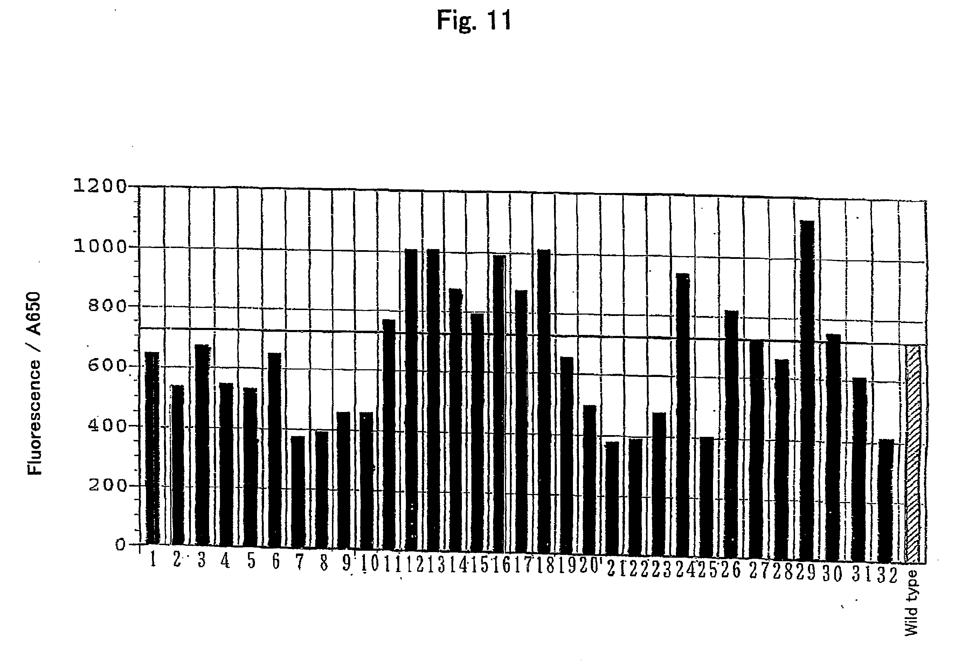

*E. coli* JM109 was transformed with this set of plasmid and coated on LB agar medium containing 10 mg/L tetracycline, and cultured overnight at 37°C for colony formation. The thus formed colonies were observed under a UV ramp at 354 nm, followed by selection of 2 colonies emitting intense fluorescence, 15 colonies emitting weak fluorescence and 1 colony emitting no fluorescence. These colonies were injected separately into 0.5 ml of LB medium containing 10 mg/L tetracycline. After an overnight culture at 37°C, 50 ml of the culture broth of each colony was transferred into a 96-well microplate containing 150 ml of physiological saline. Then, absorbance at 650 nm (A₆₅₀) was measured with 96 Microplate Reader (Molecular Device), and fluorescence intensity was measured at the excitation wavelength of 355 nm and the emission wavelength of 538 nm with a multiplate fluorescence measurement apparatus (Dainippon Pharmaceutical). The value obtained by dividing fluorescence intensity by absorbance is shown as GFP expression efficiency. Furthermore, these colonies were subjected to colony PCR using primer 4 and primer 5, followed by agarose gel electrophoresis to confirm that a DNA fragment of interest was inserted. The products of the colony PCR were purified using QIAquick PCR Purification Kit (Qiagen), followed by analysis of their nucleotide sequences with primer 4 and primer 5. As a result, the nucleotide sequences shown in Table 2 were obtained. Some clones exhibited more intense fluorescence than the clone having the wild-type nucleotide sequence of MPIF-1Δ23 (Table 2, Fig. 11).

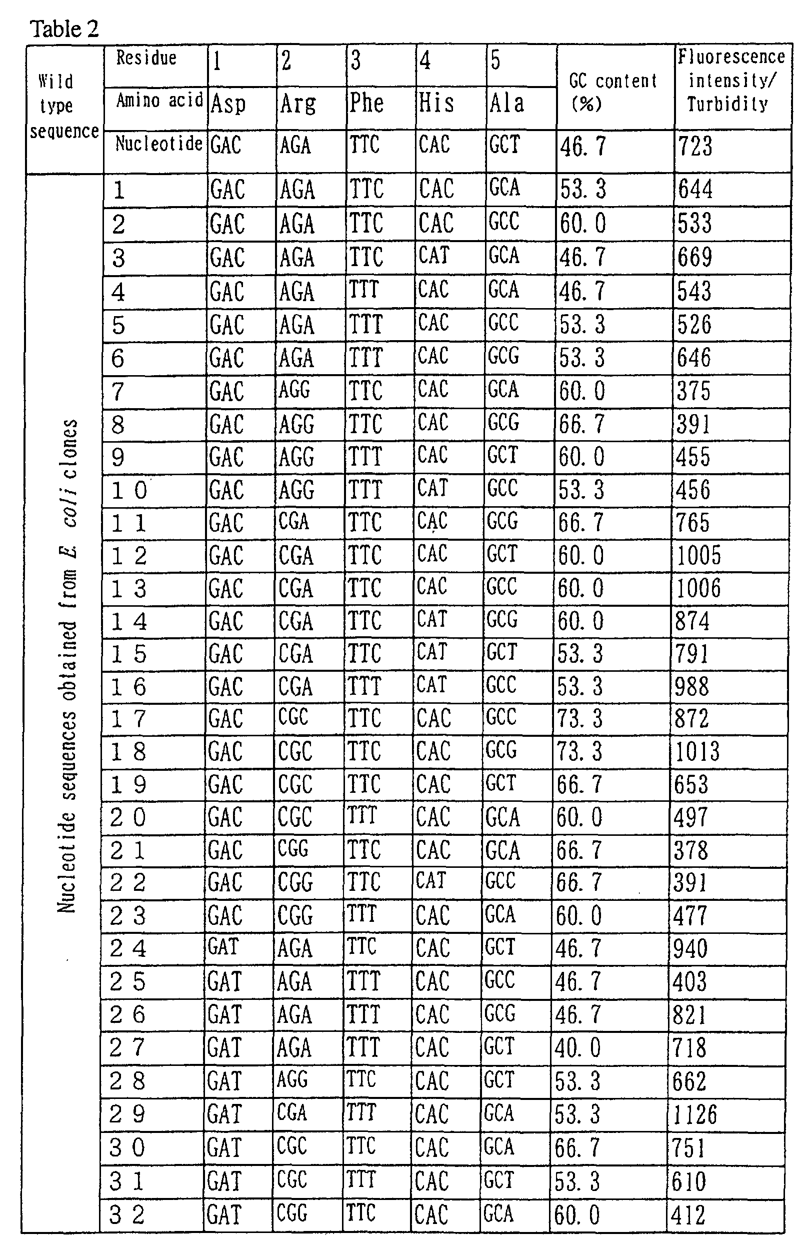

### EXAMPLE 7

### Construction of Plasmid pNPHGHlacts

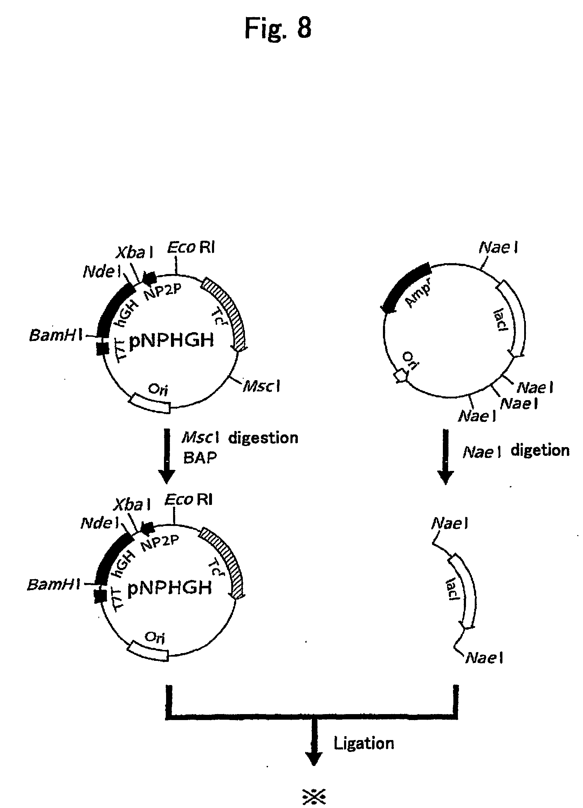

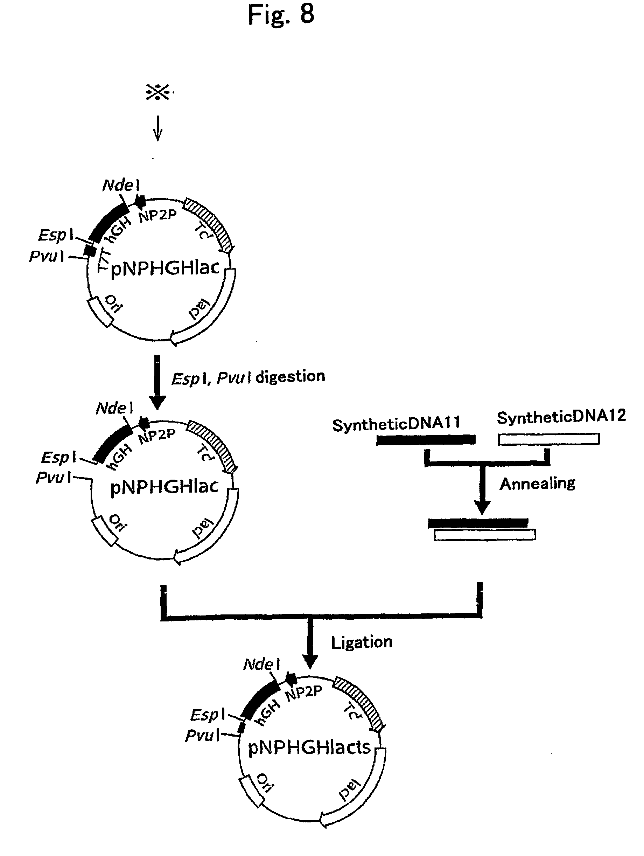

Plasmid pNPHGH constructed in Example 1 was digested with MscI, followed by dephosphorylation of the resultant ends with Bacterial Alkaline Phosphatase (Nippon Gene). On the other hand, plasmid pET11a (Takara Shuzo) was digested with NaeI, followed by agarose gel electrophoresis to cut out an approx. 1.6 kbp band. The DNA was recovered from the gel using QIAquick Gel Extraction Kit (Qiagen). This DNA fragment containing lacI was inserted into the MscI-digested site of pNPHGH using Ligation kit ver. 2 (Takara Shuzo) to thereby obtain plasmid pNPHGHlac. Further, the following operations were conducted in order to replace its T7 terminator with a synthetic terminator. Briefly, plasmid pNPHGHlac was digested with EspI and PvuI, followed by agarose gel electrophoresis to cut out an approx. 6.1 kbp band. The DNA was recovered from the gel using QIAquick Gel Extraction Kit (Qiagen). One hundred pmol each of synthetic DNA 9: TGAGCATGCATACTAGTCTCGAGTAATCCCACAGCCGCCGCCAGTTCCGCTGGC GGCGGCATTTTCGAT (SEQ ID NO: 17) and synthetic DNA 10: CGAAAATGCCGCCGCCAGCGGAACTGGCGGCGGCTGTGGGATTACTCGAGACT AGTATGCATGC (SEQ ID NO: 18) were dissolved in TE buffer, retained at 90°C for 10 min, and then gradually cooled to room temperature to perform annealing. The resultant DNA was ligated to pNPHGHlac between the EspI and PvuI sites using Ligation kit ver. 2 (Takara Shuzo) to thereby obtain plasmid pNPHGHlacts (Fig. 8).

### EXAMPLE 8

### Construction of Clones that Express MPIF-1Δ23 Encoded by Wild Type Nucleotide Sequence

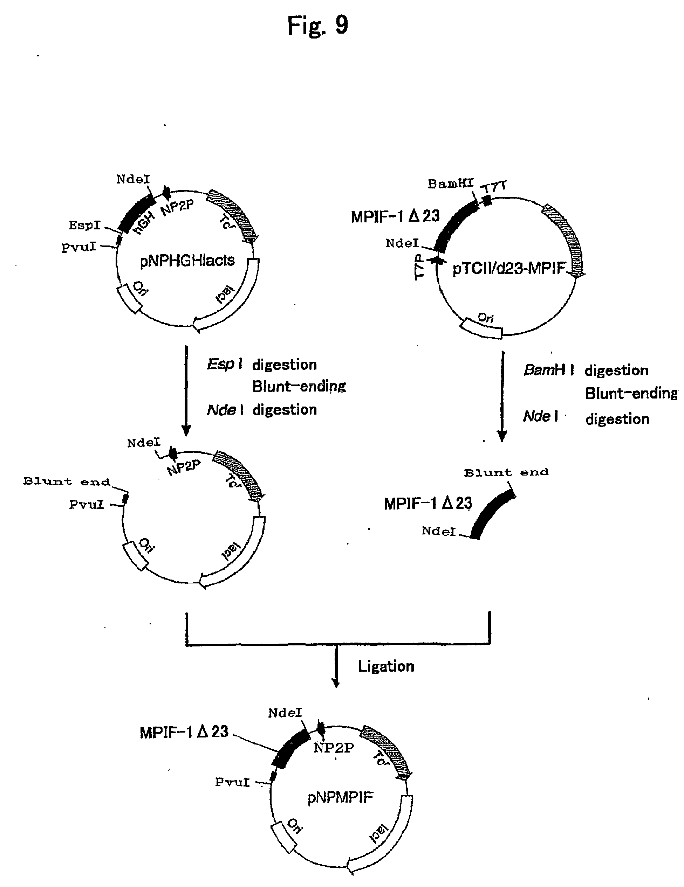

Plasmid pNPHGHlacts was digested with EspI, followed by blunt-ending with DNA Blunting Kit (Takara Shuzo) and further digestion with NdeI. The digest was subjected to agarose gel electrophoresis to cut out an approx. 3.9 kbp band, and the DNA was recovered from the gel using QIAquick Gel Extraction Kit (Qiagen). Plasmid pTCII/d23-MPIF1 described in Reference Example 1 in WO 00/40610 was digested with BamHI, followed by blunt-ending with DNA Blunting Kit (Takara Shuzo) and further digestion with NdeI. The digest was subjected to agarose gel electrophoresis to cut out an approx. 240 bp band, and the DNA was recovered from the gel using QIAquick Gel Extraction Kit (Qiagen). This DNA was ligated to pNPHGHlacts between the NdeI site and EspI (blunted) site using Ligation kit ver. 2 (Takara Shuzo) to thereby obtain plasmid pNPMPIF for expressing MPIF-1Δ23 encoded by the wild-type sequence (Fig. 9). *E coli* JM109 competent cells (Toyobo) were transformed with this plasmid to thereby obtain *E. coli* JM109/pNPMPIF having the ability to express MPIF-1Δ23.

### EXAMPLE 9

### Construction of Clones that Express MPIF-1Δ23 Encoded by Optimized Sequences

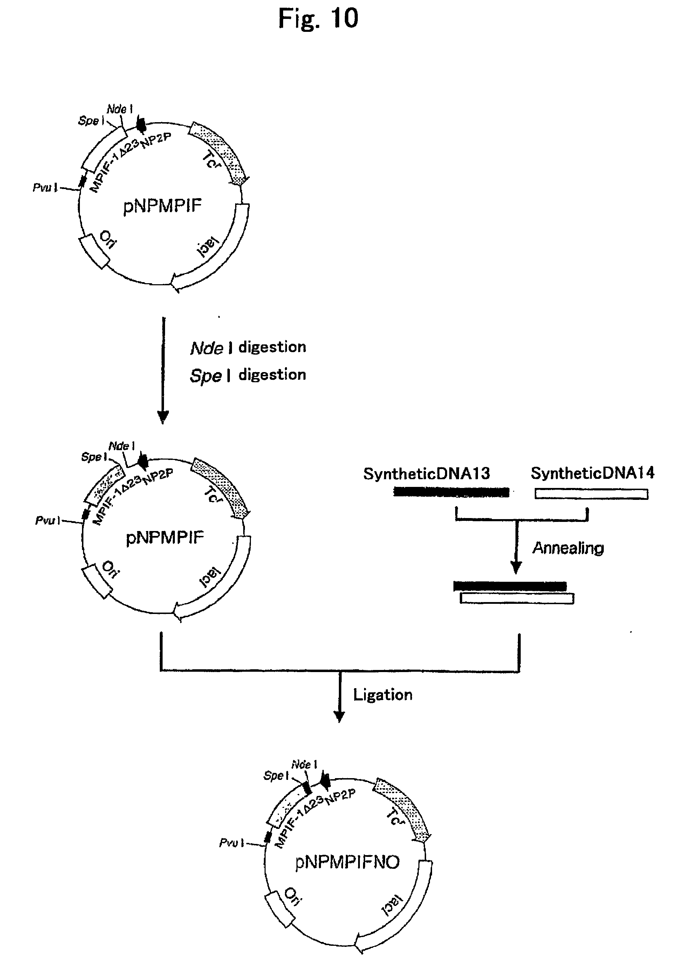

Plasmid pNPMPIF obtained in Example 8 was digested with NdeI (Takara Shuzo) and SpeI (Takara Shuzo), followed by agarose gel electrophoresis to cut out an approx. 4.8 kbp band. The DNA was recovered from the gel using QIAquick Gel Extraction Kit (Qiagen). One hundred pmol each of synthetic DNA 11: TATGGATCGATTTCACGCAA (SEQ ID NO: 19) comprising the nucleotide sequence of clone No. 29 obtained in Example 7 and synthetic DNA 12: CTAGTTGCGTGAAATCGATCCA (SEQ ID NO: 20) comprising a nucleotide sequence complementary to the nucleotide sequence of clone No. 29 were dissolved in TE buffer, retained at 90°C for 10 min, and then gradually cooled to room temperature to perform annealing. The resultant double-stranded DNA fragment was ligated to pTCII/d23-MPIF between the NdeI and SpeI sites using Ligation kit ver. 2 (Takara Shuzo) to thereby obtain plasmid pTCII/MPIFNO. pTCII/MPIFNO was digested with BamHI, followed by blunt-ending with DNA Blunting Kit (Takara Shuzo) and further digestion with NdeI. The digest was subjected to agarose gel electrophoresis to cut out an approx. 240 bp band, and the DNA was recovered from the gel using QIAquick Gel Extraction Kit (Qiagen). This DNA was ligated to pNPHGHkacts between the NdeI site and EspI (blunted) site using Ligation kit ver. 2 (Takara Shuzo) to thereby obtain plasmid pTCII/MPIFNO for expressing a MPIF-1Δ23 protein having an N-terminal portion encoded by a modified nucleotide sequence (Fig. 10). This plasmid comprises a DNA (SEQ ID NO: 120) that comprises the nucleotide sequence of clone No. 29 in Example 6 and has a nucleotide sequence encoding MPIF-1Δ23.

*E. coli* JM109 competent cells (Toyobo) were transformed with this plasmid to thereby obtain *E*. *coli* JM109/pNPMPIFNO having the ability to express MPIF-1Δ23.

### EXAMPLE 10

### Comparison of Expression Levels of MPIF-1Δ23 Proteins Encoded by Wild-Type and Optimized Sequences

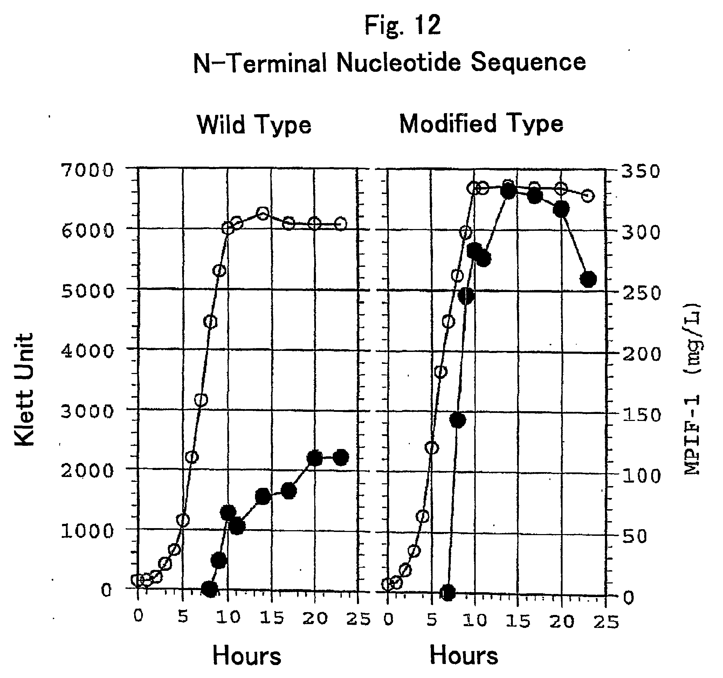

The MPIF-1Δ23-expressing *E. coli* JM109/pNPMPIF obtained in Example 6 and *E. coli* JM109/pNPMPIFNO described above were cultured separately in 1 L of LB medium (1% peptone, 0.5% yeast extract, *0.5%* sodium chloride) containing 10 mg/L tetracycline at 30°C for 16 hr. The resultant culture broth (75 ml each) was transferred to a 3-L jar fermenter containing 1.5 L of a medium for primary fermentation (1.68% sodium monohydrogenphosphate, 0.3% sodium dihydrogenphosphate, 0.1% ammonium chloride, 0.05% sodium chloride, 0.024% magnesium sulfate, 0.02% New Pole LB-625, 0.0005% thiamine hydrochloride, 1.5% glucose, 1.0% casamino acids, 1.0% yeast extract). Then, aeration agitation culture was started at 37°C under a gas flow of 16 L/min at an agitation speed of 200 rpm. When the turbidity of the culture broth reached approx. 4500 Klett units, isopropyl-β-D-thiogalactopyranoside (IPTG) was added at 5.95 mg/l min. Twenty-four min, 130 min and 200 min after the addition of IPTG, 0.75% glucose was added. The cultivation was continued up to 23 hr after the start of the cultivation. A 0.2 ml sample was taken from the culture broth at regular intervals during the cultivation and centrifuged at 12000 rpm for 10 min. The supernatant was discarded, and MPIF-1Δ23 was extracted from the resultant cells, followed by determination of MPIF-1Δ23 expression levels by ELISA. Briefly, cells obtained from 0.2 ml of the culture broth were suspended in 400 µl of 100 mM Tris-HCl (pH 7). To 50 µl of this suspension, 400 µl of an extraction buffer (100 mM Tris-HCl (pH 7) + 2.5% SDS) was added. The resultant mixture was subjected to sonication for 5 min using a ultrasonic cell disruption apparatus BIORUPTOR (Olympus) to thereby extract MPIF-1Δ23. ELISA was performed as described below using an anti-MPIF-1 antibody (DAKO Japan) as a primary antibody and the same antibody biotinylated with Sulfo-NHS-Biotin (Funakoshi) as a secondary antibody. Briefly, 100 µl of a coating buffer (0.293% sodium hydrogencarbonate, 0.159% sodium carbonate sodium azide) containing 4 µg/ml of the anti-MPIF-1 antibody was dispensed into each well of a 96-well microplate (Nunc) and left overnight at 4°C. After washing each well with a washing buffer (PBS + 0.1% Tween 20), 50 µl of MPIF-1Δ23 extract diluted with a dilution buffer (PBS, 10% Block Ace, 0.1% Tween 20, 0.02% Merthiolate) was added to each well and left for 2 hr at room temperature. After washing each well with the washing buffer, 100 µl of biotinylated anti-MPIF-1 antibody diluted with the dilution buffer (2 µg/ml) was added thereto and left for 1 hr at room temperature. After washing each well with the washing buffer, 100 µl of a 500-fold dilution of alkali phosphatase streptavidin (Vector) in the washing buffer was added thereto and left for 1 hr at room temperature. After washing each well with the washing buffer, 100 µl of a 500-fold dilution of alkali phosphatase streptavidin (Vector) in the washing buffer was added thereto and left for I hr at room temperature. After washing each well with the washing buffer, a substrate reaction was carried out using Bluephos Microwell Phosphatase Substrate System (KPL) at 37°C for 20 min. Then, the reaction was terminated by adding 100 µl of 2.5% EDTA-4Na solution. Absorbance at 620 nm was measured with a microplate reader (Labsystems). As a standard, an MPIF-1Δ23 protein prepared by the method described in WO 00/40610 was used. The time course of the cultivation and the results of measurement of expression levels are shown in Fig. 12. In the expression of MPIF-1Δ23 proteins, a higher expression of the protein having the nucleotide sequence selected in Example 6 by the process of the invention at its N-terminal was recognized, compared to the protein comprising the corresponding wild-type nucleotide sequence.

### EXAMPLE 11

### Construction of Plasmid pKMNP for Searching for High Expression Sequences

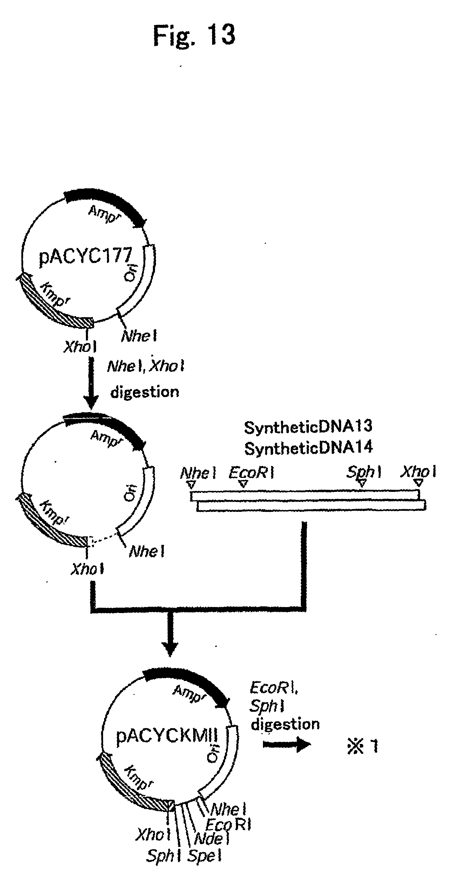

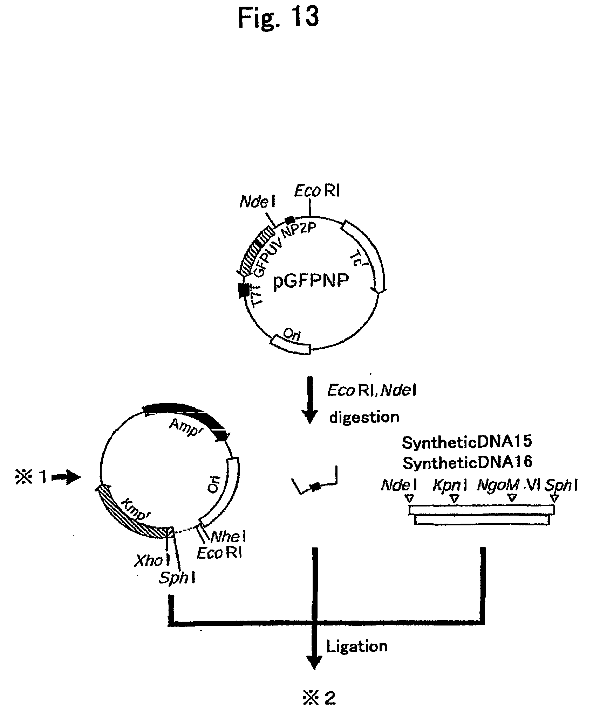

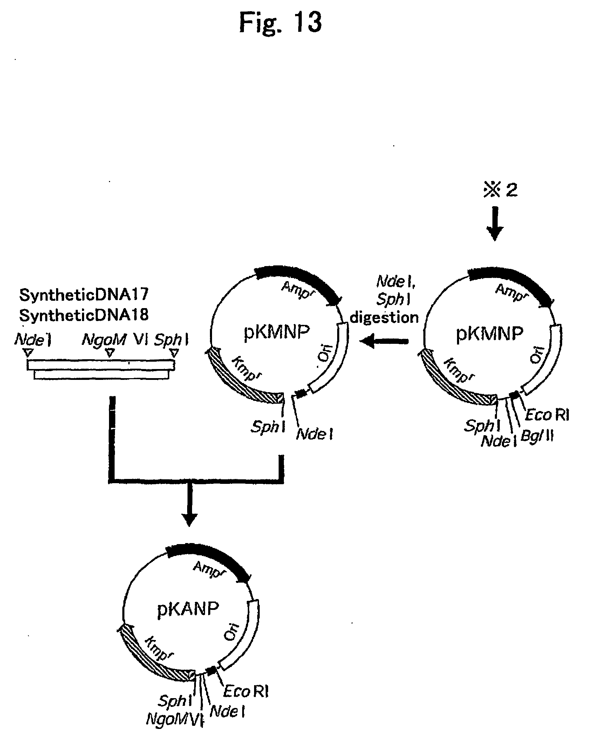

Plasmid pKMNP for searching for high expression sequences using kanamycin resistance resulted from the expression of kanamycin resistance gene was constructed as described below (Fig. 13).

Plasmid pACYC177 (Wako Purechemical Industries) was digested with restriction enzymes NheI and XhoI, and subjected to agarose gel electrophoresis to cut out an approx. 3.6 kbp band. The DNA recovered from the gel using QIAquick Gel Extraction Kit (Qiagen). One hundred pmol each of synthetic DNA 13: CTAGCGAATTCGAGCATATGAGCACTAGTGCATGCGAGCCATATTCAACGGGAA ACGTCTTGC (SEQ ID NO: 75) and synthetic DNA 14: TCGAGCAAGACGTTTCCCGTTGAATATGGCTCGCATGCACTAGTGCTCATATGCT CGAATTCG (SEQ ID NO: 76) were dissolved in TE buffer, retained at 90°C for 10 min, and then gradually cooled to room temperature to perform annealing. The thus annealed DNA fragment was inserted into pACYC177 between the NheI and XhoI sites using Ligation kit ver. 2 (Takara Shuzo) to thereby obtain plasmid pACYCMII in which EcoRI and SphI restriction sites have been introduced upstream of its kanamycin resistance gene.

Plasmid pACYCMII was digested with EcoRI and SphI, and subjected to agarose gel electrophoresis to cut out an approx. 3.6 kbp band. The DNA was recovered from the gel using QIAquick Gel Extraction Kit (Qiagen). Plasmid pGFPNP obtained in Example 1 was digested with EcoRI and NdeI, and subjected to agarose gel electrophoresis to cut out an approx. 0.15 kbp band. The DNA was recovered from the gel using QIAquick Gel Extraction Kit (Qiagen). One hundred pmol each of synthetic DNA 15: TATGAGGGTACCGCCGGCTGCATG (SEQ ID NO: 77) and synthetic DNA 16: CAGCCGGCGGTACCCTCA (SEQ ID NO: 78) were dissolved in TE buffer, retained at 90°C for 10 min, and then gradually cooled to room temperature to perform annealing. The thus annealed DNA fragment and the EcoRI-NdeI fragment from pGFPNP were inserted into pACYCMII between the EcoRI and SphI sites using Ligation kit ver. 2 (Takara Shuzo) to thereby obtain plasmid pKMNP.

Plasmid pKMNP was digested with NdeI and SphI, and subjected to agarose gel electrophoresis to cut out an approx. 3.8 kbp band. The DNA was recovered from the gel using QIAquick Gel Extraction Kit (Qiagen). One microgram each of synthetic DNA 17: TATGAGGGTACCGCCGGCACTGCATG (SEQ ID NO: 79) and synthetic DNA 18: CACTGCCGGCGGTACCCTCA (SEQ ID NO: 80) were reacted in 100 µl of a phosphorylation reaction solution [50mM Tris-HCl (pH 7.6), 10mM MgCl₂, 1mM spermidine, 10mM dithiothreitol, 0.1 mg/ml bovine serum albumin, 1mM ATP, 10 units of T4 polynucleotide kinase (Nippon Gene)] for 1 hr at 37°C to phosphorylate the 5' end. Then, the reaction solution was retained at 90°C for 10 min and gradually cooled to room temperature to perform annealing. The resultant DNA fragment was inserted into pKMNP between the NdeI and SphI sites using Ligation kit ver. 2 (Takara Shuzo) to thereby obtain plasmid pKANP. By inserting random nucleotide sequences encoding an N-terminal amino acid sequence of the protein of interest between the NdeI and NgoMIV sites of plasmid pKANP located upstream of its kanamycin resistance structural gene, it is possible to select sequences of high expression efficiency using kanamycin resistance as an indicator.

### EXAMPLE 12

### Construction of an Expression Plasmid for ZAQ-1 Ligand Encoded by Wild Type Nucleotide Sequence

A ZAQ-1 ligand expression plasmid whose structural gene is a wild type nucleotide sequence was constructed as described below.

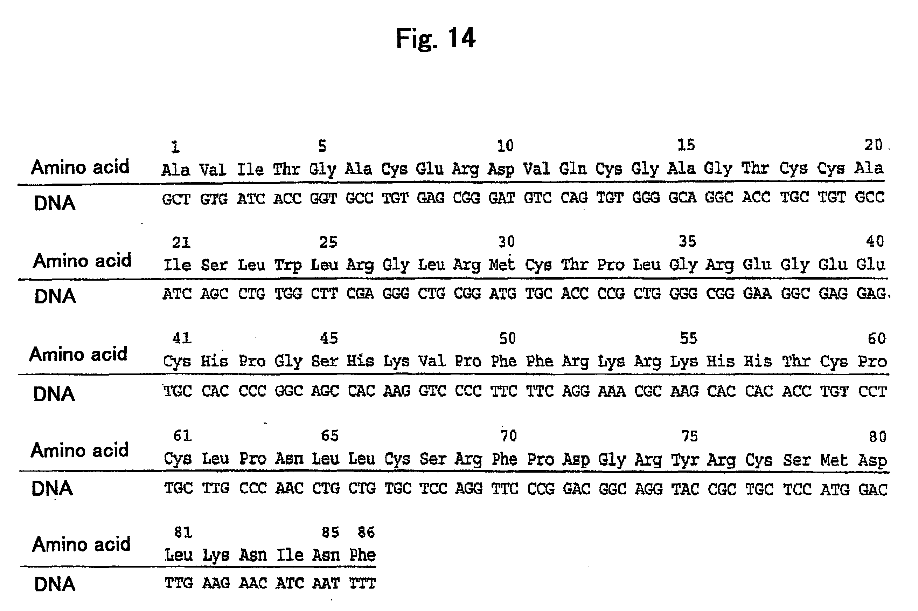

A ZAQ-1 ligand structural gene for which the amino acid sequence and the wild type nucleotide sequence are shown in Fig. 14 was amplified from plasmid pHMITG by PCR using synthetic DNA 19: CTCATATGGCTGTGATCACCGGTGCCTGTG (SEQ ID NO: 81) located immediately upstream of the structural gene and having an NdeI restriction site and Met, and synthetic DNA 20: GCGGATCCCTAAAAATTGATGTTCTTCAAG (SEQ ID NO: 82) located immediately downstream of the structural gene and having a termination codon and a BamHI restriction site. This gene was ligated to pCRII-TOPO vector using TOPO TA Cloning Kit (Invitrogen) to thereby prepare pCRII/GAL. This plasmid was transformed into TOP10 One Shot Competent Cells and plated on X-gal (5-bromo-4-chloro-3-indolyl-β-D-galactose)-coated LB agar medium (1% peptone, 0.5% yeast extract, 0.5% sodium chloride, 2% agar) containing 50 µg/ml ampicillin, followed by cultivation at 37°C for one day. Then, transformants were selected using tetracycline resistance and β galactosidase activity as indicators. The transformant was cultured overnight in LB medium (1% peptone, 0.5% yeast extract, 0.5% sodium chloride) containing 50 µg/ml ampicillin, followed by recovery of plasmid pCRII-TOPOh1ZAQ using QIAprep8 Miniprep Kit (Qiagen). This plasmid pCRII-TOPOh1ZAQ was digested with restriction enzymes NdeI and BamHI, and subjected to agarose gel electrophoresis to cut out an approx. 0.3 kbp band. The DNA was recovered from the gel using QIAquick Gel Extraction Kit (Qiagen).

Plasmid pTCII described in WO 00/40610 was digested with restriction enzymes NdeI and BamHI, and subjected to agarose gel electrophoresis to cut out an approx. 4.6 kbp band. The DNA was recovered from the gel using QIAquick Gel Extraction Kit (Qiagen). The DNA fragment prepared above comprising the ZAQ-1 ligand structural gene was ligated to plasmid pTCII between the NdeI and BamHI sites using Ligation kit ver. 2 (Takara Shuzo) to thereby prepare an expression plasmid pTCIIZAQN for expressing ZAQ-1 ligand having the wild type nucleotide sequence.

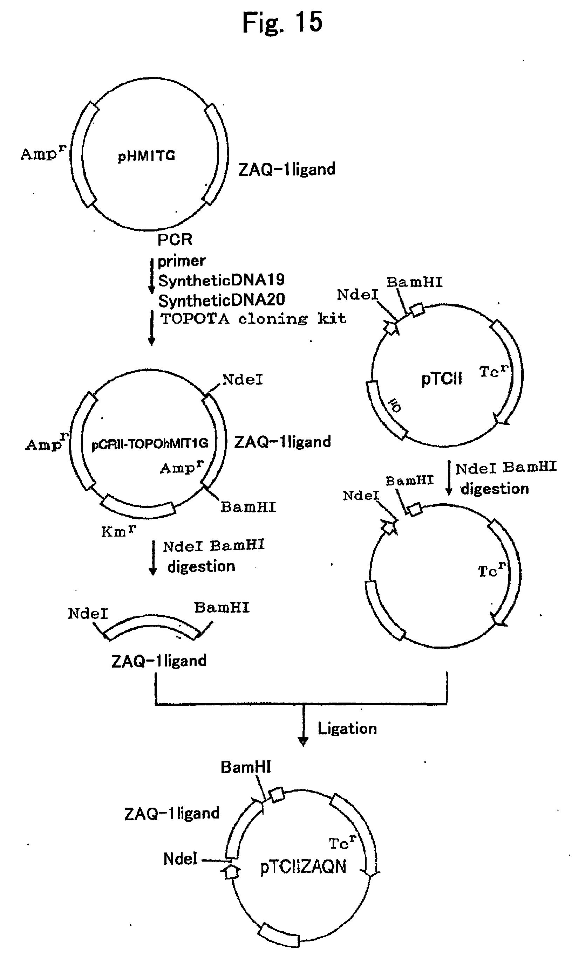

Plasmid pTCIIZAQN was transformed into *E. coli* MM294 (DE3) cells, which were plated on LB agar medium containing 10 µg/ml tetracycline and cultured at 37°C for one day. As a result, clone MM294 (DE3)/pTCIIZAQN was obtained that expresses a ZAQ-1 ligand having a nucleotide sequence with highly frequently used codons (Fig. 15).

### EXAMPLE 13

### Construction of an Expression Plasmid for ZAQ-1 Ligand Encoded by Nucleotide Sequence with Highly Frequently Used Codons

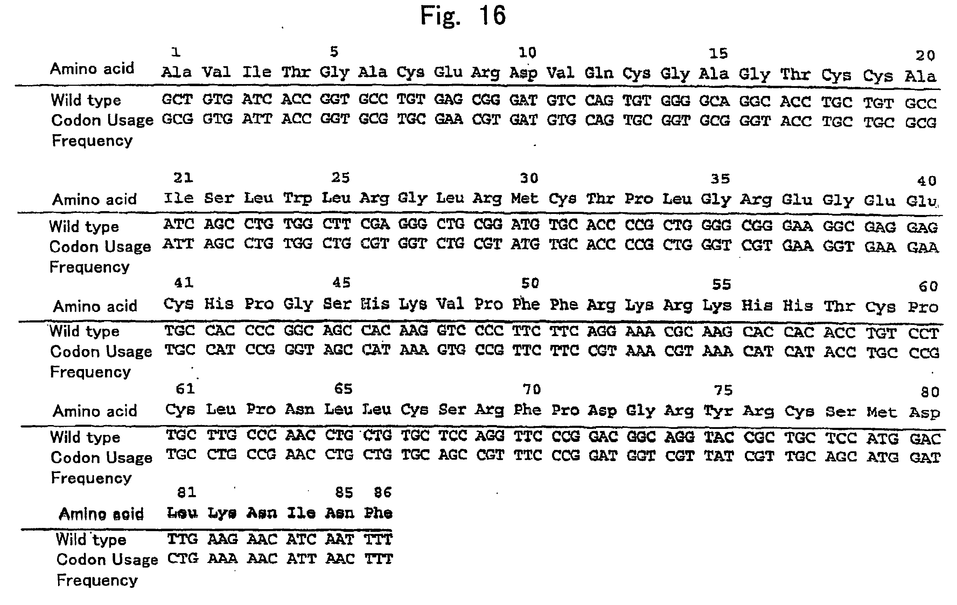

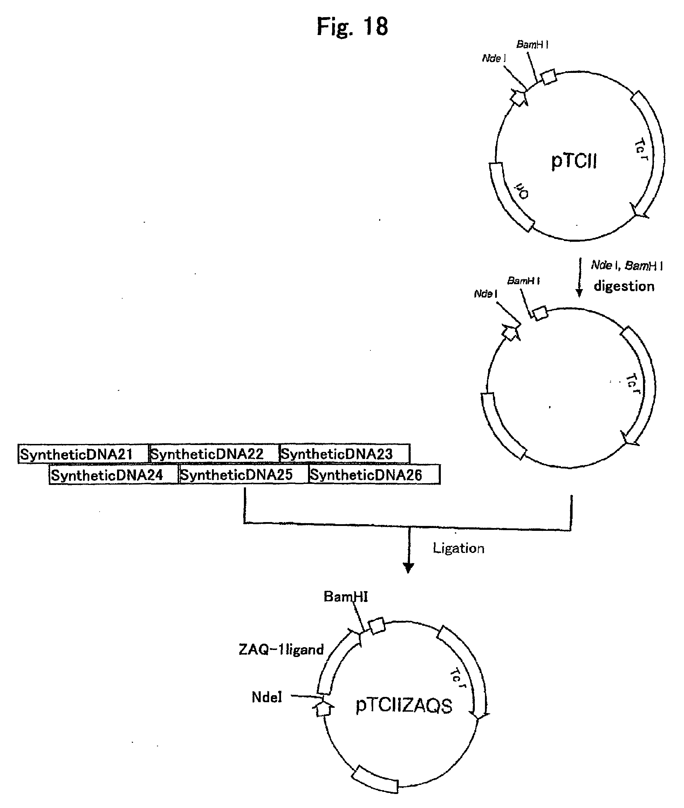

An expression plasmid was constructed that has a structural gene comprising those codons used highly frequently in *E. coli.* With reference to the frequency of usage of each codon in *E. coli* disclosed in Codon Usage Database (http://www.kazusa.or.jp/codon/), the nucleotide sequence shown in Fig. 16 was prepared and used as a ZAQ-1 ligand structural gene. In Fig. 16, the upper row shows the amino acid sequence of ZAQ-1 ligand; the middle row shows the wild type nucleotide sequence encoding ZAQ-1 ligand; and the bottom row shows the nucleotide sequence comprising highly frequently used codons. The ZAQ-1 ligand structural gene was prepared as described below (Fig. 18).

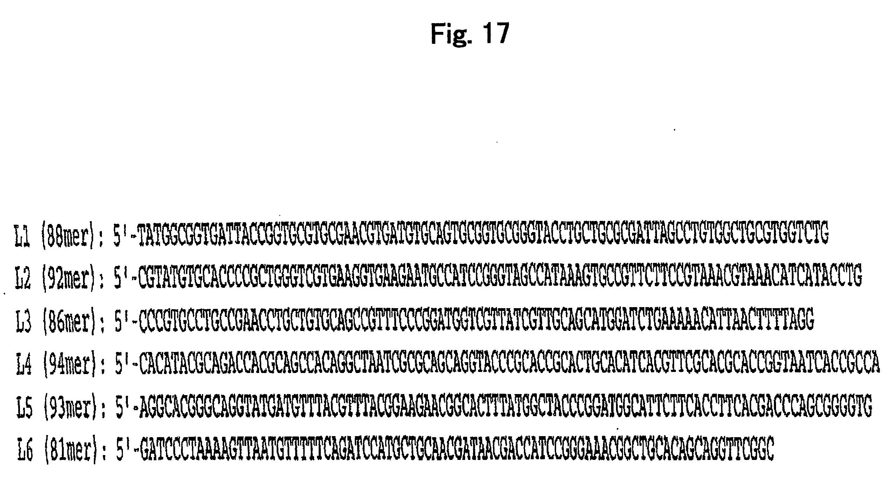

The six DNA fragments shown in Fig. 17 were used: synthetic DNA 21:

TATGGCGGTGATTACCGGTGCGTGCGAACGTGATGTGCAGTGCGGTGCGGGTAC
CTGCTGCGCGATTAGCCTGTGGCTGCGTGGTCTG (SEQ ID NO: 83),

synthetic DNA 22:
CGTATGTGCACCCCGCTGGGTCGTGAAGGTGAAGAATGCCATCCGGGTAGCCAT
AAAGTGCCGTTCTTCCGTAAACGTAAACATCATACCTG (SEQ ID NO: 84),

synthetic DNA 23:
CCCGTGCCTGCCGAACCTGCTGTGCAGCCGTTTCCCGGATGGTCGTTATCGTTG
CAGCATGGATCTGAAAAACATTAACTTTTAGG (SEQ ID NO: 85),

synthetic DNA 24:
CACATACGCAGACCACGCAGCCACAGGCTAATCGCGCAGCAGGTACCCGCACC
GCACTGCACATCACGTTCGCACGCACCGGTAATCACCGCCA (SEQ ID NO: 86),

synthetic DNA 25:
AGGCACGGGCAGGTATGATGTTTACGTTTACGGAAGAACGGCACTTTATGGCTA
CCCGGATGGCATTCTTCACCTTCACGACCCAGCGGGGTG (SEQ ID NO: 87), and

synthetic DNA 26:
GATCCCTAAAAGTTAATGTTTTTCAGATCCATGCTGCAACGATAACGACCATCCG
GGAAACGGCTGCACAGCAGGTTCGGC (SEQ ID NO: 88).

### a) Phosphorylation of DNA Oligomers

One microgram each of the four synthetic DNAs (excluding synthetic DNAs 21 and 26 that are to form 5' ends) were reacted in 100 µl of a phosphorylation reaction solution [50mM Tris-HCl (pH 7.6), 10mM MgCl₂, 1mM spermidine, 10mM dithiothreitol, 0.1 mg/ml bovine serum albumin, 1mM ATP, 10 units of T4 polynucleotide kinase (Nippon Gene)] for 1 hr at 37°C to phosphorylate the 5' ends. After phenol treatment, the aqueous layer was recovered. Two volumes of ethanol was added thereto and cooled to -70°C. Then, the DNA was precipitated by centrifugation.

### b) Ligation of DNA Fragments

The phosphorylated DNA fragments obtained in a) above and 1 µg each of synthetic DNAs 21 and 26 were mixed to give a mixed solution of 120 µl. This mixed solution was retained at 80°C for 10 min and then gradually cooled to room temperature to perform annealing. A ligation reaction was performed using TaKaRa DNA Ligation Kit ver. 2 (Takara Shuzo). Briefly, 30 µl of solution II was added to 30 µl of the annealing solution and mixed well; then, 60 µl of solution I was added thereto and reacted at 37°C for 1 hr to perform ligation. After phenol treatment, the aqueous layer was recovered. Two volumes of ethanol was added thereto and cooled to -70°C. Then, the DNA was precipitated by centrifugation.

### c) Phosphorylation of the 5' End

The precipitate was dissolved in 10 µl of TE buffer (10 mM Tris-HCl (pH 8.0), mM EDTA) and reacted in 100 µl of a phosphorylation reaction solution [50mM Tris-HCl (pH 7.6), 10mM MgCl₂, 1mM spermidine, 10mM dithiothreitol, 0.1 mg/ml bovine serum albumin, 1mM ATP, 10 units of T4 polynucleotide kinase (Nippon Gene)] at 37°C for 1 hr to phosphorylate the 5' end. After phenol treatment, the aqueous layer was recovered. Two volumes of ethanol was added thereto and cooled to -70°C. Then, the DNA was precipitated by centrifugation and dissolved in 20 µl of TE buffer.

### d) Construction of Expression Plasmid pTCIIZAQS

The NdeI-BamHI fragment from plasmid pTCII described in Example 12 and the ZAQ-1 ligand structural gene prepared as described above were subjected to a ligation reaction using TaKaRa DNA Ligation Kit ver. 2 (Takara Shuzo). Briefly, 1 µl of a solution of NdeI-BamHI fragment from pTCII and 4 µl of a solution of the ZAQ-1 ligand structural gene were mixed. To this mixture, 5 µl of solution I was added and reacted at 16°C for 30 min for ligation. Using 10 µl of this ligation solution, *E. coli* JM109 competent cells (Yoyobo) were transformed, plated on LB agar medium containing 10 µg/ml tetracycline, and cultured at 37°C for one day. The resultant tetracycline resistant colonies were selected. This transformant was cultured in LB medium overnight, followed by preparation of plasmid pTCIIZAQS using QIAprep8 Miniprep Kit (Qiagen). The nucleotide sequence of the ZAQ-1 ligand structural gene moiety was confirmed with Applied Biosystems Model 377 DNA Sequencer. Plasmid pTCIIZAQS was transformed into *E. coli* MM294 (DE3), which was then plated on LB agar medium containing 10 *µ*g/ml tetracycline and cultured at 37°C for one day. As a result, clone MM294 (DE3)/pTCIIZAQ was obtained that expresses a ZAQ-1 ligand having a nucleotide sequence comprising those codons highly frequently used in *E. coli*.

### EXAMPLE 14

### Search for ZAQ-1 Ligand Sequence Optimized for Expression

Plasmid pKANP constructed in Example 11 was digested with restriction enzymes NdeI (Takara Shuzo) and NgoMIV (New England BioLabs), and subjected to agarose gel electrophoresis to cut out an approx. 3.8 kbp band. The DNA was recovered from the gel using QIAquick Gel Extraction Kit (Qiagen).

Random nucleotide sequences encoding amino acids of an N-terminal portion of ZAQ-1 ligand were prepared as described below. Briefly, double-stranded DNA fragments were prepared with *Pyrobest* DNA polymerase (Takara Shuzo) using a 1:2 mixture of mixed bases-containing synthetic DNA 27: AGAGTTTGCCGGCACCGGTCCGGTACCCGCACCGCACTGCACRTCNCGYTCRC ANGCNCCNGTDATNACNGCCATATGTTGGCGC (SEQ ID NO: 89) and synthetic DNA 28: AGAGTTTGCCGGCACCGGTCCGGTACCCGCACCGCACTGCACRTCYCTYTCRC ANGCNCCNGTDATNACNGCCATATGTTGGCGC (SEQ ID NO: 90) as templates and synthetic DNA 29: GCGCCAACATATG (SEQ ID NO: 91). The extension reaction solution was composed of 3 µl of *Pyrobest* DNA polymerase (5 U/µl), 10 µl of 10x reaction buffer attached to the polymerase, 16 µl of dNTP mixture, 0.5 µl of synthetic DNA 35(1 µg/µl), 1µl of synthetic DNA 36(1 µg/µl), 2µl of synthetic DNA 29 (100 µmol/L) and 67.5 µl of distilled water. The extension reaction was performed at 94°C for 30 sec, at 58°C for 30 sec and at 70°C for 5 min. DNA fragments were purified from the reaction solution after the extension reaction using QIAquick Gel Extraction Kit, followed by digestion of the double-stranded DNA fragments with NdeI (Takara Shuzo) and NgoMIV (New England BioLabs). This digestion was performed as described below.

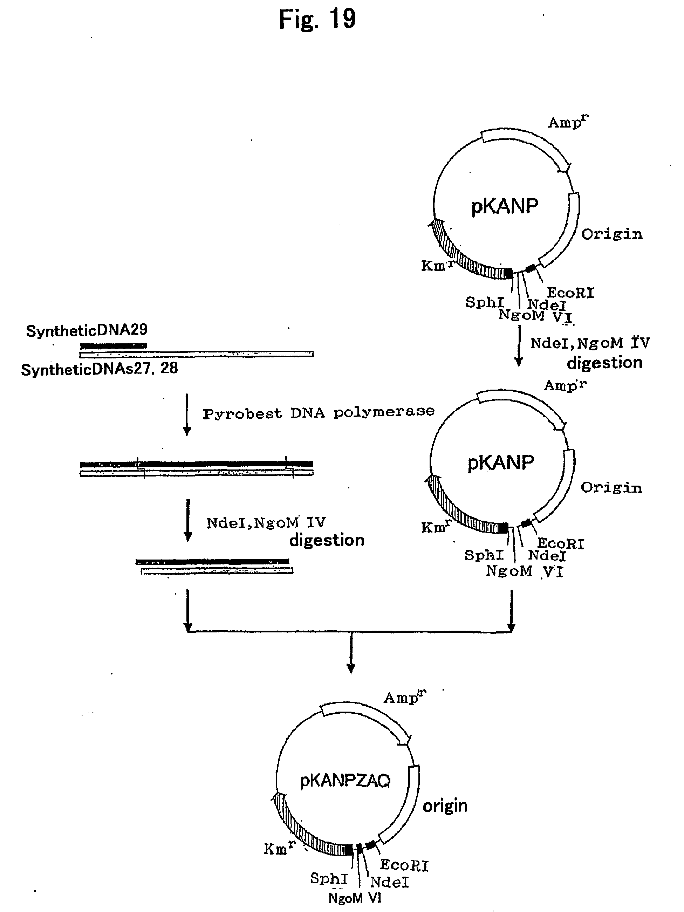

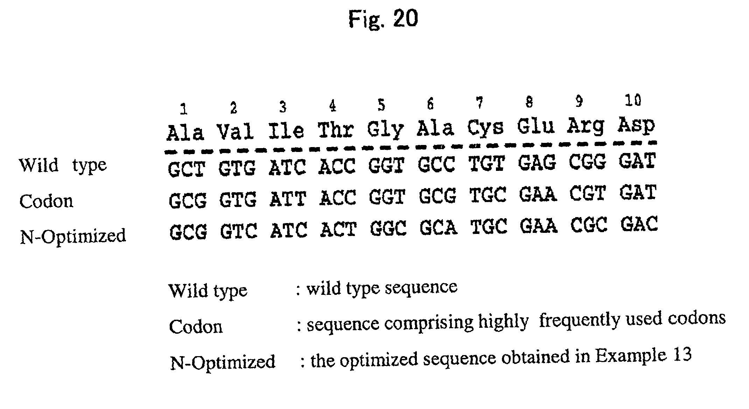

A reaction solution consisting of 27 µl of the purified DNA fragment solution, 3.5 µl of Buffer 4 (New England BioLabs), 1.5 µl of distilled water, 1.5 µl of NdeI and 1.5 µl of NgoMIV was retained at 37°C for 2 hr to perform digestion. Then, an approx. 70 bp band was cut out through agarose gel electrophoresis, and the DNA was recovered from the gel using QIAquick Gel Extraction Kit (Qiagen). These DNA fragments were inserted into pKANP between the NdeI and NgoMIV sites using Ligation kit ver. 2 (Takara Shuzo) to construct a set of plasmid pKANPZAQ for searching for optimized nucleotide sequences (Fig. 19). *E. coli* MM294 was transformed with plasmid pKANPZAQ, plated on LB agar medium containing 50 µg/ml kanamycin and cultured at 37°C for one day for colony formation. Resultant colonies were subjected to colony PCR using synthetic DNA 30: ACCTGATTGCCCGACATTATCGC (SEQ ID NO: 92) and synthetic DNA 31: CACCCTCATCAGTGCCAACATAG (SEQ ID NO: 93), and the insertion of a DNA of interest was confirmed by agarose gel electrophoresis. The product of this colony PCR was purified QIAquick PCR Purification Kit (Qiagen), and the nucleotide sequence thereof was analyzed with synthetic DNAs 30 and 31. As a result, the sequence shown in Fig. 20 was obtained.

### EXAMPLE 15

### Construction of Plasmid for Expressing ZAQ-1 Ligand Encoded by Expression Optimized Nucleotide Sequence

Expression plasmid pTCIIZAQS (obtained in Example 13) for a ZAQ-1 ligand encoded by a sequence with highly frequently used codons was digested with restriction enzymes XbaI (Takara Shuzo) and KpnI (Takara Shuzo), and subjected to agarose gel electrophoresis to cut out an approx. 4.8 kbp band. The DNA was recovered from the gel using QIAquick Gel Extraction Kit (Qiagen).

The product of colony PCR obtained in Example 14 was digested with restriction enzymes XbaI (Takara Shuzo) and KpnI (Takara Shuzo), and subjected to agarose gel electrophoresis to cut out an approx. 100 bp band. The DNA was recovered from the gel using QIAquick Gel Extraction Kit (Qiagen). This DNA fragment was inserted into pTCIIZAQS between the XbaI and KpnI sites using Ligation kit ver. 2 (Takara Shuzo) to thereby construct expression plasmid pTCIIZAQNO for a ZAQ-1 ligand encoded by a nucleotide sequence optimized for expression. The nucleotide sequence of the ZAQ-1 ligand structural gene moiety of this plasmid was confirmed with Applied Biosystems Model 3100 DNA Sequencer. The results revealed that this plasmid comprises a DNA (SEQ ID NO: 121) comprising the nucleotide sequence shown in the bottom row in Fig. 20 and yet encoding a ZAQ-1 peptide. Plasmid pTCIIZAQNO was transformed into *E. coli* MM294 (DE3). The resultant transformant was plated on LB agar medium containing 10 µg/ml tetracycline and cultured at 37°C for one day to thereby obtain expression clone MM294 (DE3)/ pTCIIZAQNO for ZAQ-1 ligand having a nucleotide sequence optimized for expression in *E. coli*.

### EXAMPLE 16

### Comparison of Expression Levels among ZAQ-1 Ligand Expression Clones

ZAQ-1 ligand expression clones MM294 (DE3)/ pTCIIZAQN, MM294 (DE3)/pTCIIZAQS and MM294 (DE3)/ pTCIIZAQNO obtained in Examples 12, 13 and 15 were cultured separately in 30 ml of LB medium (1% peptone, 0.5% yeast extract, 0.5% sodium chloride) containing 10 mg/L tetracycline at 30°C for 16 hr. The resultant culture broth (1 ml each) was transferred to a 200 ml Erlenmeyer flask with pleats containing 19 ml of a medium for primary fermentation (1.68% sodium monohydrogenphosphate, 0.3% sodium dihydrogenphosphate, 0.1% ammonium chloride, 0.05% sodium chloride, 0.024% magnesium sulfate, 0.0005% thiamine hydrochloride, 1.5% glucose and 1.0% casamino 47acids). Then, aeration agitation culture was started at 37°C at 200 rpm. When the turbidity of the culture broth reached approx. 150 Klett units, isopropyl- β -D-thiogalacto-pyranoside (IPTG) was added at a final concentration of 0.1 mmol/L min. The cultivationwas continued up to 3 hr after the start of the addition of IPTG.

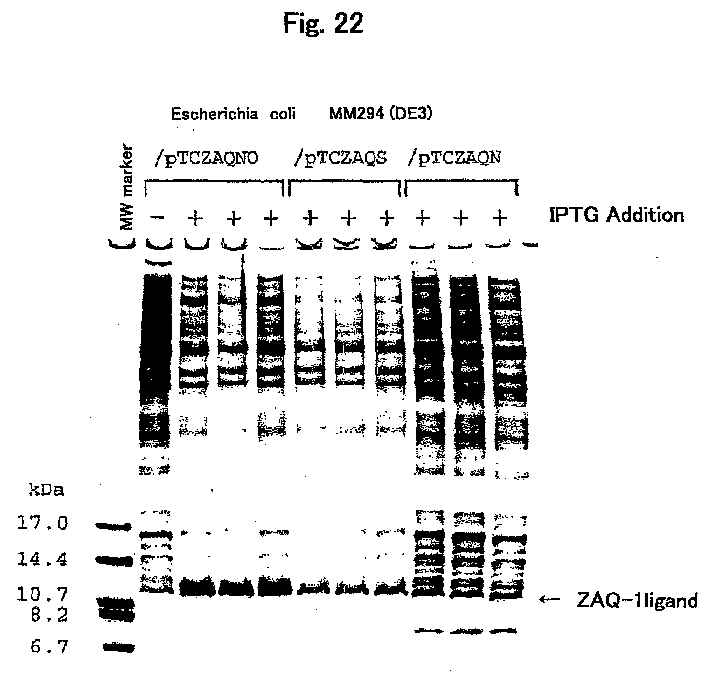

After completion of the cultivation, a 0.2 ml aliquot was taken from the culture broth and centrifuged at 12000 rpm for 10 min. The supernatant was discarded, and the resultant cells were suspended in a sample buffer (125 mM Tris-HCl, 1% sodium dodecyl sulfate, 15% glycerol, 5% 2-mercaptoethanol, 0.005% Bromophenol Blue) and treated at 95°C for 5 min, followed by electrophoresis in Multigel 15/25 (Daiichi Pure Chemicals Co., Ltd.). The resultant gel was stained with Rapid CBB KANTO (Kanto Kagaku). As a result, ZAQ-1 ligand expression clone MM294 (DE3)/pTCIIZAQNO having a nucleotide sequence optimized for expression in *E. coli* exhibited a higher ZAQ-1 ligand expression ability than *E. coli* MM294 (DE3)/pTCIIZAQN carrying an expression plasmid comprising a wild type nucleotide sequence encoding an N-terminal portion of ZAQ-1 ligand and ZAQ-1 ligand expression clone MM294 (DE3)/pTCIIZAQS having a nucleotide sequence comprising those codons used highly frequently in *E. coli* (Fig. 22). From these results, it has become clear that a structural gene using a nucleotide sequence found by the process of the invention is more suitable for expression than a structural gene using a nucleotide sequence comprising highly frequently used codons.

### EXAMPLE 17

### Construction of Rat GALP Direct Expression Plasmid (with Wild Type Nucleotide Sequence)

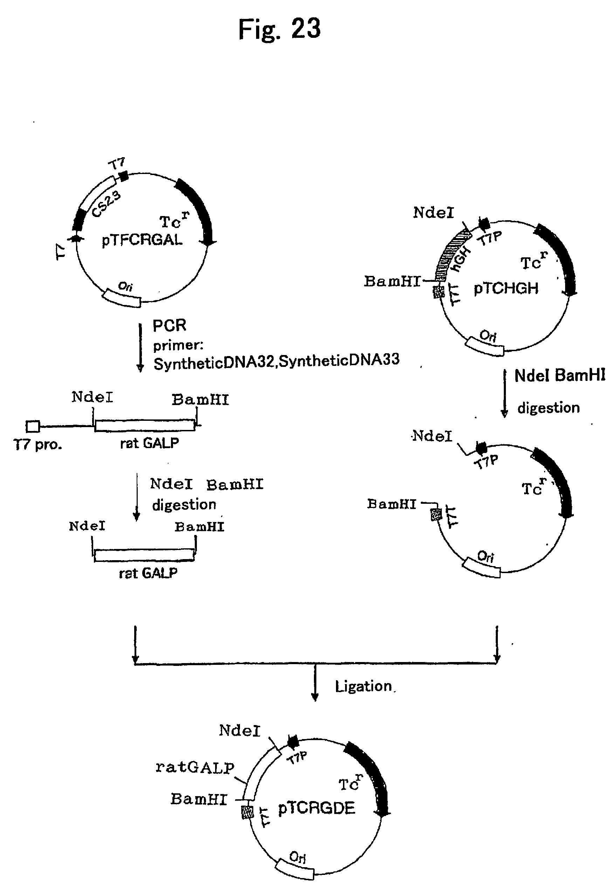

A rat GALP direct expression plasmid having a rat GALP structural gene consisting of the wild type nucleotide sequence was constructed as described below (Fig. 23).

Plasmid pTCHGH-Na described in Reference Example 1 in WO 00/20439 was digested with restriction enzymes NdeI and BamHI, and subjected to agarose gel electrophoresis to cut out an approx. 4.6 kbp band. The DNA was recovered from the gel using QIAquick Gel Extraction Kit (Qiagen) and used as an expression vector.

The GALP structural gene moiety was amplified from the rat GALP fusion protein expression plasmid pTFCRGAL obtained in Example 3. As a primer corresponding to the 17 promoter moiety of pTFCRGAL, synthetic DNA 32: TAATACGACTCACTATAGGG (SEQ ID NO: 94) was used. As a primer to introduce a stop codon and BamHI restriction site downstream of the C-terminal of rat GALP, synthetic DNA 33: ATACATGGATCCTCAGGTCATCCTTGGAGAGC (SEQ ID NO: 95) was used. The amplification was carried out in 100 µl of a reaction solution (containing 50 ng of plasmid pTFCRGAL, 200 pmol each of primers, 1 µl of *Pyrobest* DNA polymerase (Takara Shuzo), 8 µl of dNTP solution attached to the polymerase and 10 µl of x10 reaction buffer attached to the polymerase) for 25 cycles, each cycle consisting of at 94°C for 10 sec, at 55°C for 30 sec and at 72°C for 60 sec. The amplified DNA fragment was purified from the reaction solution using QIAquick PCR Purification Kit (Qiagen). The purified DNA fragment was reacted at 37°C for 12 hr in 30 *µ*l of a reaction solution containing 1 µl of NdeI (Takara Shuzo), 1 µl of BamHI (Takara Shuzo) and 5 µl of Buffer K (Takara Shuzo). After electrophoresis using 4% agarose gel, an approx. 0.2 kbp band was cut out, and the DNA was recovered from the gel using QIAquick Gel Extraction Kit (Qiagen). This DNA fragment was ligated to pTCHGH-Na between the NdeI and BamHI sites using Ligation kit ver. 2 (Takara Shuzo) to thereby obtain expression plasmid pTCRGDE for direct expression of rat GALP.

*E. coli* MM294 (DE3) was transformed with this expression plasmid pTCRGDE to thereby obtain *E. coli* MM 294 (DE3)/pTCRGDE.

### EXAMPLE 18

### Construction of Rat GALP Direct Expression Plasmid (Optimization of N-terminal Nucleotide Sequence)

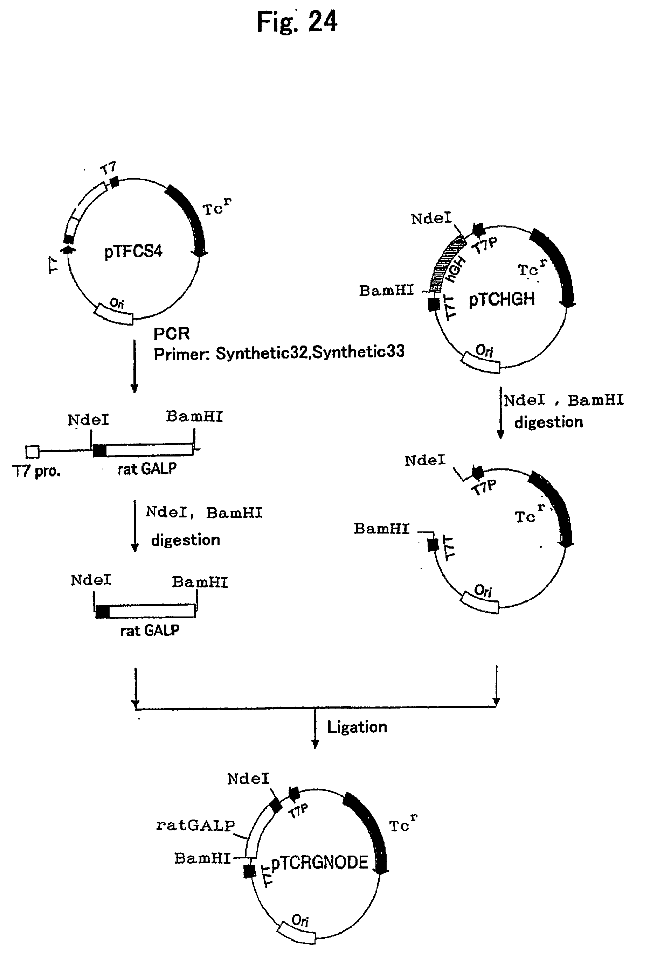

A rat GALP direct expression plasmid having a structural gene in which an N-terminal portion of rat GALP is replaced with the nucleotide sequence optimized for expression found in Example 2 was constructed as described below (Fig. 24).

Expression plasmid pTCRGNODE for direct expression of rat GALP was obtained in the same manner as in Example 17 except that the rat GALP fusion protein expression plasmid pTFCS4 obtained in Example 4 was used as a template. *E. coli* MM294 (DE3) was transformed with expression plasmid pTCRGNODE to thereby obtain *E. coli* MM294 (DE3)/pTCRGNODE.

### EXAMPLE 19

### Search for GALP Full-Length Sequence Optimized for Expression

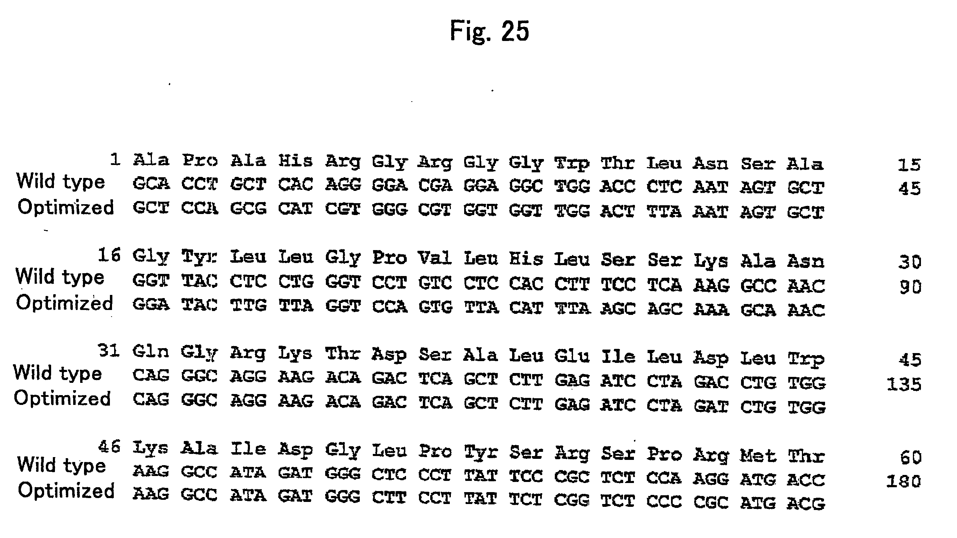

Search for optimized sequences was conducted not only for the nucleotide sequence encoding the N-terminal of GALP searched for in Example 2 but also for other nucleotide sequences encoding other parts. Briefly, the amino acid sequence of rat GALP consisting of 60 amino acids was divided into 4 blocks. Then, nucleotide sequences optimized for expression were searched for in the same manner as in Example 2 for the 3 blocks excluding the N-terminal block examined in Example 2. As a result, the sequence shown in Fig. 25 was obtained.

### EXAMPLE 20

### Construction of Expression Plasmid for GAL Encoded by the Full Length-Optimized Sequence

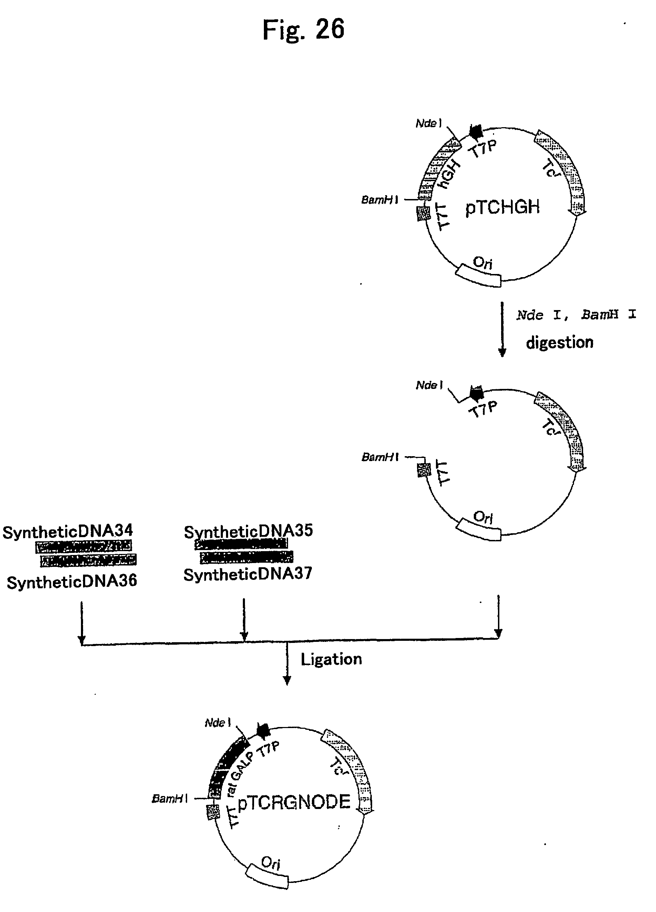

Expression plasmid for rat GALP having the nucleotide sequence found in Example 17 was constructed as described below (Fig. 26).

A rat GALP structural gene was constructed using synthetic DNA 34: TATGGCTCCAGCGCATCGTGGGCGTGGTGGTTGGACTTTAAATAGTGCTGGATA CTTGTTAGGTCCAGTGTTACATTTAAGCAGCAAAGCAAACCAGGGC (SEQ ID NO: 96), synthetic DNA 35: AGGAAGACAGACTCAGCTCTTGAGATCCTAGATCTGTGGAAGGCCATAGATGG GCTTCCTTATTCTCGGTCTCCCCGCATGACGTGAG (SEQ ID NO: 97), synthetic DNA 36: TTGCTTTGCTGCTTAAATGTAACACTGGACCTAACAAGTATCCAGCACTATTTAA AGTCCAACCACCACGCCCACGATGCGCTGGTGCCA (SEQ ID NO: 98) and synthetic DNA 37: GATCCTCACGTCATGCGGGGAGACCGAGAATAAGGAAGCCCATCTATGGCCTTC CACAGATCTAGGATCTCAAGAGCTGAGTCTGTCTTCCTGCCCTGGT (SEQ ID NO: 99). Briefly, synthetic DNAs 34 to 37 were dissolved separately in TE buffer to give a concentration of 50 mmol/L. Three µl of each of the synthetic DNA solution was reacted in a phosphorylation reaction solution [50mM Tris-HCl (pH 7.6), 10mM MgCl₂, 1mM spermidine, 10mM dithiothreitol, 0.1 mg/ml bovine serum albumin, 1mM ATP, 10 units of T4 polynucleotide kinase (Nippon Gene)] for 1 hr at 37°C to phosphorylate the 5'end. The phosphorylated synthetic DNAs 34 and 36 or synthetic DNAs 35 and 37 were mixed, retained at 90°C for 10 min and then gradually cooled to room temperature for annealing. Each mixture was subjected to agarose gel electrophoresis to cut out an approx. 0.1 kbp band, and the DNA was recovered from the gel using QIAquick Gel Extraction Kit (Qiagen). Thus, two double-stranded DNA fragments representing the N-terminal half and C-terminal half of rat GALP were obtained.

The NdeI-BamHI fragment from pTCHGH-Na described in Example 17 was mixed with the above-obtained two double-stranded DNA fragments representing the N-terminal half and C-terminal half of rat GALP and ligated together using Ligation kit ver. 2 (Takara Shuzo) to thereby construct plasmid pTCRGTODE for direct expression of rat GALP encoded by a full length-optimized nucleotide sequence. This plasmid comprises a DNA (SEQ ID NO: 122) comprising the nucleotide sequence shown at the bottom row in Fig. 25 and having a nucleotide sequence encoding rat GALP. *E. coli* MM294 (DE3) was transformed with this expression plasmid pTCRGTODE to thereby obtain *E. coli* MM294 (DE3)/pTCRGTODE.

### EXAMPLE 21

### Direct Expression of GALP Encoded by the Full Length-Optimized Sequence

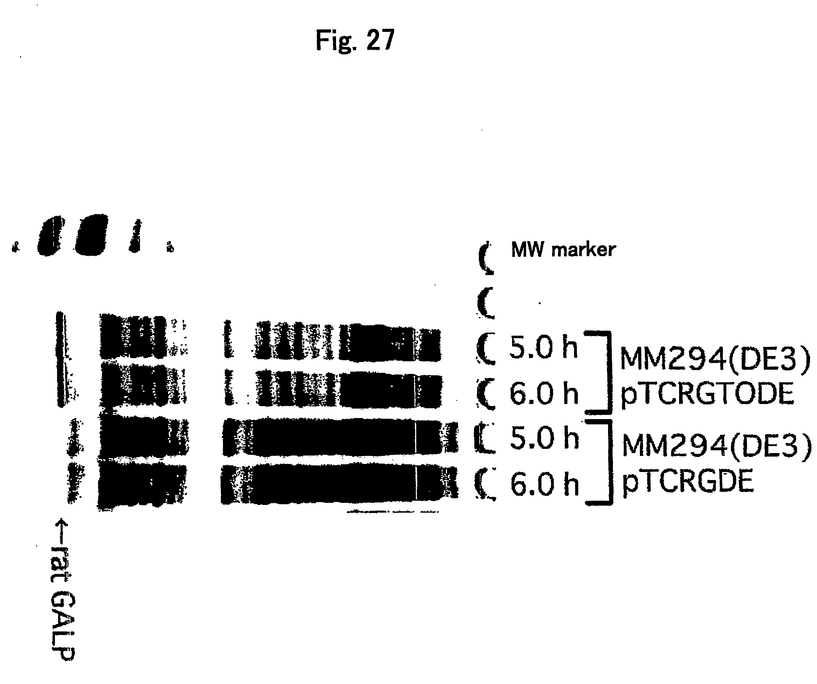

*E. coli* MM294 (DE3)/pTCRGDE obtained in Example 17, *E. coli* MM294 (DE3)/pTCRGNODE obtained in Example 18 and *E. coli* MM294 (DE3)/pTCRGTODE obtained in Example 20 were cultured separately in 1 L of LB medium (1% peptone, 0.5% yeast extract, 0.5% sodium chloride) containing 10 mg/L tetracycline at 30°C for 16 hr. The resultant culture broth (75 ml each) was transferred to a 3-L jar fermenter containing 1.5 L of a medium for primary fermentation (1% peptone, 0.5% yeast extract, 0.5% sodium chloride, 0.02% New Pole LB-625). Then, aeration agitation culture was started at 37°C under a gas flow of 1.5 L/min at an agitation speed of 550 rpm. When the turbidity of the culture broth reached approx. 150 Klett units, isopropyl-β-D-thiogalactopyranoside (IPTG) was added at 50 µmol/L min. A 0.2 ml sample was taken from the culture broth at regular intervals during the cultivation and centrifuged at 12000 rpm for 10 min. The supernatant was discarded, and the resultant cells were suspended in a sample buffer (125 mM Tris-HCl, 1% sodium dodecyl sulfate, 15% glycerol, 5% 2-mercaptoethanol, 0.005% Bromophenol Blue) and treated at 95°C for 5 min, followed by electrophoresis in Multigel 15/25 (Daiichi Pure Chemicals Co., Ltd.). The resultant gel was stained with Rapid CBB KANTO (Kanto Kagaku). As a result, a band of rat GALP was not observed on the SDS-PAGE in the lanes of *E. coli* MM294 (DE3)/pTCRGDE carrying an expression plasmid whose rat GALP structural gene entirely consists of the natural type nucleotide sequence and rat GALP expression clone *E. coli* MM294 (DE3)/pTCRGNODE having a nucleotide sequence optimized only in an N-terminal portion. A band of rat GALP was recognized in the lane of rat GALP expression clone *E. coli* MM294 (DE3)/pTCRGTODE having a nucleotide sequence optimized for the full-length of rat GALP structural gene for the purpose of expression in *E. coli.* Fig. 27 shows the results for MM294 (DE3)/pTCRGDE and MM294 (DE3)/pTCRGTODE. Even when direct expression of a protein encoded by the wild-type nucleotide sequence is difficult in the expression of relatively low molecular weight proteins as seen in this case, it can be expected to make direct expression possible by optimizing the nucleotide sequence for expression according to the process of the invention. Further, it has been demonstrated that high expression is possible by optimizing the nucleotide sequence for the full length of a structural gene when expected expression cannot be obtained by optimizing the nucleotide sequence encoding an N-terminal portion of the gene.

### EXAMPLE 22

### Construction of Plasmids for Measuring Expression Efficiencies in Animal Cells

### 1) Construction of Plasmid pQBI25-2

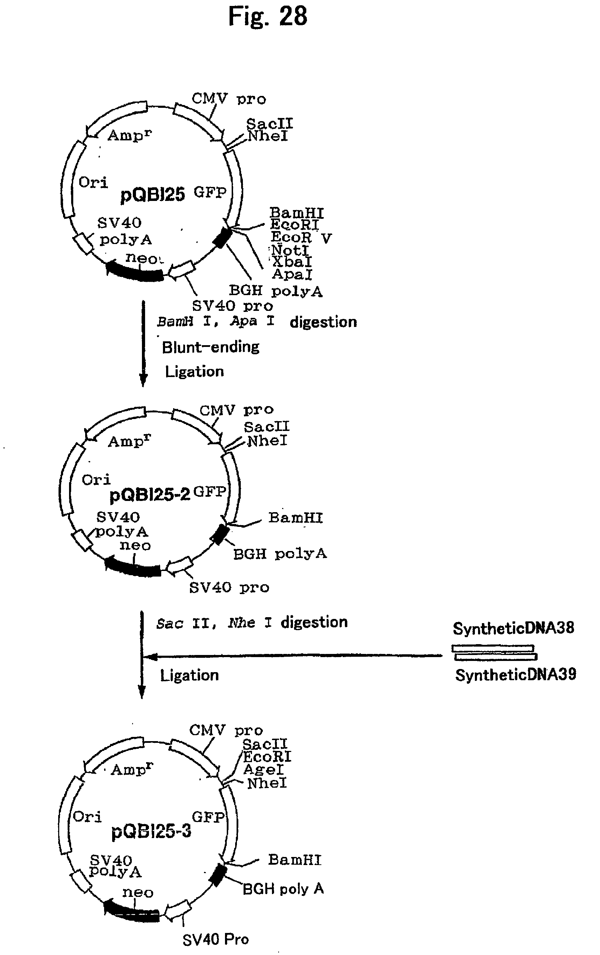

Plasmid pQBI25 (Takara Shuzo) was digested with BamHI (Takara Shuzo) and ApaI (Takara Shuzo) in order to eliminate the EcoRI, EcoRV, NotI, XbaI and ApaI restriction sites located within the multicloning site downstream of the GFP structural gene, followed by blunt-ending using DNA Blunting Kit (Takara Shuzo). Subsequently, an approx. 6.1 kbp band was cut out by agarose gel electrophoresis. The DNA was recovered from the gel using QIAquick Gel Extraction Kit (Takara Shuzo), followed by self-ligation using Ligation Kit Version 2 (Takara Shuzo) to thereby obtain plasmid pQBI25-2. Plasmid pQBI25-2 was digested with SacII (Takara Shuzo) and NheI (Takara Shuzo), and an approx. 6.1 kbp band was cut out by agarose gel electrophoresis. The DNA was recovered from the gel using QIAquick Gel Extraction Kit (Takara Shuzo). One hundred pmol each of synthetic DNA 38: GGAATTCGCTCGCACCGGTAGAAAAAATGG (SEQ ID NO: 100) comprising EcoRI and AgeI restriction sites and synthetic DNA 39: CTAGCCATTTTTTCTACCGGTGCGAGCGAATTCCGC (SEQ ID NO: 101) comprising a nucleotide sequence complementary thereto were dissolved in TE buffer, retained at 90°C for 10 min, and then gradually cooled to room temperature for annealing. The resultant double-stranded DNA fragment was inserted into pQBI25-2 between the ScaII and NheI sites using Ligation Kit Version 2 (Takara Shuzo) to thereby obtain plasmid pQBI25-3 (Fig. 28).

Plasmid pQBI25-3 is an expression plasmid that comprises a GFP structural gene transcribed from CMV promoter in eukaryotic cells and has SacII, EcoRI and AgeI restriction sites upstream of the GFP structural gene. A DNA fragment can be inserted into this plasmid using these restriction sites.

### EXAMPLE 23

### Construction of Expression Plasmid for Comparing Expression Levels of GFP Proteins with Different Nucleotide Sequences encoding N-Terminal Amino Acids

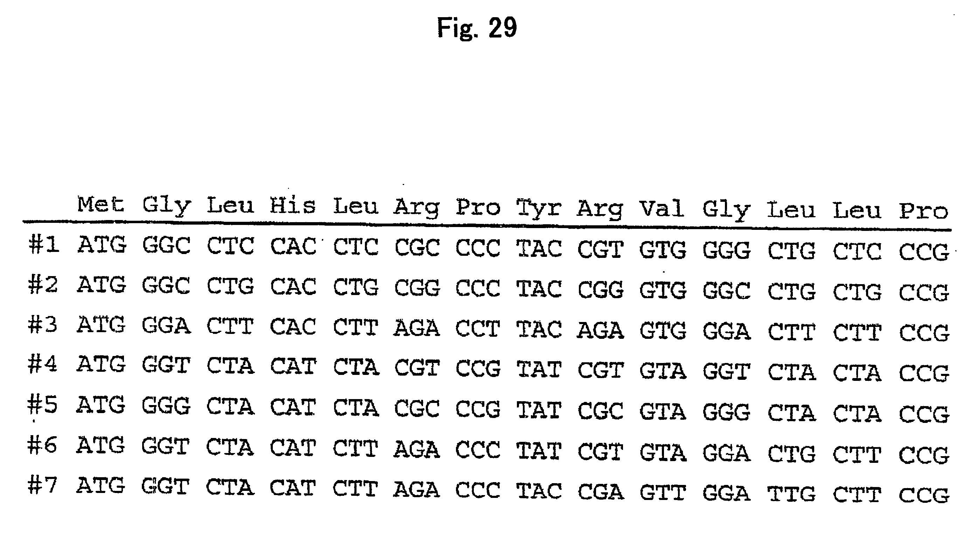

A set of expression plasmids that have seven nucleotide sequences each encoding the amino acid sequence from the N-terminal to the 13th residue of human LLPL (lecithin cholesterol acyltransferase-like lysophospholipase, Biochem Biophys Res Commun. Vo. 257, 50 (1999)) upstream of their GFP structural gene were prepared as described below. Sequence #1 represents the wild type nucleotide sequence (Fig. 29).

Plasmid pQGI-25-3 obtained in Example 22 was digested with EcoRI (Takara Shuzo) and AgeI (Takara Shuzo), and an approx. 6.1 kbp band was cut out by agarose gel electrophoresis. The DNA was recovered from the gel using QIAquick Gel Extraction Kit (Takara Shuzo).

The following synthetic DNAs (50 pmol each) were dissolved in TE buffer separately, and their 5' ends were phosphorylated with T4 Polynucleotide Kinase (Wako Purechemical Industries). Sequence #1 was obtained by mixing the 5' end phosphorylation reaction solutions of synthetic DNA 40: AATTCCGTCGTACACCATGGGCCTCCACCTCCGCCCCTACCGTGTGGGGCTGCT C (SEQ ID NO: 102) and synthetic DNA 41: CCGGGAGCAGCCCCACACGGTAGGGGCGGAGGTGGAGGCCCATGGTGTACGA CGG (SEQ ID NO: 103) comprising a nucleotide sequence complementary thereto, retaining the mixture at 90°C for 10 min and gradually cooling to room temperature for annealing. The resultant double-stranded DNA fragment was ligated into plasmid pQBI25-3 between the EcoRI and AgeI sites using Ligation Kit Version 2 (Takara Shuzo) to thereby obtain plasmid pQBI25-3LLPL#1. Sequence #2 was prepared from synthetic DNA 42: AATTCCGTCGTACACCATGGGCCTGCACCTGCGGCCCTACCGGGTGGGCCTGCT G (SEQ ID NO: 104) and synthetic DNA 43: CCGGCAGCAGGCCCACCCGGTAGGGCCGCAGGTGCAGGCCCATGGTGTACGAC GG (SEQ ID NO: 105) comprising a nucleotide sequence complementary thereto; sequence #3 was prepared from synthetic DNA 44: AATTCCGTCGTACACCATGGGACTTCACCTTAGACCTTACAGAGTGGGACTTCT T (SEQ ID NO: 106) and synthetic DNA 45: CCGGAAGAAGTCCCACTCTGTAAGGTCTAAGGTGAAGTCCCATGGTGTACGAC GG (SEQ ID NO: 107) comprising a nucleotide sequence complementary thereto; sequence #4 was prepared from synthetic DNA 46: AATTCCGTCGTACACCATGGGTCTACATCTACGTCCGTATCGTGTAGGTCTACTA (SEQ ID NO: 108) and synthetic DNA 47: CCGGTAGTAGACCTACACGATACGGACGTAGATGTAGACCCATGGTGTACGACG G (SEQ ID NO: 109) comprising a nucleotide sequence complementary thereto; sequence #5 was prepared from synthetic DNA 48: AATTCCGTCGTACACCATGGGGCTACATCTACGCCCGTATCGCGTAGGGCTACTA (SEQ ID NO: 110) and synthetic DNA 49: CCGGTAGTAGCCCTACGCGATACGGGCGTAGATGTAGCCCCATGGTGTACGACG G (SEQ ID NO: 111) comprising a nucleotide sequence complementary thereto; sequence #6 was prepared from synthetic DNA 50: AATTCCGTCGTACACCATGGGTCTACATCTTAGACCCTATCGTGTAGGACTGCTT (SEQ ID NO: 112) and synthetic DNA 51: CCGGAAGCAGTCCTACACGATAGGGTCTAAGATGTAGACCCATGGTGTACGACG G (SEQ ID NO: 113) comprising a nucleotide sequence complementary thereto; sequence #7 was prepared from synthetic DNA 52: AATTCCGTCGTACACCATGGGTCTACATCTTAGACCCTACCGAGTTGGATTGCTT (SEQ ID NO: 114) and synthetic DNA 53: CCGGAAGCAATCCAACTCGGTAGGGTCTAAGATGTAGACCCATGGTGTACGACG G (SEQ ID NO: 115) comprising a nucleotide sequence complementary thereto; and inserted into the plasmid in the same manner as described for sequence #1 to thereby obtain plasmids pQBI25-3#2 through pQBI25-3#7.

### EXAMPLE 24

### Comparison of Expression Levels of GFP Proteins with Different Nucleotide Sequences in the N-terminal in Temporary Expression in Animal Cells

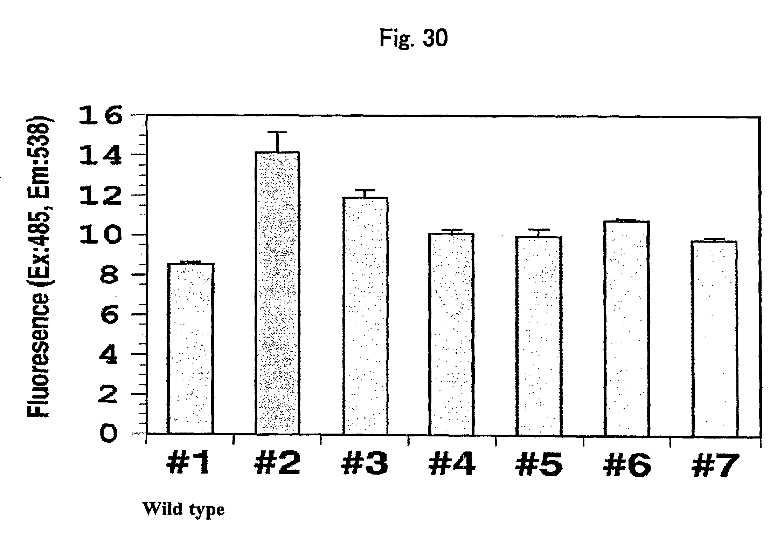

COS-7 cells cultured in 24-well plates were transformed with plasmids pQBI25-3#1 through pQBI25-3#7 obtained in Example 23 using LipofectAmine PLUS (Gibco BRL) according to the protocol attached thereto. Forty-two hours after the transformation, fluorescence intensity at the excitation wavelength of 435 nm and emission wavelength of 538 nm was measured with a multiplate fluorescence measurement apparatus (Dainippon Pharmaceutical). As a result, the nucleotide sequences #2 through #7 generally exhibited higher fluorescence than sequence #1 having the N-terminal nucleotide sequence of the wild-type LLPL. The results also revealed that the expression levels of GFP (reporter gene) vary depending on the nucleotide sequence encoding the N-terminal portion of LLPL (Fig. 30).

### EXAMPLE 25

### Comparison of Expression Levels of GFP Proteins with Different Nucleotide Sequences in the N-terminal in Stable Expression in Animal Cells

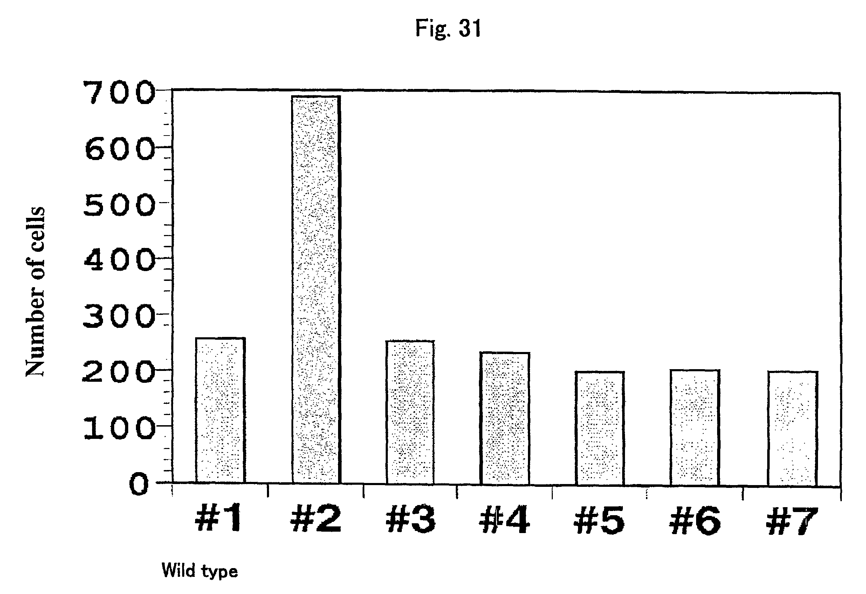

Plasmids pQBI25-3LLPL#1 through pQBI25-3LLPL#7 obtained in Example 23 were cut at the ScaI site located in the ampicillin resistance gene with ScaI (Takara Shuzo) to obtain linear DNAs. CHO-K1 cells cultured in a usual manner were scraped off, washed with PBS and suspended in PBS to give a concentration of 1x10⁷ cells/0.8 ml. Ten µg each of the linear DNAs from plasmids pQBI25-3LLPL#1 through pQBI25-3LLPL#7 were added separately to the cell suspension, which was then left stationary in ice for 5 min. Subsequently, transformation was carried out at 960 µF and 250 V with an electroporation apparatus (Bio-Rad). The cells were left stationary in ice for 10 min, then returned to a medium and cultured. Thirty hours after the transformation, the cells were transferred to a medium supplemented with geneticin (Gibco BRL) at 0.8 mg/ml and cultured further to select resistant clones. Thirteen days after the transformation, the cells were scraped off and subjected to measurement. Briefly, using a flowcytometer (Falcon), the fluorescence of individual cells excited by argon laser of 488 nm was measured with a detector equipped with a 530 nm filter. For each type of the transformants obtained with the above-described expression plasmids, 10,000 cells were measured. Then, GFP expression levels were compared using the number of cells whose fluorescence intensity (measured value) is 101 or above when the voltage of the photomultiplier tube in the detector was set at 500 V. As a result, when sequences #3 through #7 were used, the number of such cells was equal to or below that number when sequence #1 (having the N-terminal nucleotide sequence of the wild-type LLPL) was used. When sequence #2 was used, the number of such cells was more than 2 fold of that number when sequence #1 was used. It was shown that the expression levels of GFP (reporter gene) vary depending on the nucleotide sequence encoding the N-terminal portion of LLPL even in stable expression in animal cells (Fig. 31).

### INDUSTRIAL APPLICABILITY

According to the present invention, a protein of interest can be produced in a large quantity using recombinant DNA technology. Thus, the present invention is useful as a process for industrial production of a protein of interest. Furthermore, according to the present invention, a transformant producing a protein of interest in a large quantity can be obtained easily and simply.

## Claims

1. A process of producing a DNA that encodes a protein of interest and is suitable for expression, comprising the following steps:
1) preparing recombinant vectors by using a mixture of a plurality of DNAs encoding the full length or a partial amino acid sequence of the protein of interest and integrating each of said DNAs into a vector retaining a promoter DNA functional in a host cell and a reporter gene so that said DNA is located downstream of the promoter DNA and upstream of the reporter gene,
2) transforming the host cell with the recombinant vectors,
3) culturing the resultant transformants,
4) measuring the expression of the reporter gene, and
5) preparing a DNA that comprises the same nucleotide sequence as that of the DNA encoding the full length or the partial amino acid sequence of the protein of interest possessed by a transformant in which a high expression of the reporter gene has been confirmed and that encodes the protein of interest.

2. The process according to claim 1, wherein the full length or a partial amino acid sequence of the protein of interest is the full length of the protein of interest or a partial amino acid sequence comprising the N-terminal of the protein of interest.

3. A process of producing a protein of interest or a salt thereof, comprising the following steps:
1) preparing recombinant vectors by using a mixture of a plurality of DNAs encoding the full length or a partial amino acid sequence of the protein of interest and integrating each of said DNAs into a vector retaining a promoter DNA functional in a host cell and a reporter gene so that said DNA is located downstream of the promoter DNA and upstream of the reporter gene,
2) transforming the host cell with the recombinant vectors,
3) culturing the resultant transformants,
4) measuring the expression of the reporter gene,
5) preparing a recombinant vector comprising a DNA that comprises the same nucleotide sequence as that of the DNA encoding the full length or the partial amino acid sequence of the protein of interest possessed by a transformant in which a high expression of the reporter gene has been confirmed and that encodes the protein of interest,
6) transforming a host cell with the recombinant vector, and
7) culturing the resultant transformant.

4. The process according to claim 2, wherein the full length or a partial amino acid sequence of the protein of interest is the full length of the protein of interest or a partial amino acid sequence comprising the N-terminal of the protein of interest.

5. A process of producing a DNA that encodes a eukaryote-derived protein of interest and is suitable for expression in a prokaryote, comprising the following steps:
1) preparing recombinant vectors by using a mixture of a plurality of DNAs encoding an N-terminal amino acid sequence of the eukaryote-derived protein of interest and integrating each of said DNAs into a vector retaining a promoter DNA functional in prokaryotes and a reporter gene so that said DNA is located downstream of the promoter DNA and upstream of the reporter gene,
2) transforming the prokaryote with the recombinant vectors,
3) culturing the resultant prokaryotic transformants,
4) measuring the expression of the reporter gene, and
5) preparing a DNA that comprises the same nucleotide sequence as that of the DNA encoding the N-terminal amino acid sequence of the protein of interest possessed by a transformant in which a high expression of the reporter gene has been confirmed and that encodes the protein of interest.

6. A process of producing an eukaryote-derived protein of interest or a salt thereof in a prokaryote, comprising the following steps:
1) preparing recombinant vectors by using a mixture of a plurality of DNAs encoding an N-terminal amino acid sequence of the eukaryote-derived protein of interest and integrating each of said DNAs into a vector retaining a promoter DNA functional in prokaryotes and a reporter gene so that said DNA is located downstream of the promoter DNA and upstream of the reporter gene,
2) transforming the prokaryote with the recombinant vectors,
3) culturing the resultant prokaryotic transformants,
4) measuring the expression of the reporter gene,
5) preparing a recombinant vector comprising a DNA that comprises the same nucleotide sequence as that of the DNA encoding the N-terminal amino acid sequence of the protein of interest possessed by a transformant in which a high expression of the reporter gene has been confirmed and that encodes the protein of interest,
6) transforming the prokaryote with the recombinant vector, and
7) culturing the resultant prokaryotic transformant.

7. A non-wild type DNA obtained by the production process according to claim 1, that comprises the same nucleotide sequence as that of the DNA encoding the full length or a partial amino acid sequence of a protein of interest possessed by a transformant in which a high expression of the reporter gene has been confirmed and that encodes the full length of the protein of interest.

8. A non-wild type DNA obtained by the production process according to claim 1 encoding the full length of a protein of interest, wherein the DNA is composed of a DNA encoding a partial amino acid sequence of the protein of interest possessed by a transformant in which a high expression of the reporter gene has been confirmed and a DNA(s) encoding the remaining amino acid sequence(s) of said protein ligated to the 3' end or/and 5' end of said DNA.

9. A DNA having the nucleotide sequence as shown in SEQ ID NO: 119, SEQ ID NO: 120, SEQ ID NO: 121 or SEQ ID NO: 122.

10. A recombinant vector comprising the DNA according to any one of claims 7 to 9.

11. A transformant transformed with the recombinant vector according to claim 10.

12. A process of producing a protein of interest or a salt thereof, comprising culturing the transformant according to claim 11 and allowing the transformant to produce the protein of interest.
